# EUROPEAN PATENT APPLICATION

(11) **EP 4 741 516 A1**
(43) Date of publication of application: **13.05.2026**
(21) Application number: 25215109.7
(22) Date of filing: 11.11.2025
(51) Int. Cl.: C12Q 1/6886, G16H 50/20

(54) **METHODS AND SYSTEMS FOR CLASSIFYING CANCER AND DETECTING IMPROVED CANCER THERAPIES**

(30) Priority: 12.11.2024 US 202463719617 P
(71) Applicant: Tempus AI, Inc., Chicago, IL 60654 (US)
(72) Inventor: CORCORAN, Emma Tung, Chicago, IL 60654 (US); SELITSKY, Sara, Chicago, IL 60654 (US)
(74) Representative: Barker Brettell LLP

(57) **Abstract**

Disclosed herein are methods (100) and systems (200) for classifying a cancer from a subject. The methods (100) and systems (200) classify the cancer based on similar characteristics, e.g., molecular profiles. The methods (100) and systems (200) may be predictive of the subject's response to treatments based on the classification of the cancer. The methods (100) and systems (200) may be used to define improved therapies for subjects with cancers with limited treatment options, e.g., rare cancers.

## Description

### CROSS-REFERENCE TO RELATED APPLICATIONS

The present application claims priority to U.S. Provisional Patent Application No. 63/719,617 that was filed November 12, 2024, the entire contents of which are hereby incorporated by reference.

### TECHNICAL FIELD

This present disclosure relates to systems, methods, and compositions useful for profiling a subject's cancer by classifying the cancer by a particular cancer subtype. The present disclosure also relates to systems and methods for diagnosing, matching a patient with appropriate treatments, monitoring, or predicting disease, condition, or therapeutic outcomes based on the cancer subtype of a subject.

### BACKGROUND

Squamous cell carcinomas (SCCs) can occur in a variety of tissues with varying frequencies. Rare cancers are unlikely to be the subject of clinical trials, in part, due to the difficulty of recruiting a sufficient subject population. The limited number of clinical trials further complicates the diagnosis and treatment of these diseases, SCCs in different tissue types may have similar morphologies. Therefore, there is a need in the art for methods to characterize SCCs, and other cancers, based on their molecular profile which may lead to improved diagnostics, improved treatment options, and improved recruiting of subjects with rare cancers into clinical trials.

### SUMMARY

To the accomplishment of the foregoing and related ends, the invention, then, comprises the features hereinafter fully described. The following description and the annexed drawings set forth in detail certain illustrative aspects of the invention. However, these aspects are indicative of but a few of the various ways in which the principles of the invention can be employed. Other aspects, advantages and novel features of the invention will become apparent from the following detailed description of the invention when considered in conjunction with the drawings.

In an aspect of the current disclosure, methods are provided. In some embodiments, the methods comprise: obtaining, with a computer system, sequencing read data collected from a sample from the cancer of the subject, the read data comprising RNA sequencing data; classifying, with the computer system, the cancer as a subtype of cancer, using a trained machine learning algorithm, wherein the subtype of cancer comprises a plurality of cell proliferative diseases with common characteristics, wherein the common characteristics comprise similar molecular profiles, wherein the trained machine learning algorithm is trained on a data set of sequencing read data collected from a cohort of subjects suffering from cancer.

In some embodiments, the sample comprises at least one of a tumor sample, blood sample, or cell free DNA. In some embodiments, the plurality of cell proliferative diseases includes squamous cell carcinomas (SCC). In some embodiments, the SCC includes anogenital, cervical, esophageal, head and neck, lung, skin, urothelial, colorectal, and vulvar squamous cell carcinomas. In some embodiments the common characteristics further include similar phenotypes, prognosis, and predicted responses to treatment.

In some embodiments, the similar molecular profiles comprise expression levels of one or more of RNF186, CCL15, TMIGD1, RPL10L, ATOH1, ANKS4B, ALPI, SCL17A4, B3GNT6, MOGAT3, SFTA3, GGTLC1, NAPSA, SFTPD, MS4A15, VWA3A, ANKRD66, HABP2, CPAMD8, KCNK3, CFAP95, CFAP43, OSGIN1, SRXN1, G6PD, ETNK2, DGKG, NDGA1, LDC1, RAB3B, TAGA3, PLCXD2, GSTM2, WNT5A, RAB25, TTLL10, SGPP2, SPINK9, IGSF9, ARHGEF26, PIR, RAPGEFL1, CIMAP2, SCNN1A, ZBTB7C, BDNF, ARG1, TREX2, CMA1, KRTAP5-4, LIPM, SPTLC3, GCSAML, HAL, LGALSL, VSIG8, TMC4, ELMOD1, SMPD3, GRACDL, DPF1, RAX, GATM, KLHL35, TMEM236, ACTBL2, TCEA3, EPB41LB, CT62, DKK3, FJX1, CASP5, MANEAL, or NUP210.

In some embodiments, the cohort of subjects comprises subjects diagnosed with at least 5 different types of cancers. In some embodiments, each subject in the cohort of subjects has been diagnosed with a squamous cell carcinoma.

In some embodiments, the trained machine learning algorithm comprises at least one of a gradient boosting model, a random forest model, a neural network, a regression model, ElasticNet, or a Naive Bayes model.

In some embodiments, the method further comprises generating a report. The report may include the subtype of cancer, the plurality of cell proliferative diseases with common characteristics, and the molecular profiles. The report may further include a list of treatment options. In some embodiments, treatment options are identified based on the plurality of cell proliferative diseases with common characteristics and the molecular profiles.

In some embodiments the cancer may have limited treatment options comprising at least one of ineffective treatments, few treatments, and no known treatments. In some embodiments the cancer with little limited treatments is vulvar squamous cell carcinoma.

In some embodiments, the molecular profiles comprise RNA expression data and the computer system classifies the cancer based on expression of a plurality of signature genes in the RNA sequencing data.

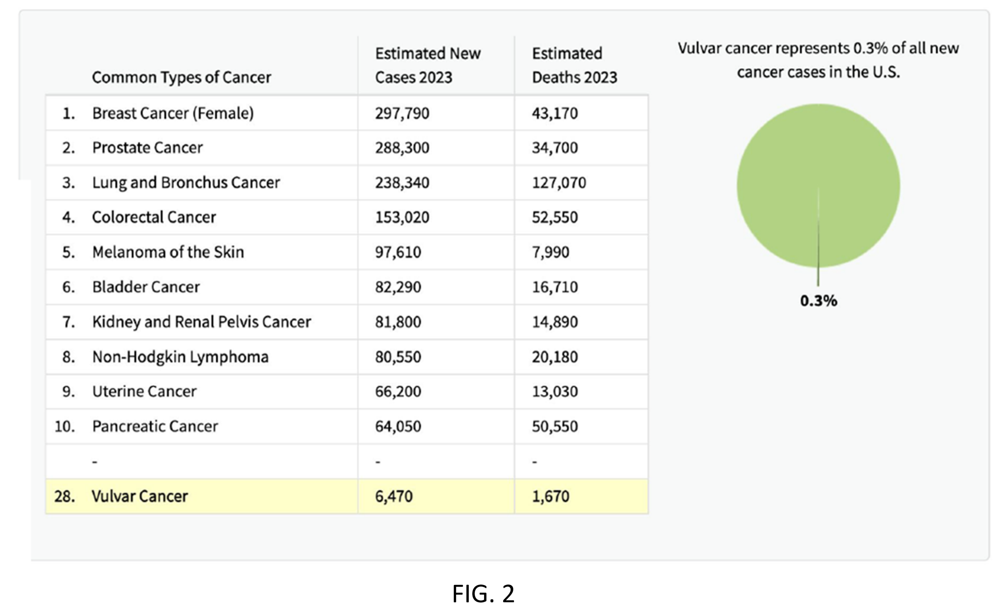

In some embodiments, the plurality of signature genes comprises two or more genes selected from one of (i), (ii), (iii), (iv), (v), or (vi):
(i) CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, or ZSWIM5;
(ii) ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, or WNT16;
(iii) RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRK1, POF1B, CBX2, CEACAM1, THBS1, NEBL, CCDC185, C20orf144, or CHODL;
(iv) OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, Clorf226, ENTHD1, SLC7A11, UGT1A1, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, or LGSN;
(v) SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, Cl6orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, Clorf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, or HYDIN;
(vi) RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULTlBl, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, or BTNL3.

In an aspect of the current disclosure, methods are provided. In some embodiments, the methods comprise: obtaining, with a computer system, sequencing read data collected from a sample from a cancer of a subject, the read data comprising RNA sequencing data; classifying, with the computer system, the cancer as a subtype of cancer, using a trained machine learning algorithm, wherein the subtype of cancer comprises a plurality of cell proliferative diseases with common characteristics wherein the common characteristics comprise similar molecular profiles, wherein the trained machine learning algorithm is trained on a data set of sequencing read data collected from a cohort of subjects suffering from cancer.

In some embodiments, methods of classifying a cancer from a subject are provided and comprise: obtaining, with a computer system, sequencing read data collected from a sample from the cancer of the subject, the read data comprising RNA sequencing data; classifying, with the computer system, the cancer as a subtype of cancer, using a trained machine learning algorithm, wherein the subtype of cancer comprises a plurality of cell proliferative diseases with common characteristics wherein the common characteristics comprise similar molecular profiles, wherein the trained machine learning algorithm is trained on a data set of sequencing read data collected from a cohort of subjects suffering from cancer.

In some embodiments, methods of diagnosing a cancer from a subject are provided and comprise obtaining, with a computer system, sequencing read data collected from a sample of the cancer, the read data comprising RNA sequencing data; classifying, with the computer system, the cancer as a subtype of cancer, using a trained machine learning algorithm, wherein the subtype of cancer comprises a plurality of cell proliferative diseases with common characteristics wherein the common characteristics comprise similar molecular profiles, wherein the trained machine learning algorithm is trained on a data set of sequencing read data collected from a cohort of subjects suffering from cancer.

In some embodiments, methods of identifying treatment options for a subject suffering from a cancer for which there are limited treatments are provided and comprise: obtaining, with a computer system, sequencing read data collected from a sample of the cancer from the subject, wherein the read data comprising RNA sequencing data; classifying, with the computer system, the cancer as a subtype of cancer, using a trained machine learning algorithm, wherein the subtype of cancer comprises a plurality of cell proliferative diseases with common characteristics wherein the common characteristics comprise similar molecular profiles, wherein the trained machine learning algorithm is trained on a data set of sequencing read data collected from a cohort of subjects suffering from cancer. In some embodiments, the sample comprises at least one of a tumor sample, blood sample, or cell free DNA. In some embodiments, the plurality of cell proliferative diseases comprises squamous cell carcinomas (SCC). In some embodiments, the squamous cell carcinomas comprises anogenital, cervical, esophageal, head and neck, lung, skin, urothelial, colorectal, and vulvar squamous cell carcinomas. In some embodiments, the common characteristics further comprises similar phenotypes, prognosis, and predicted responses to treatment. In some embodiments, the similar phenotypes comprise symptoms, comorbidities, and lifestyle habits. In some embodiments, the comorbidities comprise HPV status. In some embodiments, the prognosis comprises survivability, aggressiveness, and stage. In some embodiments, the predicted response to treatment comprises predicted response to chemotherapy. In some embodiments, the predicted response to treatment comprises predicted response to an immunotherapy, or a chemotherapy, or targetable mutation small molecule inhibitors, such as PIK3CA inhibitors. In some embodiments, the immunotherapy comprises an immune checkpoint inhibitor (ICI). In some embodiments, the chemotherapy comprises a platinum-based therapy or a taxane therapy. In some embodiments, the platinum-based therapy comprises carboplatin. In some embodiments, the taxane therapy comprises paclitaxel. In some embodiments, the similar molecular profiles comprise expression levels of one or more of RNF186, CCL15, TMIGD1, RPL10L, ATOH1, ANKS4B, ALPI, SCL17A4, B3GNT6, MOGAT3, SFTA3, GGTLC1, NAPSA, SFTPD, MS4A15, VWA3A, ANKRD66, HABP2, CPAMD8, KCNK3, CFAP95, CFAP43, OSGIN1, SRXN1, G6PD, ETNK2, DGKG, NDGA1, LDC1, RAB3B, TAGA3, PLCXD2, GSTM2, WNT5A, RAB25, TTLL10, SGPP2, SPINK9, IGSF9, ARHGEF26, PIR, RAPGEFL1, CIMAP2, SCNN1A, ZBTB7C, BDNF, ARG1, TREX2, CMA1, KRTAP5-4, LIPM, SPTLC3, GCSAML, HAL, LGALSL, VSIG8, TMC4, ELMOD1, SMPD3, GRACDL, DPF1, RAX, GATM, KLHL35, In some embodiments, the cohort of subjects comprises subjects diagnosed with at least 5 different types of cancers. In some embodiments, each subject in the cohort of subjects has been diagnosed with a squamous cell carcinoma. In some embodiments, the trained machine learning algorithm comprises at least one of a gradient boosting model, a random forest model, a neural network, a regression model, ElasticNet, or a Naive Bayes model. In some embodiments, the trained machine learning algorithm is ElasticNet. In some embodiments, the method further comprises generating a report. In some embodiments, the report comprises the subtype of cancer, the plurality of cell proliferative diseases with common characteristics, and the molecular profiles. In some embodiments, the report further comprises patient data. In some embodiments, the report further comprises a list of treatment options. In some embodiments, the diagnosed cancer comprises a squamous cell carcinoma. In some embodiments, the diagnosed cancer does not comprise a squamous cell carcinoma. In some embodiments, limited treatments comprise at least one of ineffective treatments, few treatments, and no known treatments. In some embodiments, the treatment options are identified based on the plurality of cell proliferative diseases with common characteristics and the molecular profile. In some embodiments, the cancer with limited treatments is vulvar squamous cell carcinoma. In some embodiments, the molecular profiles comprise RNA expression data and the computer system classifies the cancer based on expression of a plurality of signature genes in the RNA sequencing data. In some embodiments, the plurality of signature genes comprises two or more genes selected from one of (i), (ii), (iii), (iv), (v), or (vi):
(i) CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, or ZSWIM5;
(ii) ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, or WNT16;
(iii) RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRK1, POF1B, CBX2, CEACAM1, THBS1, NEBL, CCDC185, C20orf144, or CHODL;
(iv) OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, C1orf226, ENTHD1, SLC7A11, UGT1A1, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, or LGSN;
(v) SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, Cl6orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, Clorf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, or HYDIN;
(vi) RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, or BTNL3.

In some embodiments, methods of classifying a cancer are provided and the methods comprising: obtaining, with a computer system, sequencing read data collected from a sample of the cancer, the read data comprising RNA sequencing data; classifying, with the computer system, the cancer as a subtype of cancer, using a trained machine learning algorithm, wherein the subtype of cancer comprises a plurality of cell proliferative diseases with common characteristics, wherein the common characteristics comprise similar molecular profiles, wherein the molecular profiles comprise RNA expression data and the computer system classifies the cancer based on expression of a plurality of signature genes in the RNA sequencing data, and wherein the trained machine learning algorithm is trained on a data set of sequencing read data collected from a cohort of subjects suffering from cancer. In some embodiments, the plurality of signature genes comprises two or more genes selected from the group consisting of CRACDL, DPF 1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, and ZSWIM5. In some embodiments, the plurality of signature genes comprises CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, and ZSWIM5. In some embodiments, the plurality of signature genes comprises two or more genes selected from the group consisting of ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, and WNT16. In some embodiments, the plurality of signature genes comprises ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, and WNT16. In some embodiments, the plurality of signature genes comprises two or more genes selected from the group consisting of RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, C1orf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRK1, POF1B, CBX2, CEACAM1, THBS1, NEBL, CCDC185, C20orf144, and CHODL. In some embodiments, the plurality of signature genes comprises RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRK1, POF1B, CBX2, CEACAM1, THBS1, NEBL, CCDC185, C20orf144, and CHODL. In some embodiments, the plurality of signature genes comprises two or more genes selected from the group consisting of OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, Clorf226, ENTHD1, SLC7All, UGT1Al, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, and LGSN. In some embodiments, the plurality of signature genes comprises OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, Clorf226, ENTHD1, SLC7All, UGT1Al, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, and LGSN. In some embodiments, the plurality of signature genes comprises two or more genes selected from the group consisting of SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, Cl6orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, Clorf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, and HYDIN. In some embodiments, the plurality of signature genes comprises SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, Cl6orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, Clorf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, and HYDIN. In some embodiments, the plurality of signature genes comprises two or more genes selected from the group consisting of RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, and BTNL3. In some embodiments, plurality of signature genes comprises RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, and BTNL3. In some embodiments, the sample comprises at least one of a tumor sample, blood sample, or cell free DNA. In some embodiments, the plurality of cell proliferative diseases comprises squamous cell carcinomas (SCC). In some embodiments, the squamous cell carcinomas comprises anogenital, cervical, esophageal, head and neck, lung, skin, urothelial, colorectal, and vulvar.

In some embodiments, the common characteristics further comprises similar phenotypes, prognosis, and predicted responses to treatment. In some embodiments, the similar phenotypes comprise symptoms, comorbidities, and lifestyle habits. In some embodiments, the comorbidities comprise HPV status. In some embodiments, the prognosis comprises survivability, aggressiveness, and stage. In some embodiments, the predicted response to treatment comprises predicted response to chemotherapy. In some embodiments, the predicted response to treatment comprises predicted response to an immunotherapy, or a chemotherapy. In some embodiments, the immunotherapy comprises an immune checkpoint inhibitor (ICI). In some embodiments, the chemotherapy comprises a platinum-based therapy or a taxane therapy. In some embodiments, the platinum-based therapy comprises cisplatin. In some embodiments, the taxane therapy comprises paclitaxel. In some embodiments, each subject in the cohort of subjects has been diagnosed with a cancer that is different from other subjects in the cohort of subjects. In some embodiments, each subject in the cohort of subjects has been diagnosed with a squamous cell carcinoma. In some embodiments, the trained machine learning algorithm comprises at least one of a gradient boosting model, a random forest model, a neural network, a regression model, ElasticNet, or a Naive Bayes model. In some embodiments, the trained machine learning algorithm is ElasticNet. In some embodiments, the method further comprises generating a report. In some embodiments, the report comprises the subtype of cancer, the plurality of cell proliferative diseases with common characteristics, and the molecular profiles. In some embodiments, the report further comprises patient data. In some embodiments, the report further comprises recommended treatment options. In some embodiments, the cancer comprises a squamous cell carcinoma. In some embodiments, the cancer does not comprise a squamous cell carcinoma. In some embodiments, limited treatments comprise at least one of ineffective treatments, few treatments, and no known treatments. In some embodiments, the treatment options are identified based on the plurality of cell proliferative diseases with common characteristics and the molecular profile. In some embodiments, the cancer with limited treatments is vulvar squamous cell carcinoma.

Provided herein are systems comprising one or more processor and one or more memory that are configured to perform the disclosed methods.

Provided herein are computer readable media (CRM) comprising instructions stored thereon that, when executed by a processor, perform the disclosed methods. For example, the CRM comprises instructions stored thereon that, when executed by a processor, obtain, with a computer system, sequencing read data collected from a sample of the cancer, the read data comprising RNA sequencing data; classify, with the computer system, the cancer as a subtype of cancer, using a trained machine learning algorithm, wherein the subtype of cancer comprises a plurality of cell proliferative diseases with common characteristics, wherein the common characteristics comprise similar molecular profiles, wherein the molecular profiles comprise RNA expression data and the computer system classifies the cancer based on expression of a plurality of signature genes in the RNA sequencing data, and wherein the trained machine learning algorithm is trained on a data set of sequencing read data collected from a cohort of subjects suffering from cancer. In some embodiments, the plurality of signature genes comprises two or more genes selected from the group consisting of CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, and ZSWIM5. In some embodiments, the plurality of signature genes comprises CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, and ZSWIM5. In some embodiments, the plurality of signature genes comprises two or more genes selected from the group consisting of ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, and WNT16. In some embodiments, the plurality of signature genes comprises ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, and WNT16. In some embodiments, the plurality of signature genes comprises two or more genes selected from the group consisting of RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRK1, POF1B, CBX2, CEACAM1, THBS1, NEBL, CCDC185, C20orf144, and CHODL. In some embodiments, the plurality of signature genes comprises RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRK1, POF1B, CBX2, CEACAM1, THBS1, NEBL, CCDC185, C20orf144, and CHODL. In some embodiments, the plurality of signature genes comprises two or more genes selected from the group consisting of OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, C1orf226, ENTHD1, SLC7All, UGT1A1, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, and LGSN. In some embodiments, the plurality of signature genes comprises OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, C1orf226, ENTHD1, SLC7All, UGT1A1, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, and LGSN. In some embodiments, the plurality of signature genes comprises two or more genes selected from the group consisting of SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, Cl6orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, C1orf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, and HYDIN. In some embodiments, the plurality of signature genes comprises SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, Cl6orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, Clorf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, and HYDIN. In some embodiments, the plurality of signature genes comprises two or more genes selected from the group consisting of RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, and BTNL3. In some embodiments, plurality of signature genes comprises RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, and BTNL3. In some embodiments, the sample comprises at least one of a tumor sample, blood sample, or cell free DNA. In some embodiments, the plurality of cell proliferative diseases comprises squamous cell carcinomas (SCC). In some embodiments, the squamous cell carcinomas comprises anogenital, cervical, esophageal, head and neck, lung, skin, urothelial, colorectal, and vulvar.

In some embodiments, the common characteristics further comprises similar phenotypes, prognosis, and predicted responses to treatment. In some embodiments, the similar phenotypes comprise symptoms, comorbidities, and lifestyle habits. In some embodiments, the comorbidities comprise HPV status. In some embodiments, the prognosis comprises survivability, aggressiveness, and stage. In some embodiments, the predicted response to treatment comprises predicted response to chemotherapy. In some embodiments, the predicted response to treatment comprises predicted response to an immunotherapy, or a chemotherapy. In some embodiments, the immunotherapy comprises an immune checkpoint inhibitor (ICI). In some embodiments, the chemotherapy comprises a platinum-based therapy or a taxane therapy. In some embodiments, the platinum-based therapy comprises cisplatin. In some embodiments, the taxane therapy comprises paclitaxel. In some embodiments, each subject in the cohort of subjects has been diagnosed with a cancer that is different from other subjects in the cohort of subjects. In some embodiments, each subject in the cohort of subjects has been diagnosed with a squamous cell carcinoma. In some embodiments, the trained machine learning algorithm comprises at least one of a gradient boosting model, a random forest model, a neural network, a regression model, ElasticNet, or a Naive Bayes model. In some embodiments, the trained machine learning algorithm is ElasticNet. In some embodiments, the method further comprises generating a report. In some embodiments, the report comprises the subtype of cancer, the plurality of cell proliferative diseases with common characteristics, and the molecular profiles. In some embodiments, the report further comprises patient data. In some embodiments, the report further comprises recommended treatment options. In some embodiments, the cancer comprises a squamous cell carcinoma. In some embodiments, the cancer does not comprise a squamous cell carcinoma. In some embodiments, limited treatments comprise at least one of ineffective treatments, few treatments, and no known treatments. In some embodiments, the treatment options are identified based on the plurality of cell proliferative diseases with common characteristics and the molecular profile. In some embodiments, the cancer with limited treatments is vulvar squamous cell carcinoma.

### BRIEF DESCRIPTION OF THE DRAWINGS

FIGS. 1A-1B. Schematic overviews of some embodiments of methods described herein. FIG. 1A. Schematic of workflow to classify cancer subtypes using pan-cancer subtype model. FIG. 1B. Schematic of workflow to train classifier.
FIGS. 2. Prevalence of cancer types. Estimated number of new diagnoses and deaths for a variety of cancers.
FIGS. 3A-3B. Characterizing pan-SCC cancer type relationship. FIG. 3A. PCA analysis across SCC cancer types using gene expression from RNA-seq. FIG. 3B. UMAP scores across SCC cancer types using gene expression from RNA-seq.
FIG. 4. Gene expression UMAP dimension reduction from across 27 different cancer types (n=2,743 samples). SCC transcriptomes cluster based on squamous gene-expression rather than by tissue or origin. SCC is plotted in color while non-SCC are represented in gray as a reference.
FIGS. 5A and 5B. FIG. 5A. Heatmap of SCC first line therapy treatment frequencies. Each cell in the heatmap represents the percent of samples treated with each therapy for each cancer type. FIG. 5B. Extended heatmap of SCC first line therapy treatment frequency.
FIGS. 6A-6D. Characterization of vSCC cohort (n=273 with RNA-seq, 268 with WES). FIG. 6A. Breakdown by tissue site of biopsy. 71.3% of samples are from primary tissues. FIG. 6B. Breakdown by patient stage at biopsy collection. The majority of samples are from later stage recurrent, more aggressive tumors. FIG. 6C. Breakdown by status of biopsy relative to treatment. Each representation of samples across treatment timeline groups. FIG. 6D. Age distribution of cohort at the time of biopsy. Median age of cohort is 69.6 years.
FIGS. 7A and 7B. vSCC alterations stratify based on HPV status. FIG. 7A. Representation of timing between solid and liquid biopsies matching. FIG. 7B. summary table of Hazard Ratio of vSCC stratified by different characteristics.
FIGS. 8A-8B. FIG. 8A. Principle component analysis of vSCC samples. FIG. 8B. NES of vSCC clusters with other genes/pathways.
FIGS. 9. vSCC clinical comparison by cluster. FIG. 9 Summary table of vSCC cohorts stratified by vSCC subtype.
FIGS. 10A-10D. FIG. 10A. OS naive KM curve for vSCC cohort. FIG. 10B OS naive KM curves stratified by vSCC subtype. FIG. 10C. Forestplot of vSCC cohort stratified by vSCC subtype. FIG. 10D. Summary table of vSCC outcomes stratified by vSCC subtype.
FIG. 11. Pan-SCC cohort summary table.

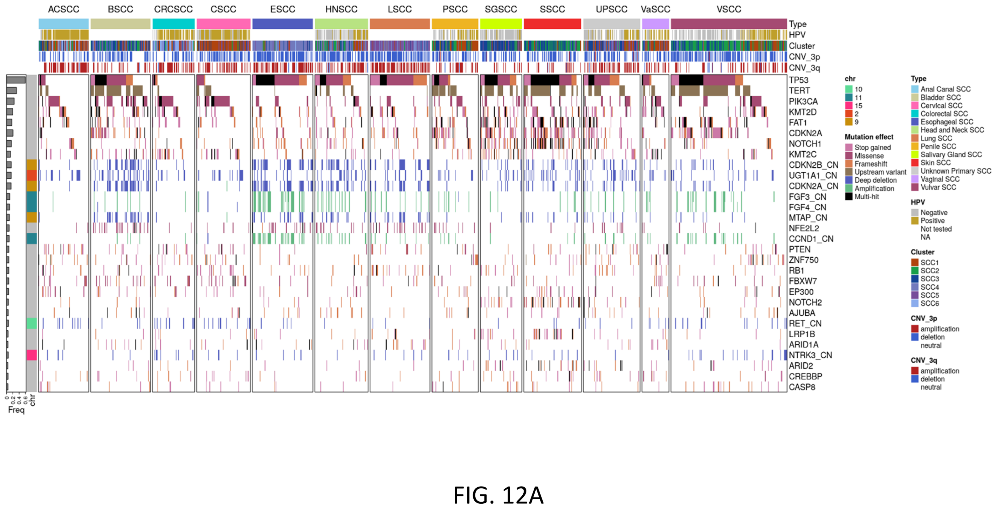

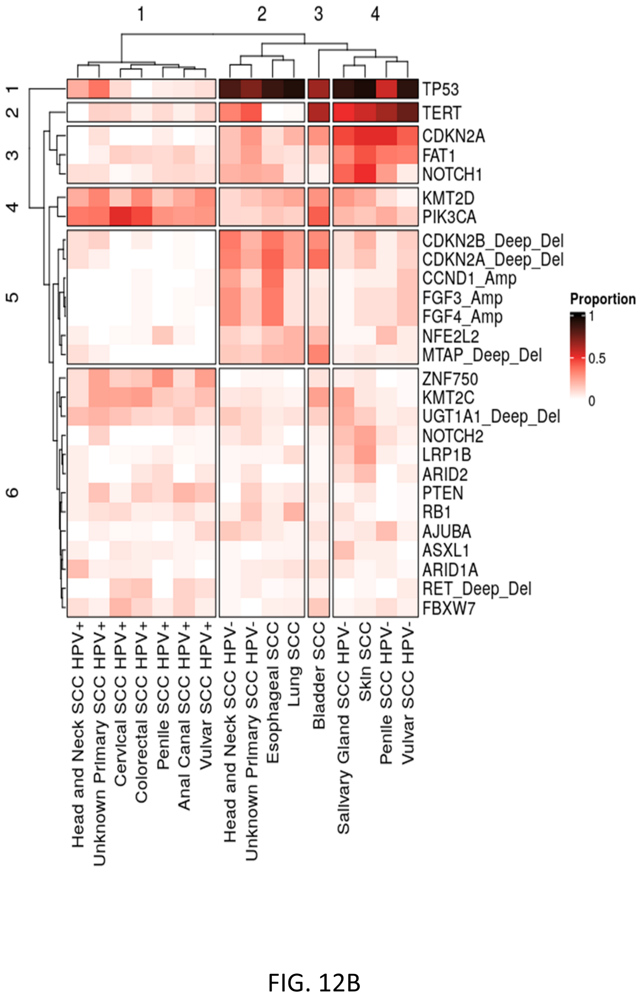

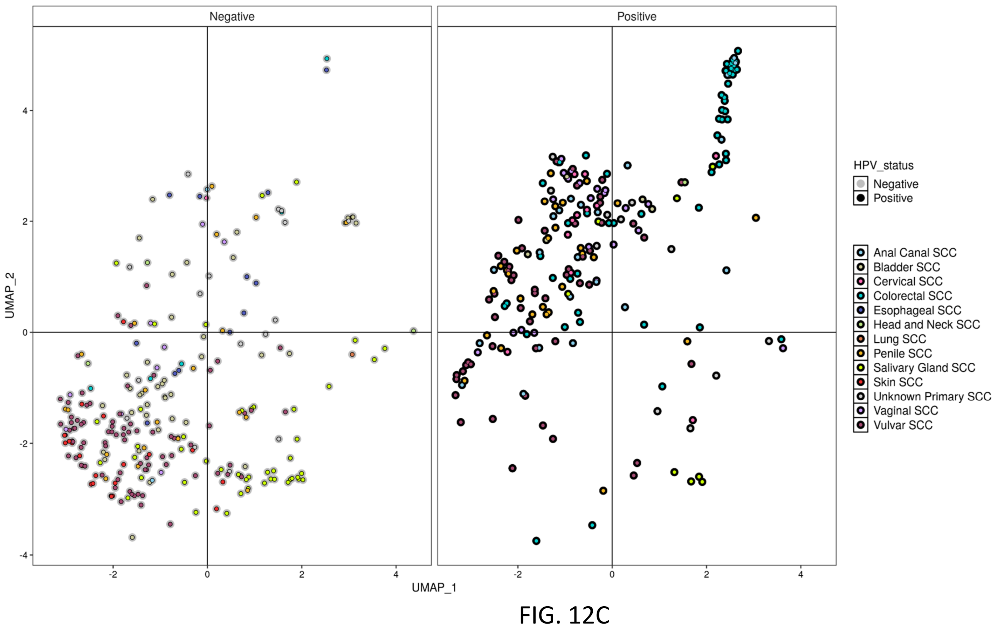

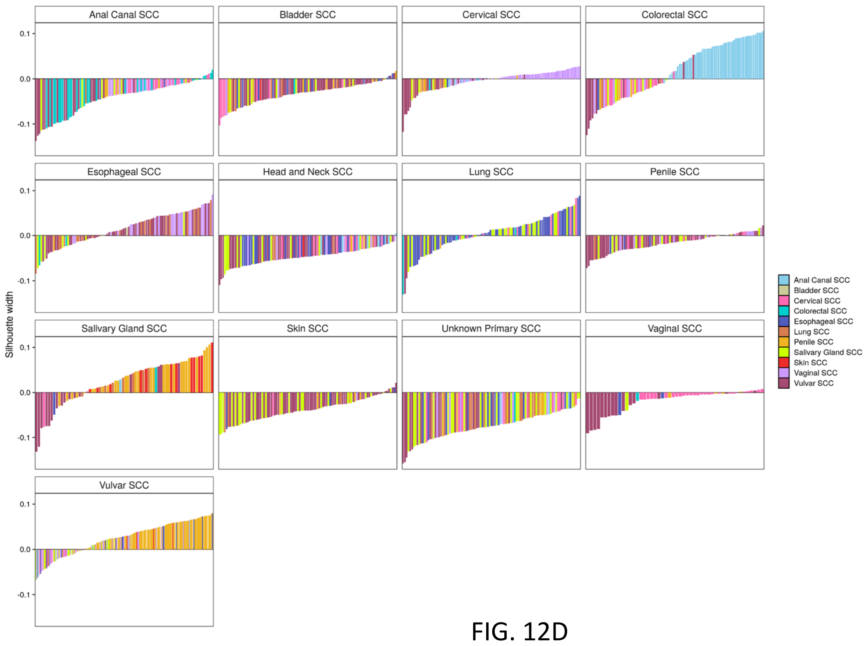

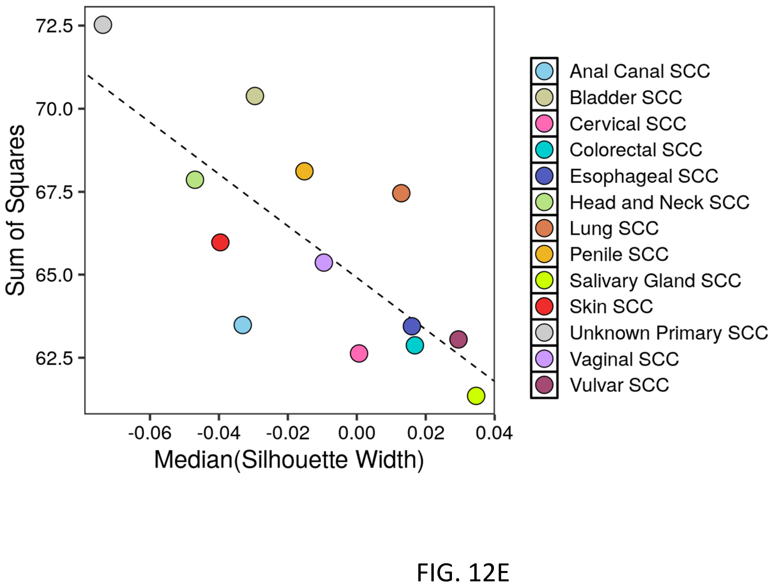

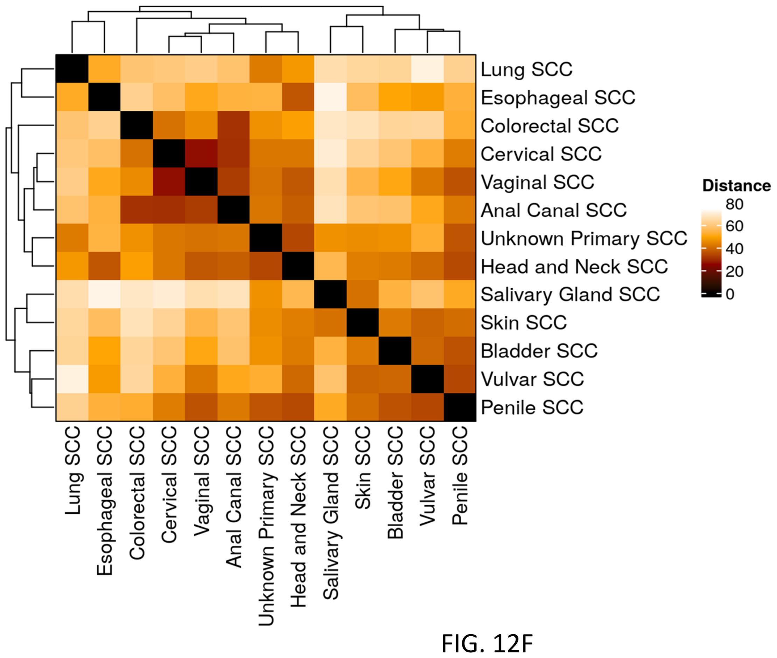

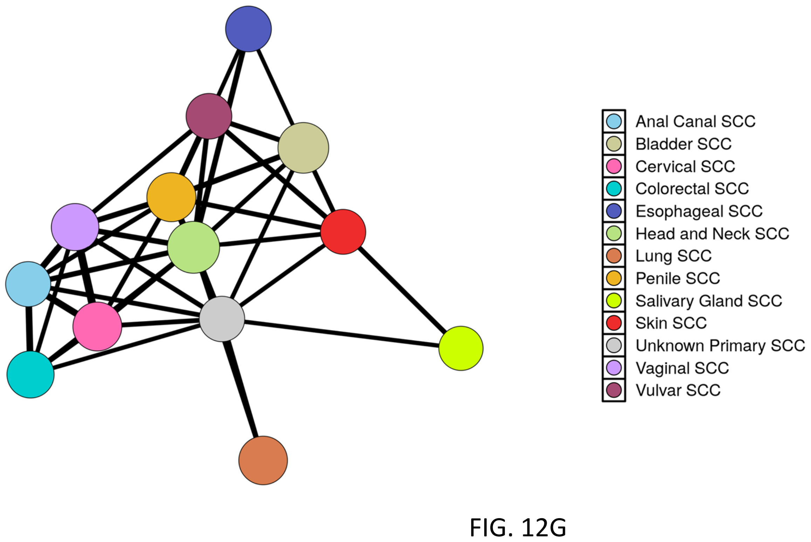

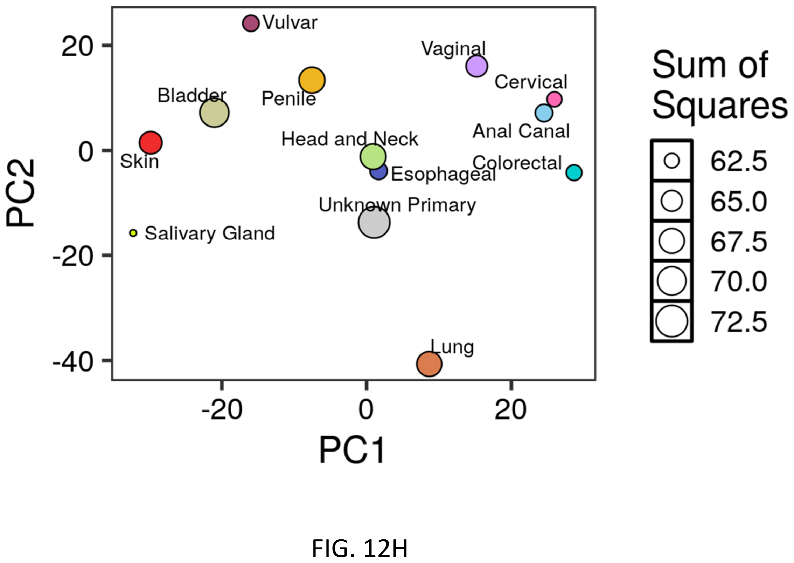

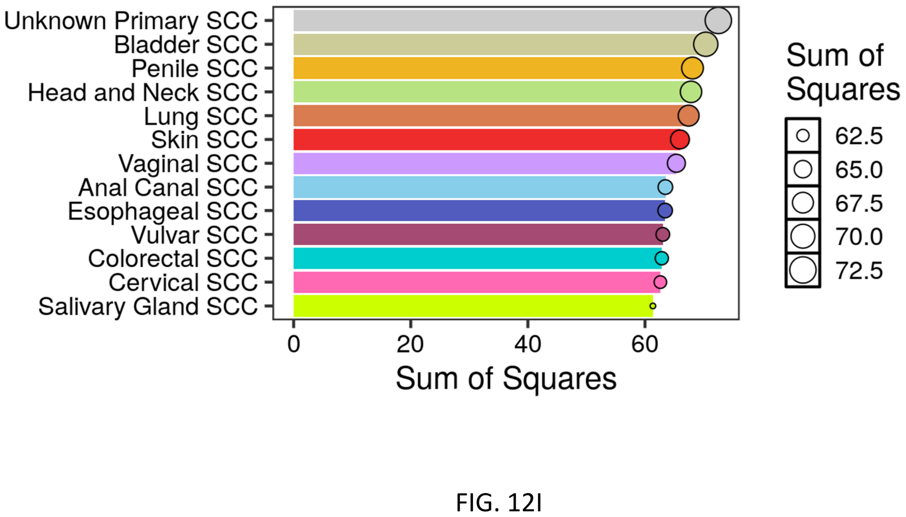

FIGS. 12A-12I. Characterizing pan-SCC cancer type relationship. FIG. 12A. Summary of mutational patterns summarized by SCC type. FIG. 12B. Heatmap of cancer types and mutational patterns. FIG. 12C. UMAP scores across SCC cancer types, sorted by HPV status. FIG. 12D. Silhouette widths across SCC cancer types. FIG. 12E. Sum of squares versus median silhouette width. FIG. 12F. Heatmap of Euclidian distances across SCC cancer types. FIG. 12G. Schematic representation of relatedness across SCC cancer type. FIG. 12H. PCA analysis of sum of squares across SCC cancer type. FIG. 12I. Sum of Squares across SCC cancer type.

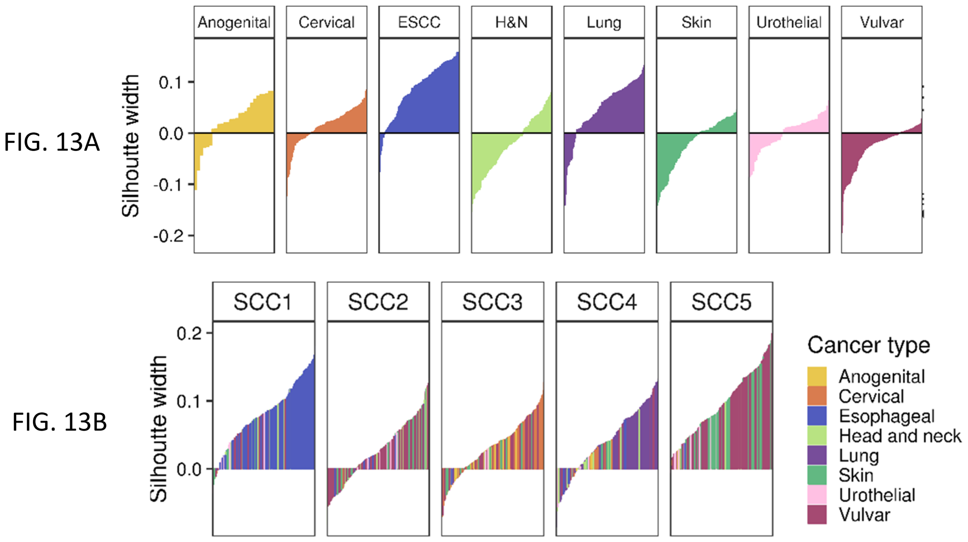

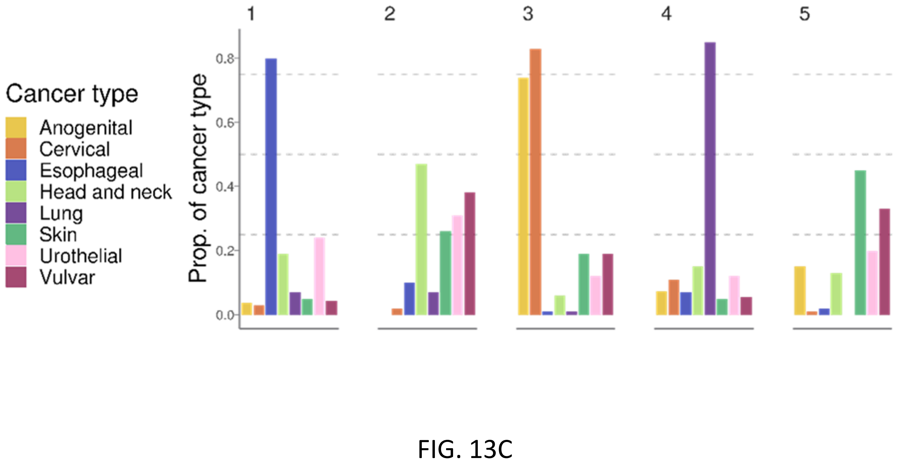

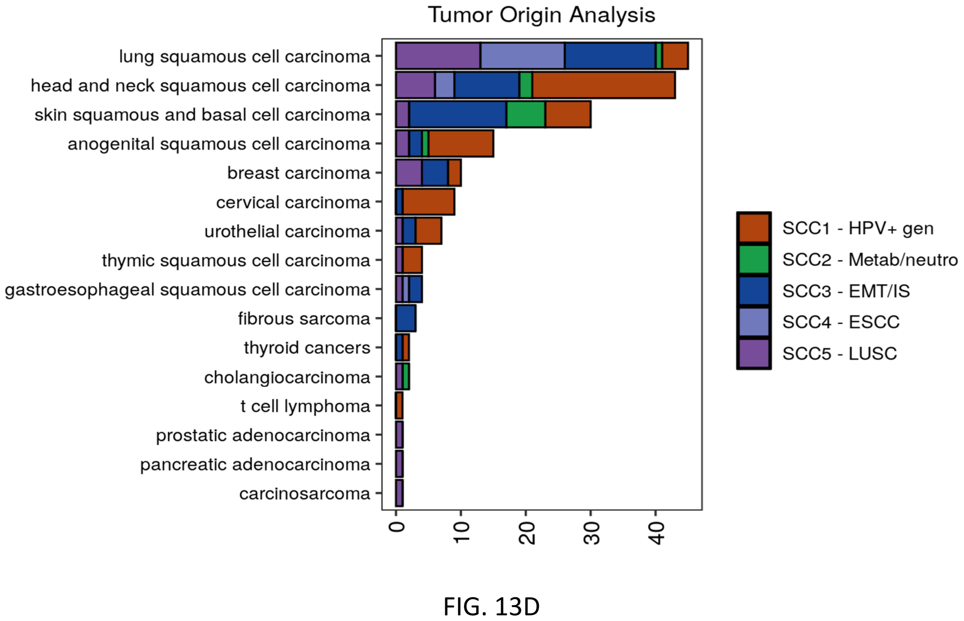

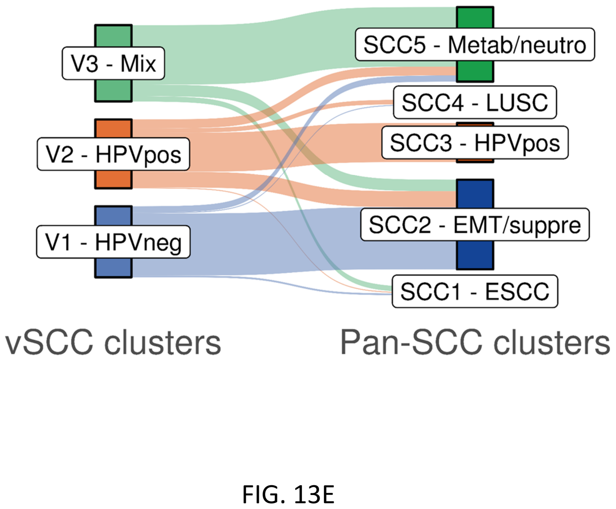

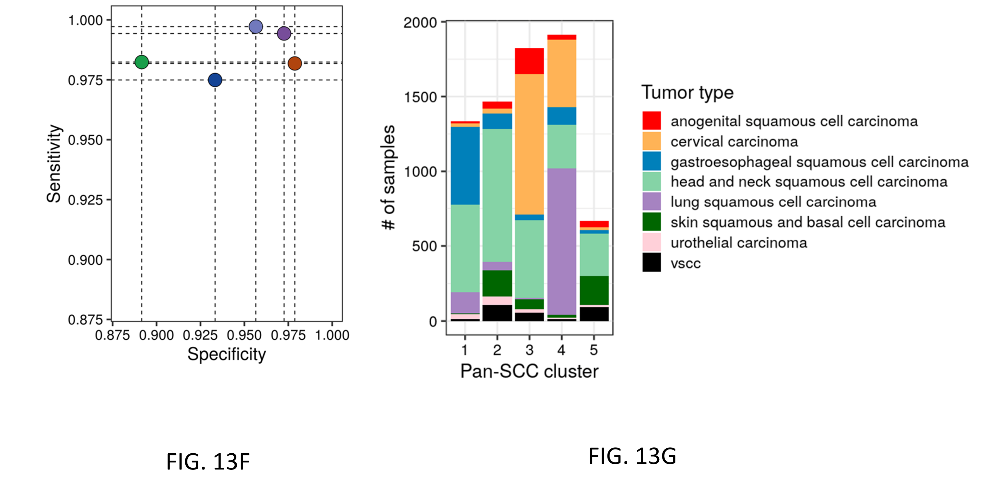

FIGS. 13A-13G. Pan-SCC analysis with five subtypes (pan-SCC 5S) leads to robust cluster membership. FIG. 13A. Silhouette width results for SCC types. Measuring the SW with tissue types as "cluster." 13B. pan-SCC 5S SW plot. FIG. 13C. Barplot represents the proportion of each cancer type belonging to each of the pan-SCC 5S subtypes. FIG. 13D. Tumor origin analysis stratified by pan-SCC 5S subtype. FIG. 13E. Sankey plot representing the proportion of vSCC samples from each of the 3 vSCC clusters mapping to each pan-SCC 5S subtypes. FIG. 13F. Sensitivity and specificity across pan-SCC 5S subtypes. FIG. 13G. Number of samples by SCC cancer across pan-SCC 5S subtypes.

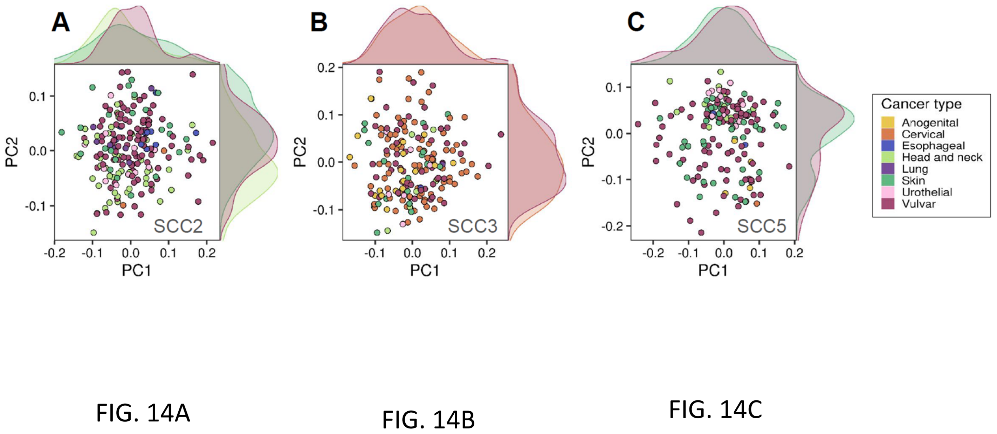

FIGS. 14A-14C. Gene expression analysis of vSCC within pan-SCC 5S subtypes which vSCCs were mostly found in pan-SCC 5S subtypes 2, 3, and 5 revealed that vSCCs were almost indistinguishable from skin SCC. FIG. 14A. Within pan-SCC 5S subtype 2, vSCCs vs skin SCC had only 3 differentially expressed genes (DEGs), whereas vSCC vs head and neck has 193 DEGs. FIG. 14B. Within pan-SCC 5S subtype 3, vSCCs vs. cervical cancer had 128 DEGs. FIG. 14C. Within pan-SCC 5S subtype 5, vSCC vs skin had 11 DEGs. DEG criteria: linear regression, FDR multiple testing correction, Z<0.1. Principal component 1 and 2 for all samples within each pan-SCC 5S subtype which had >20 vSCC samples. Density plots on the outside of the scatter plots represent the PC1 (x-axis) or PC2 (y-axis) density for cancer types present in >20 samples for each pan-SCC 5S subtype.

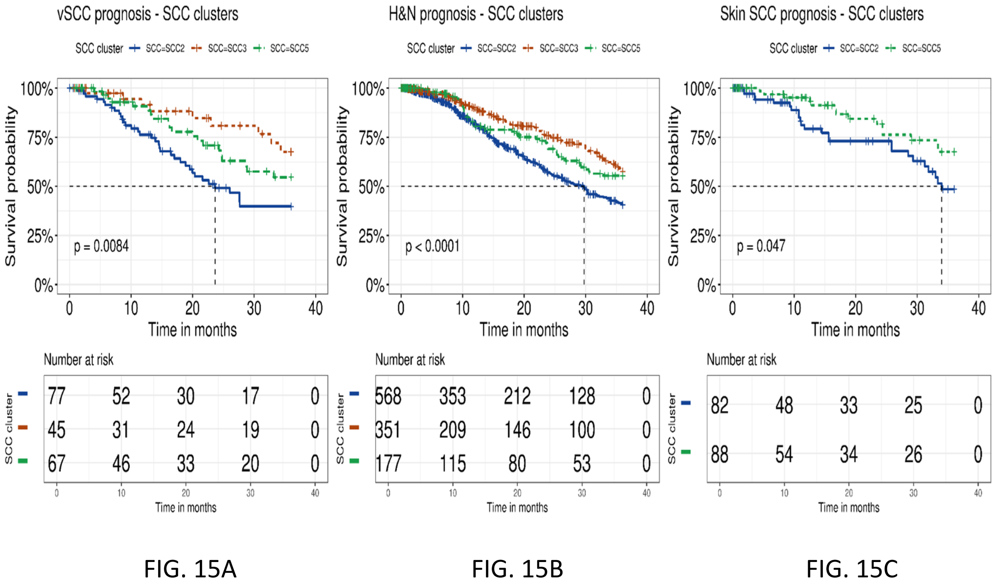

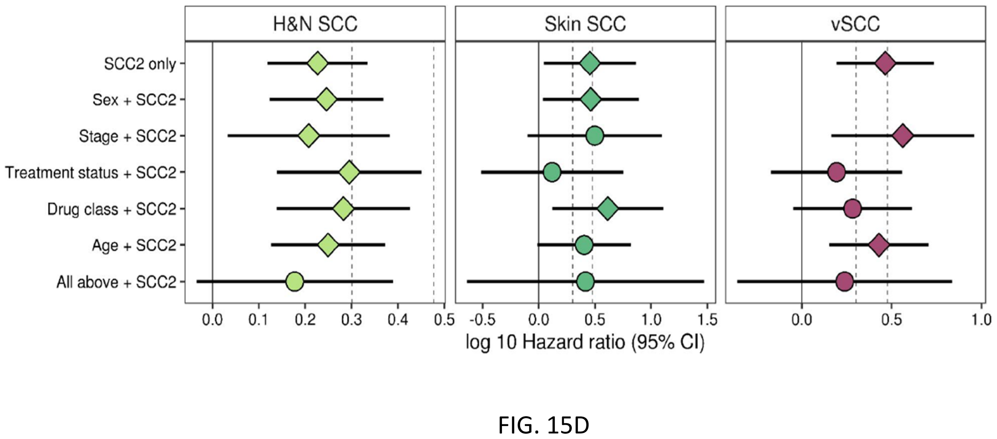

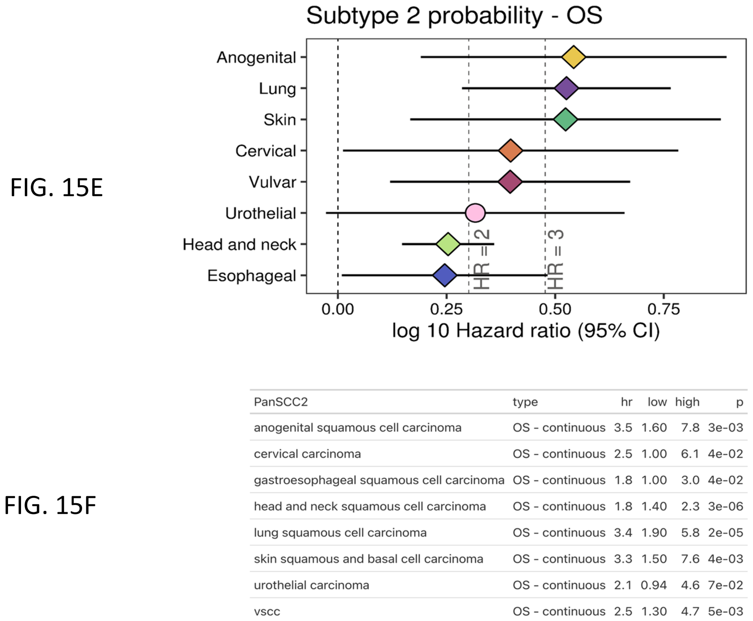

FIGS. 15A-15F. Survivability of selected SCCs stratified by pan-SCC 5S subtype. FIG. 15A Kaplan-Meier plots displaying overall survival probability for vSCC prognosis by 5 pan-SCC subtypes 2, 3, and 5. FIG. 15B. Kaplan-Meier plots displaying overall survival probability for H&N prognosis by 5-pan SCC subtypes 2, 3, and 5. FIG. 15C. Kaplan-Meier plots displaying overall survival probability for skin SCC prognosis by 5 pan-SCC subtypes 2 and 5. P-value on plot was determined using Log Rank Test. FIG. 15D. 5 pan-SCC subtype 2 probability was associated with OS after accounting for clinical covariates. Cox proportional hazards association with 5 pan-SCC subtype 2, or 5 pan-SCC subtype 2 probability within an additional covariate. "All above" refers to a multivariate model including stage, sex (if not vSCC), treatment status, and age. FIG. 15E. 5 pan-SCC subtype 2 survivability across SCC cancer type. FIG. 15F. Summary of 5 pan-SCC subtype 2 survivability analysis.
FIGS. 16A-16D. Using pan-SCC 5S subtype 2. FIG. 16A. Lung squamous cell split by treatment. FIG. 16B shows survivability of LUSC carboplatin + paclitaxel. FIG. 16C shows state and number at risk over time for subtype 2 positive and subtype 2 negative. FIG. 16D shows a table summarizing the survivability analysis subtype 2 across different regimens.
FIGS. 17A-17C. FIG. 17A shows H&N SCC split by treatment. FIG. 17B shows survivability probability of H&N chemotherapy based on subtype 2 status. FIG. 17C shows number at risk based on subtype 2 status.
FIGS. 18A-18C. FIG. 18A shows cervical SCC split by treatment. FIG. 18B shows survivability probability of cervical SCC cisplatin based on subtype 2 status. FIG. 18C shows number at risk based on subtype 2 status.
FIGS. 19A-19C. FIG. 19A. pan-SCC 5S clustering, shown by number of samples of different cancer types from TCGA. FIG. 19B. 5 pan-SCC clustering on training data set for reference. FIG. 19C. Number of samples grouped by HPV status for each 5 pan-SCC cluster.
FIGS. 20A-20D. FIG. 20A. PFI H&N prognosis based on 5 pan-SCC clusters subtype 2 and subtype 5. FIG. 20B. OS H&N prognosis based on 5 pan-SCC clusters subtype 2 and 5. FIG. 20C-20D. Number at risk vs time in months based on PFI (FIG. 20C) and OS (FIG. 20D).
FIGS. 21A, 21B, 21C, and 21D. FIG. 21A: pan-SCC 6S summary table by subtype. FIG. 21B: UMAP1 vs UMAP 2, pan-SCC 6S cluster profiles. FIG. 21C. SW for each pan-SCC 6S subtype.
FIG. 21D. Bar plot of proportion of each SCC type in pan-SCC 6S subtype.
FIG. 22. Characterization of pan-SCC 6S subtype profiles. UMAP1 vs UMAP2 of each SCC type by pan-SCC 6S subtype.
FIGS. 23A - FIG. 23C. FIG. 23A. Heatmap of Euclidean distance of pan-SCC 6S subtypes. FIG. 23B. Graphical representation of connectedness of pan-SCC 6S subtypes based on Euclidean distances. FIG. 23C. PC1 vs PC2 of pan-SCC 6S subtypes, sized based on sum of squares.
FIGS. 24A-24E. FIG. 24A. Specificity and sensitivity of 6 pan-SCC subtypes. FIG. 24B. Proportion of 6 pan-SCC subtypes by SCC type. FIG. 24C. Heatmap relating pan-SCC 5S subtypes and pan-SCC 6S subtypes, by SCC tissue type. FIG. 24D shows max score of different SCC types in model development. FIG. 24E shows the distribution of which SCC types were included in the model and which were not included in the model.
FIGS. 25A-25C. FIG. 25A. OS survival probability in anal SCC. FIG. 25B. OS survival probability in anal SCC stratified by 6 pan-SCC subtype. FIG. 25C. Hazard ratio of anal SCC over relevant variables.
FIGS. 26A-26C. FIG. 26A. OS survival probability in cervical SCC. FIG. 26B. OS survival probability in cervical SCC stratified by 6 pan-SCC subtype. FIG. 26C. Hazard ratio of cervical SCC over relevant variables.
FIGS. 27A-27C. FIG. 27A. OS survival probability in esophageal SCC. FIG. 27B. OS survival probability in esophageal SCC stratified by 6 pan-SCC subtype. FIG. 27C. Hazard ratio of esophageal SCC over relevant variables.
FIGS. 28A-28C. FIG. 28A. OS survival probability in H&N SCC. FIG. 28B. OS survival probability in H&N SCC stratified by 6 pan-SCC subtype. FIG. 28C. Hazard ratio of H&N SCC over relevant variables.
FIGS. 29A-29C. FIG. 29A. OS survival probability in lung SCC. FIG. 29B. OS survival probability in lung SCC stratified by 6 pan-SCC subtype. FIG. 29C. Hazard ratio of lung SCC over relevant variables.
FIGS. 30A-30C. FIG. 30A. OS survival probability in lung SCC. FIG. 30B. OS survival probability in lung SCC stratified by 6 pan-SCC subtype. FIG. 30C. Hazard ratio of lung SCC over relevant variables.
FIGS. 31A-31B. Applying model to TCGA database. FIG. 31A overall survival model scores across TCGA. FIG. 31B. Progression-free interval (PFI) across TCGA database.
FIG. 32. Schematic of an exemplary process in accordance with some embodiments of the disclosed subject matter.
FIG. 33. Schematic of a system for classifying cancer subtypes in accordance with some embodiments of the disclosed subject matter.

### DETAILED DESCRIPTION OF THE DISCLOSURE

### Overview

Some cancers are infrequently diagnosed and, thus, under-researched and difficult to treat. Rare cancers suffer from a lack of clinical trials, in many cases, based on the difficulty of recruiting participants. Described herein are systems for model-based classification of a specific cancer histological-type into subtypes.

A particular advantage of the disclosed methods is the ability to leverage large data sets to inform clinical, treatment, or trial recruiting decisions, especially for rare cancers or cancers with limited or no treatment options. By classifying a subject as having a particular pan-cancer subtype, it becomes possible to leverage a larger knowledge base, associated with molecularly similar cancers, to inform the above-described decisions. For instance, certain SCC subtypes are rare and difficult to treat, e.g., vulvar squamous cell carcinoma (vSCC). Certain vSCC tumors share molecular similarities with skin SCCs, which are more common and have more established treatment approaches. Therefore, understanding skin SCCs can be used to inform descriptions or treatment of a patient with vSCC, subsequent to classification by the disclosed methods.

In some embodiments, a pan-SCC cohort may include subjects diagnosed with SCC. Each subject in the pan-SCC cohort may be diagnosed with a variety of tissue-specific SCCs (e.g., a pan-SCC cohort can include subjects diagnosed with, e.g., anogenital, cervical, esophageal, head and neck, lung, skin, urothelial, colorectal, or vulvar squamous cell carcinomas).

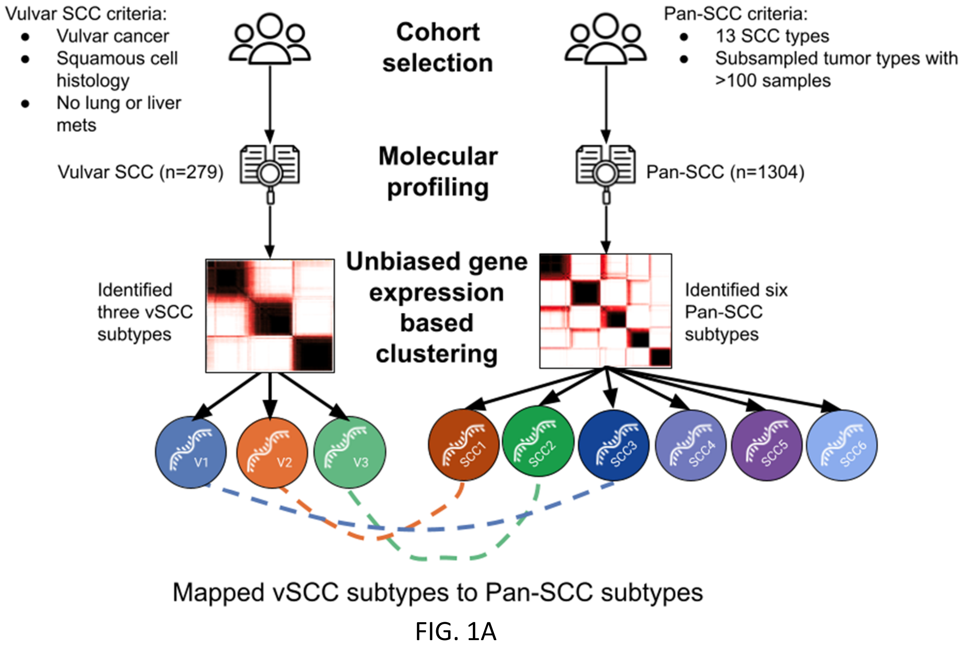

In certain embodiments of the disclosed methods and systems, a subject's cancer is first classified, based on molecular profile, in relation to other cancers of the same type, e.g., a vSCC tumor is classified in relation to the molecular profile of a cohort of other vSCC tumors. FIG. 1A shows an exemplary workflow of the disclosed methods. A cohort of a specific cancer class (e.g., vSCC) is analyzed to determine molecular profiles of subjects in the cohort; clustering is then used to identify cancer class subtypes (e.g., vSCC subtypes). A pan-cancer cohort (e.g., patients with 13 different types of SCC) is analyzed to determine molecular profiles of subjects in the cohort; clustering is then used to identify pan-cancer subtypes (e.g., pan-SCC subtypes). Cancer-specific subtypes may then be mapped to pan-cancer subtypes (e.g., vSCC subtypes may be mapped to pan-SCC subtypes). Trained algorithms can be used to classify subjects as having a cancer-specific subtype or a pan-cancer subtype.

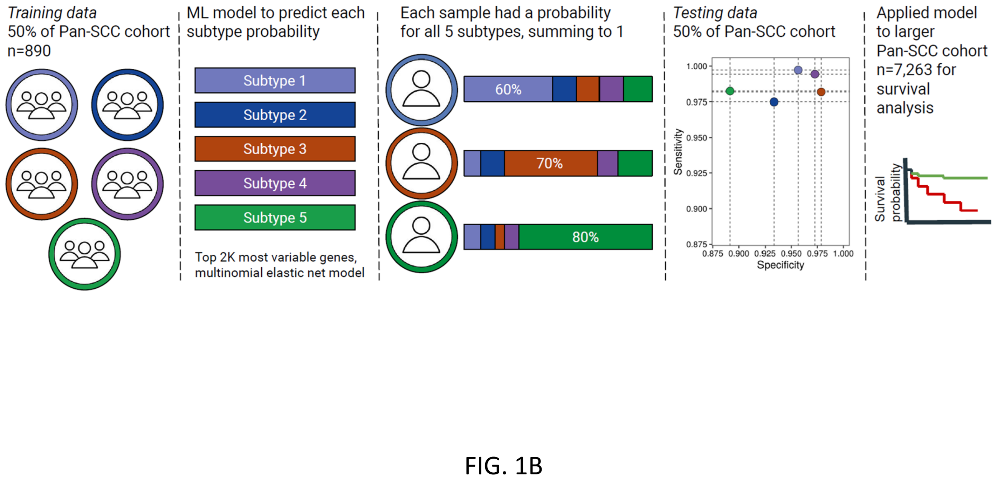

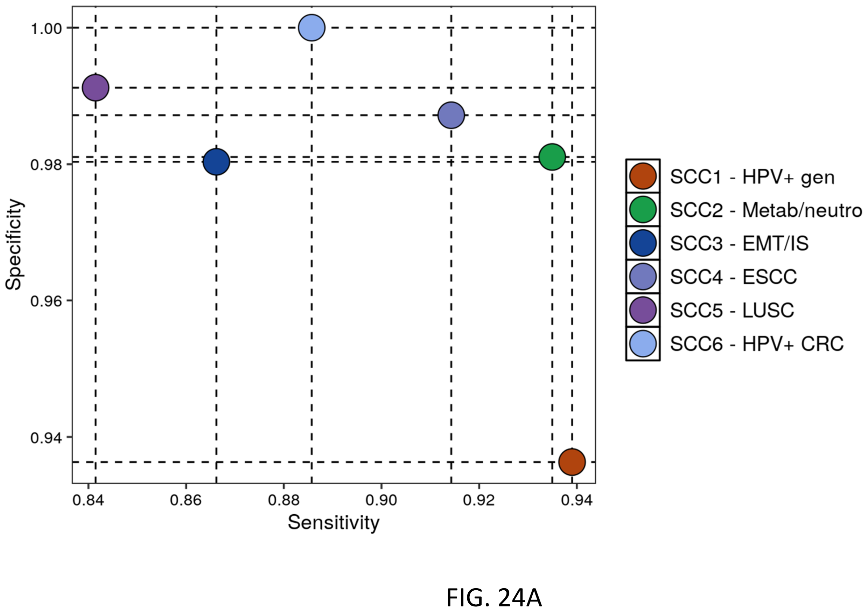

FIG. 1B shows an exemplary workflow to train an algorithm (e.g., machine learning model). Training data, comprising RNA sequencing samples from a subset of the larger cohort, is used to train a machine learning model to classify samples as a cancer subtype. The trained model may be tested for sensitivity and specificity and applied to a larger cohort for further analysis (*see,* e.g., FIG. 24A).

The various aspects of the subject invention are described with reference to the annexed drawings, wherein like reference numerals correspond to similar elements throughout the several views. It should be understood, however, that the drawings and detailed description relating thereto are not intended to limit the claimed subject matter to the particular form disclosed. Rather, the intention is to cover all modifications, equivalents, and alternatives falling within the spirit and scope of the claimed subject matter.

In the detailed description, reference is made to the accompanying drawings which form a part hereof, and in which is shown by way of illustration, specific embodiments in which the disclosure may be practiced. These embodiments are described in sufficient detail to enable those of ordinary skill in the art to practice the disclosure. It should be understood, however, that the detailed description and the specific examples, while indicating examples of embodiments of the disclosure, are given by way of illustration only and not by way of limitation. From this disclosure, various substitutions, modifications, additions, rearrangements, or combinations thereof within the scope of the disclosure may be made and will become apparent to those of ordinary skill in the art.

In accordance with common practice, the various features illustrated in the drawings may not be drawn to scale. The illustrations presented herein are not meant to be actual views of any particular method, device, or system, but are merely idealized representations that are employed to describe various embodiments of the disclosure. Accordingly, the dimensions of the various features may be arbitrarily expanded or reduced for clarity. In addition, some of the drawings may be simplified for clarity. Thus, the drawings may not depict all of the components of a given apparatus (e.g., device) or method. In addition, like reference numerals may be used to denote like features throughout the specification and figures.

The various illustrative logical blocks, modules, circuits, and algorithm acts described in connection with embodiments disclosed herein may be implemented as electronic hardware, computer software, or combinations of both. To clearly illustrate this interchangeability of hardware and software, various illustrative components, blocks, modules, circuits, and acts are described generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the embodiments of the disclosure described herein.

In addition, it is noted that the embodiments may be described in terms of a process that is depicted as a flowchart, a flow diagram, a structure diagram, or a block diagram. Although a flowchart may describe operational acts as a sequential process, many of these acts can be performed in another sequence, in parallel, or substantially concurrently. In addition, the order of the acts may be re-arranged. A process may correspond to a method, a function, a procedure, a subroutine, a subprogram, etc. Furthermore, the methods disclosed herein may be implemented in hardware, software, or both. If implemented in software, the functions may be stored or transmitted as one or more instructions or code on a computer-readable medium. Computer-readable media includes both computer storage media and communication media including any medium that facilitates transfer of a computer program from one place to another. It should be understood that any reference to an element herein using a designation such as "first," "second," and so forth does not limit the quantity or order of those elements, unless such limitation is explicitly stated. Rather, these designations may be used herein as a convenient method of distinguishing between two or more elements or instances of an element. Thus, a reference to first and second elements does not mean that only two elements may be employed there or that the first element must precede the second element in some manner. Also, unless stated otherwise a set of elements may comprise one or more elements.

Hereafter, unless indicated otherwise, the following terms and phrases will be used in this disclosure as described.

As used in this specification and the claims, the singular forms "a," "an," and "the" include plural forms unless the context clearly dictates otherwise. For example, the term "a polypeptide fragment" should be interpreted to mean "one or more a polypeptide fragment" unless the context clearly dictates otherwise. As used herein, the term "plurality" means "two or more."

As used herein, "about," "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean up to plus or minus 10% of the particular term and "substantially" and "significantly" will mean more than plus or minus 10% of the particular term.

As used herein, the terms "include" and "including" have the same meaning as the terms "comprise" and "comprising." The terms "comprise" and "comprising" should be interpreted as being "open" transitional terms that permit the inclusion of additional components further to those components recited in the claims. The terms "consist" and "consisting of" should be interpreted as being "closed" transitional terms that do not permit the inclusion of additional components other than the components recited in the claims. The term "consisting essentially of" should be interpreted to be partially closed and allowing the inclusion only of additional components that do not fundamentally alter the nature of the claimed subject matter.

As used herein, the term "subject" may be used interchangeably with the term "patient" or "individual" and may include an "animal" and in particular a "mammal." Mammalian subjects may include humans and other primates, domestic animals, farm animals, and companion animals such as dogs, cats, guinea pigs, rabbits, rats, mice, horses, cattle, cows, and the like.

As used herein a "subject sample" or a "biological sample" from the subject refers to a sample taken from the subject, such as, but not limited to a tissue sample (for example fat, muscle, skin, neurological, tumor, biopsy, etc.) or fluid sample (for example, saliva, blood, serum, plasma, urine, stool, cerebrospinal fluid, etc.), and or cells, cultured cells (for example, organoids) or sub-cellular structures such as vesicles and exosomes.

As used herein, the terms "component," "system" and the like are intended to refer to a computer-related entity, either hardware, a combination of hardware and software, software, or software in execution. For example, a component may be, but is not limited to being, a process running on a processor, a processor, an object, an executable, a thread of execution, a program, and/or a computer. By way of illustration, both an application running on a computer and the computer can be a component. One or more components may reside within a process and/or thread of execution and a component may be localized on one computer and/or distributed between two or more computers or processors.

The word "exemplary" is used herein to mean serving as an example, instance, or illustration. Any aspect or design described herein as "exemplary" is not necessarily to be construed as preferred or advantageous over other aspects or designs.

Furthermore, the disclosed subject matter may be implemented as a system, method, apparatus, or article of manufacture using programming and/or engineering techniques to produce software, firmware, hardware, or any combination thereof to control a computer or processor-based device to implement aspects detailed herein. The term "article of manufacture" (or alternatively, "computer program product") as used herein is intended to encompass a computer program accessible from any computer-readable device, carrier, or media. For example, computer readable media can include but are not limited to magnetic storage devices (such as hard disk, floppy disk, magnetic strips), optical disks (such as compact disk (CD), digital versatile disk (DVD)), smart cards, and flash memory devices (such as card, stick). Additionally, it should be appreciated that a carrier wave can be employed to carry computer-readable electronic data such as those used in transmitting and receiving electronic mail or in accessing a network such as the Internet or a local area network (LAN). Transitory computer-readable media (carrier wave and signal based) should be considered separately from non-transitory computer-readable media such as those described above. Of course, those skilled in the art will recognize many modifications may be made to this configuration without departing from the scope or spirit of the claimed subject matter.

Unless indicated otherwise, while the disclosed system is used for many different purposes (such as data collection, data analysis, data display, treatment, research, etc.), in the interest of simplicity and consistency, the overall disclosed system will be referred to hereinafter as "the disclosed system".

As used herein, the term "clinical data" refers to information related to a patient or a cohort subject that is typically obtained by questioning the subject, observing the subject, or testing the subject. Exemplary clinical data include, but are not limited to physical characteristic (e.g., sex, height, weight, age, overall health, smoking history, history of transmissible disease, e.g., human papillomavirus (HPV) infection, etc.), medical history, current and past diagnosis, current and past treatment regimens administered, patient compliance, treatment outcomes (for example, response to treatment), imaging analysis such as x-rays, CT-scans, facial imaging, and body movement recordings, physical conditions, changes, etc.

In one example, the invention disclosed here may be a system, other class of device, and/or method to help a medical provider make clinical decisions based on a combination of molecular and clinical data, which may include comparing the molecular and clinical data of a patient to an aggregated data set of molecular and/or clinical data from multiple patients (e.g., a cohort of subjects) and/or a knowledge database (KDB) of clinicogenomic data. Additionally, the invention disclosed here may be used to capture, ingest, cleanse, structure, and combine robust clinical data and detailed molecular data to determine the significance of correlations, patterns and trends to generate reports for physicians, analyze or confirm the accuracy of a diagnosis, predict the likelihood that a patient responds to a specific treatment, recommend or discourage specific treatments for a patient, support biomarker discovery, bolster clinical research efforts, monitor treatment and dosing decisions, expand indications of use for treatments currently in market and clinical trials, and expedite federal or regulatory body approval of treatment compounds. In one example, the invention disclosed here may help academic medical centers, pharmaceutical companies and community providers improve care options and treatment outcomes for patients, especially patients experiencing any psychiatric disorders or illnesses, including, but not limited to squamous cell carcinomas including SCC in the lung, head and neck, skin, cervical, urothelial, esophageal, and anogenital, including anal, penile, and vulvar.

The terms "subject" and "patient" are used interchangeably herein. The subject is desirably a human subject, although it is to be understood that the methods described herein are effective with respect to all vertebrate species, which are intended to be included in the term "subject." Accordingly, a "subject" can include a human subject for medical purposes, such as for the treatment of an existing condition or disease or the prophylactic treatment for preventing the onset of a condition or disease, or an animal subject for medical, veterinary purposes, or developmental purposes. Suitable animal subjects include mammals including, but not limited to, primates, e.g., monkeys, apes, and the like; bovines, *e.g.,* cattle, oxen, and the like; ovines, *e.g.,* sheep and the like; caprines, e.g., goats and the like; porcines, *e.g.,* pigs, hogs, and the like; equines, *e.g.,* horses, donkeys, zebras, and the like; felines, including wild and domestic cats; canines, including dogs; lagomorphs, including rabbits, hares, and the like; and rodents, including mice, rats, and the like. Further, a "subject" can include a patient diagnosed with or suspected of having a condition or disease, such as a cancer.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness such as a cancer, either SCC or not SCC, e.g., a specific treatment or drug, pattern of dosing, etc.

As used herein, the terms "control," "control sample," "reference," "reference sample," "normal," and "normal sample" describe a sample from a non-diseased tissue. In some embodiments, such a sample is from a subject that does not have a particular condition (*e.g.*, diagnosed cancer). In other embodiments, such a sample is an internal control from a subject, *e.g*., who may or may not have the particular disease or disorder and is from a pre-treatment sample from the subject. For example, where a blood or saliva sample is obtained from a subject diagnosed with one or more psychiatric disorders, an internal control sample may be obtained from the subject prior to any treatment. The pre-treatment sample may show, for example and elevated level of expression from one or more genes. After treatment, another sample may be analyzed, to determine whether the treatment alters expression levels. Accordingly, a reference sample can be obtained from the subject or from a database, *e.g*., from a second subject.

As used herein "molecular data" includes information such as the sequence and/or amount (*e.g*., expression level, or duplication/deletion information) of one or more proteins, DNA, or RNA samples of a subject, a control subject, or a cohort. By way of example but not by way of limitation, in some embodiments, molecular data includes DNA sequence information including but not limited to whole genome, whole exome genetic data, single nucleotide variants (SNV), insertion/deletions (indels), copy number variation (CNV), fusion variants, RNA expression data (including miRNA expression), microbiome information, haplotypes or alleles information including star alleles, haplotype groups or diplotypes including star allele combinations, mass array data, microarray data. Whole exome genetic data pertaining to any of the exons in the human genome may further include intronic regions targeted, for example, by intron-specific probes spiked into a whole exome panel. Molecular data as used herein also includes targeted panels of DNA or RNA data (including sequence data and/or expression level data), and targeted panels of protein data. By way of example but not by way of limitation, a targeted panel includes an assay designed for evaluating or analyzing only specific genetic sequences such as specific genes, parts of genes, or specific non-coding sequences (e.g., introns or promoter regions), or specific proteins, as opposed to whole genome analysis for example. Molecular data may be obtained by methods well known in the art; such methods are not intended to be limiting. By way of example, in some embodiments, molecular data is derived from a multi-gene panel sequencing reaction, and comprises a plurality of nucleic sequences obtained from one or more of whole exome sequence data, mass array data, sequenced data from one or more introns, and sequence data from one or more gene regulatory regions.

For example, the methods and systems described herein may be used on information generated from next generation sequencing (NGS) techniques. NGS involves using specialized equipment such as a next generation gene sequencer, which is an automated instrument that determines the order of nucleotides in DNA and RNA. The instrument reports the sequences as a string of letters, called a read, which the analyst may compare to one or more reference genomes of the same genes. A reference genome may be compared to a library of normal and variant gene sequences associated with certain conditions. In one exemplary embodiment, extracted DNA or RNA from blood, saliva, biopsy, or other biological patient samples are single- or paired-end sequenced using an NGS platform, such as a platform offered by Illumina. The DNA or RNA may be extracted from cells in the specimen or may be cell-free. The subject from whom the sample was collected may have been diagnosed with cancer. The results of sequencing (herein, the "raw sequencing data") may be passed through a bioinformatics pipeline where the raw sequencing data is analyzed. The raw sequencing data may pertain to a combination of every exon and selected introns in the human genome, another set of targeted genomic regions, or whole genome. After sequencing information is run through the bioinformatics pipeline, it may be evaluated for quality control, such as through an automated quality control system. If the sample does not pass an initial quality control step, it may be manually reviewed. If the sample passes an automated quality control system or is manually passed, an alert may be published to a message bus that is configured to listen for messages from quality control systems. This message may contain sample identifiers, as well as the location of BAM files. A BAM file (.bam) is the binary version of a SAM file. A SAM file (.sam) is a tab-delimited text file that contains sequence alignment data (such as the raw sequencing data). When a message is received, a service may be triggered to evaluate the sequencing data for pharmacogenomics factors.

As used herein, the term "BAM File" or "Binary file containing Alignment Maps" refers to a file storing sequencing data aligned to a reference sequence (*e.g.*, a reference genome or exome). In some embodiments, a BAM file is a compressed binary version of a SAM (Sequence Alignment Map) file that includes, for each of a plurality of unique sequence reads, an identifier for the sequence read, information about the nucleotide sequence, information about the alignment of the sequence to a reference sequence, and optionally metrics relating to the quality of the sequence read and/or the quality of the sequence alignment. While BAM files generally relate to files having a particular format, for simplicity they are used herein to simply refer to a file, of any format, containing information about a sequence alignment, unless specifically stated otherwise.

BAM files can be generated by aligning raw molecular data to a reference genome. For example, raw molecular data can be stored in BCL, FASTA, and/or FASTQ file formats. A suitable process can align the raw molecular data to a human reference sequence and generate aligned sequence reads. The aligned sequence reads can be stored in SAM and/or BAM file formats.

As used herein, the term "sequencing probe" refers to a molecule that binds to a nucleic acid with affinity that is based on the expected nucleotide sequence of the RNA or DNA present at that locus.

As used herein, the term "targeted panel" or "targeted gene panel" refers to a combination of probes for sequencing (*e.g.,* by next-generation sequencing) nucleic acids present in a biological sample from a subject (*e.g.,* a saliva or a blood sample), selected to map to one or more loci of interest on one or more chromosomes. In some embodiments, the loci are informative for cancer diagnosis.

As used herein, the term, "reference exome" refers to any sequenced or otherwise characterized exome, whether partial or complete, of any tissue from any organism or pathogen that may be used to reference identified sequences from a subject. Typically, a reference exome will be derived from a subject of the same species as the subject whose sequences are being evaluated. Example reference exomes used for human subjects as well as many other organisms are provided in the on-line genome browser hosted by the National Center for Biotechnology Information ("NCBI"). An "exome" refers to the complete transcriptional profile of an organism or pathogen, expressed in nucleic acid sequences. As used herein, a reference sequence or reference exome often is an assembled or partially assembled exomic sequence from an individual or multiple individuals. In some embodiments, a reference exome is an assembled or partially assembled exomic sequence from one or more human individuals. The reference exome can be viewed as a representative example of a species' set of expressed genes. In some embodiments, a reference exome comprises sequences assigned to chromosomes.

As used herein, the term "reference genome" refers to any sequenced or otherwise characterized genome, whether partial or complete, of any organism or pathogen that may be used to reference identified sequences from a subject. Exemplary reference genomes used for human subjects as well as many other organisms are provided in the on-line genome browser hosted by the National Center for Biotechnology Information ("NCBI") or the University of California, Santa Cruz (UCSC). As used herein, "cancer" refers to any one or more of a wide range of benign growths or malignant tumors, including those that are capable of invasive growth and metastases through a human or animal body or a part thereof, such as, for example, via the lymphatic system and/or the blood stream. As used herein, the term "tumor" includes benign growths, malignant tumors and solid growths. Typical cancers include but are not limited to carcinomas, lymphomas, or sarcomas, such as, for example, skin cancer, e.g., SCC, ovarian cancer, colon cancer, breast cancer, pancreatic cancer, lung cancer, prostate cancer, urinary tract cancer, uterine cancer, acute lymphatic leukemia, Hodgkin's disease, small cell carcinoma of the lung, melanoma, neuroblastoma, glioma, and soft tissue sarcoma of humans. A "cancer" refers to a singular type of cancer (e.g., squamous cell carcinoma or, more particularly, vulvar squamous cell carcinoma). This may refer to cancers with a common tissue location (e.g., cancer in the lung or skin). A tissue-specific cancer may exist entirely in one tissue, or it may have metastasized to additional locations.

### Methods

The disclosed methods may be used to characterize a subject's cancer as belonging to a particular subtype based on molecular or other characteristics. The characterized subtype may include cancers for which there are established and/or effective treatment protocols. Thus, the disclosed methods may offer new treatment options for subjects with cancers thought to be untreatable or for subjects with rare cancers for which there are limited treatment options. Cancers with "limited treatment options" refers to cancers with established treatments that are known to be partially effective (e.g., not prevent symptoms, not prevent progression of the disease, lead to destructive side effects, etc.) or palliative in nature. Limited treatment options may also refer to a status where few treatments are established (e.g., approved drugs, established treatment regimens, etc.). "No treatment options" refers to a lack of any established treatments (e.g., no approved drugs, little evidence of effective treatments, etc.).

A subject may be diagnosed with a cancer with few or no treatment options. The disclosed methods may classify a subject's cancer as having a subtype which is molecularly similar to another group of cancers with improved treatment options as compared to the subject's cancer, as originally diagnosed. Improved treatment options refers to treatments that have improved outcomes, e.g., increased likelihood of response in a subject, as measured by known outcomes in cohorts of subjects with the molecularly similar cancer, compared to treatments for the subject's diagnosed cancer. For example, a subject may be diagnosed with the rare cancer vulvar squamous cell carcinoma affecting ~6500 subjects in the U.S. annually. The disclosed methods may classify the subject's cancer as belonging to a subtype of SCC that is more similar to skin squamous cell carcinoma that may be treated, e.g., with an immunotherapy, e.g., ICI. In one example, the disclosed methods have determined which subtypes of SCC are predicted to have better response to a particular therapy and/or a better prognosis than another subtype and the disclosed methods can determine which subtype a patient is most likely to have.

Further, the disclosed methods may be used to enroll a subject in a clinical trial based on the subtyping of the cancer; molecular-based enrollment instead of diagnosis-based enrollment. For example, a subject may be diagnosed with a rare cancer, for which there are no clinical trials enrolling subjects. Alternatively, there may be clinical trials enrolling subjects for a promising therapeutic, e.g., an immunotherapy, but that are not enrolling patients with the rare cancer. The disclosed methods may be used to classify the subject's cancer as molecularly similar to the enrolling patient population to design clinical trials to include subjects with the rare cancer. Thus, the subject may be eligible to receive the promising therapeutic.

In an aspect of the current disclosure, methods are provided. In some embodiments, the methods comprise obtaining, with a computer system, sequencing read data collected from a sample from a cancer of a subject, the read data comprising RNA sequencing data; classifying, with the computer system, the cancer as a subtype of cancer, using a trained machine learning algorithm, wherein the subtype of cancer comprises a plurality of cell proliferative diseases with common characteristics wherein the common characteristics comprise similar molecular profiles, wherein the trained machine learning algorithm is trained on a data set of sequencing read data collected from a cohort of subjects suffering from cancer.

A "cancer subtype," as used in the context of this disclosure, refers to a group of proliferative cell diseases with common characteristics. A cancer subtype may be single-cancer or single-tissue subtypes (e.g., vSCC subtype, lung cancer subtype). A cancer subtype may be a pan-cancer subtype. A pan-cancer subtype refers to a common characteristic profile that is shared amongst multiple types of cancer. For instance, a pan-cancer subtype may include cancers from multiple tissue types (e.g., a pan-cancer subtype can include vSCC and skin SCC).

"Common characteristics" may refer to similar molecular profiles (e.g., gene expression, genetic mutations, etc.). Common characteristics may also refer to similar comorbidities or shared behavioral patterns. For instance, common characteristics may refer to HPV status, or lifestyle factors, e.g., smoking, etc. HPV status may be determined by methods known in the art, e.g., standard laboratory testing for viral nucleic acids. Lifestyle factors may be determined by, e.g., a history and physical examination performed by a physician and included in medical records, e.g., electronic medical records. Subjects with a shared cancer subtype may have or be predicted to have similar phenotypes, prognostics, and responses to treatment.

As used herein, "read data" refers to sequencing read data. The sequencing read data may be from a next generation sequencing reaction and may comprise RNA sequencing or DNA sequencing, methods for performing both of which are routine in the art and can be performed using a commercially available platform. In some embodiments, the methods may comprise obtaining sequencing data that is pre-processed and comprises RNA expression levels. In other embodiments, the methods comprise performing RNA and, optionally, DNA sequencing, processing the read data from the RNA and, optionally, DNA sequencing reactions, and proceeding with the disclosed methods using the sequenced data.

The disclosed methods comprise classifying cancer as a subtype of cancer using a trained machine learning algorithm. As used herein, "classifying" refers to grouping or associating related entities, e.g., grouping or associating cancers based on similar characteristics, e.g., similar molecular profiles.

The methods may further comprise administering a therapy to the subject, e.g., an immunotherapy, a chemotherapy, a radiation therapy, a hormone therapy, or a surgical therapy. A "therapeutically effective amount" of a therapy, e.g., a therapeutically effective amount of a chemotherapy, refers to an amount of the therapy that is effective for improving one or more sign or symptom in the subject. In one example, the subject is suffering from cancer and a therapeutically effective amount of a therapy is administered to the subject causing one or more sign or symptom of the cancer, e.g., tumor burden, tumor size, number of tumors, grade of tumor, prognosis of disease, etc., to be improved.

### Model-based classification of cancer subtypes

An algorithm can be trained to classify subjects as having a cancer subtype. An algorithm can be trained based on training data comprising a cohort of subjects, each subject being diagnosed with a cell proliferative disorder. A subject in the cohort would include sequencing data and a corresponding subtype the subject belongs to. The training data may also include patient health information, such as age, sex, demographic information, and comorbidities, e.g., HPV status, smoking history, or other etiologies.

A trained algorithm would thus be able to receive subject sequence information, and optionally receive subject health information, and be used to classify the subject as having a cancer subtype.

In some embodiments, the trained algorithm produces a predicted cancer subtype. In some embodiments, the trained algorithm produces a predicted cancer subtype and a corresponding confidence in the prediction. In some embodiments, the trained algorithm produces multiple predicted cancer subtypes and the likelihood a subject belongs to each subtype. In some embodiments, the trained algorithm produced a predicted score for each cancer subtype.

Any suitable algorithm may be used, including a neural network, artificial intelligence, random forest/random trees, or Bayesian classifiers. An algorithm may be trained through any suitable method, including but not limited to linear regression, logistic regression, ridge regression, lasso, or ElasticNet.

The disclosed subtypes may be broadly applicable as predictors of survival in multiple different types of cancer. Pan-cancer S6 subtype 5 ("model 5") is associated with overall survival in head and neck SCC, esophageal SCC, anal canal SCC, and lung SCC. Similarly, pan-cancer subtype 1 is associated with overall survival in cervical SCC, esophageal SCC, head and neck SCC, bladder SCC, and penile SCC.

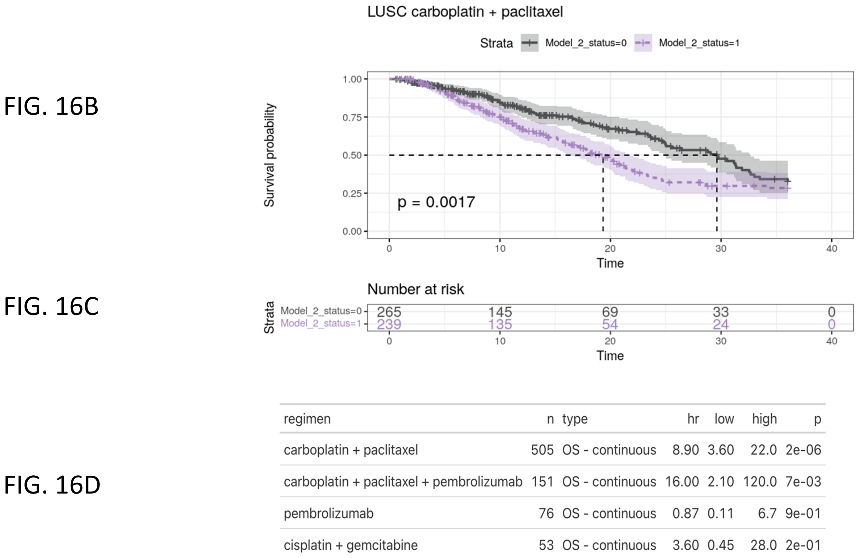

Further, the disclosed methods may be predictive of overall survival after treatment with chemotherapeutic drugs. Referring now to FIG. 16B, subjects that are positive for the pan-cancer S5 subtype 2 (subtype 2), have significantly lower overall survival probability compared to subjects that are not positive for the subtype 2 signature.

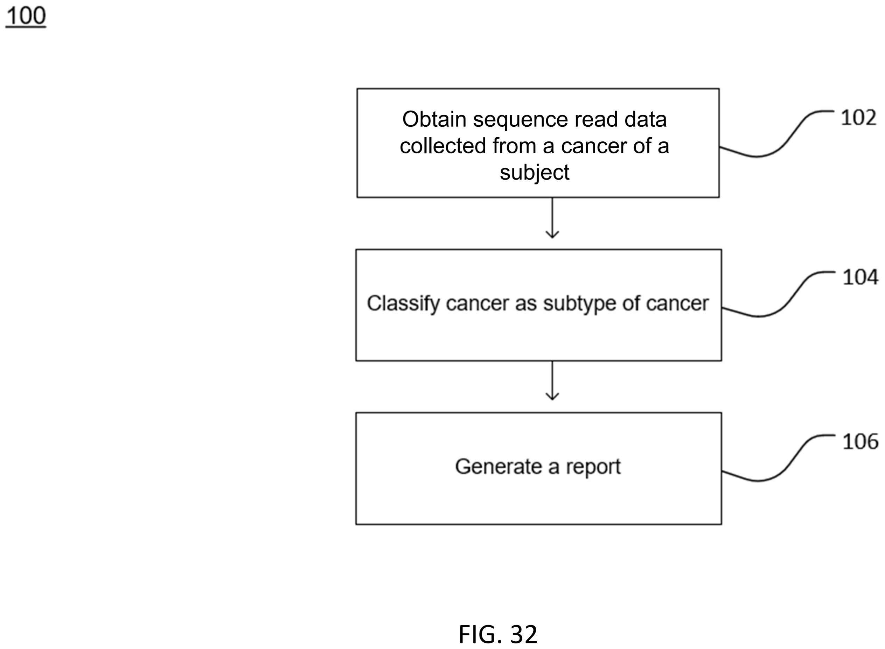

FIG. 32 shows an example process 100 for classifying a subject based on sequence information. At 102, process 100 can access subject sequencing data comprising RNA sequencing data. The sequencing data may indicate the expression level of a plurality of genes in the subject. Patient health information (e.g., information indicating sex, HPV status, history of smoking, etc.).

At 104, process 100 can analyze the biomarker data using a trained machine learning algorithm to classify the subject as having a subtype of cancer. The trained machine learning algorithm is accessed with a computer system. Accessing the trained machine learning algorithm may include accessing model parameters (e.g., weights, biases, or both) that have been optimized or otherwise estimated by training the machine learning algorithm on training data. In some instances, retrieving the machine learning algorithm can also include retrieving, constructing, or otherwise accessing the particular machine learning algorithm or model architecture to be implemented. For instance, data pertaining to the layers in a neural network architecture (e.g., number of layers, type of layers, ordering of layers, connections between layers, hyperparameters for layers); the leaves, nodes, and branches in a decision tree model; or the like, may be retrieved, selected, constructed, or otherwise accessed.

In general, the sequencing data can be input to one or more trained machine learning algorithms, models, or programs to generate feature data. In still other instances, the biomarker data can be input to one or more artificial intelligence (AI) algorithms, models, or programs to generate the predicted and/or estimated absorbed radiation dose. The trained AI or machine learning algorithm, model, or program can implement a linear regression model or a tree-based model (e.g., a decision tree, a random forest model, etc.). Additionally or alternatively, the AI or machine learning algorithm, model, or program can implement a neural network, a generative adversarial network (GAN), a large language model (LLM), a support vector machine, a naive Bayes classifier, a nearest neighbor model, a gradient boosting model (e.g., a gradient boosting machine (GBM), an XGBoost model, an AdaBoost model, etc.), or the like.

The trained machine learning algorithm may be trained on labeled data collected from a plurality of subjects. In general, the training data can include expression and/or expression levels of one or more signature genes, such as one or more of the signature genes described in the present disclosure, e.g., in Tables 8-13. In some embodiments, the training data may include data that have been labeled, e.g., labeled with a cancer subtype, lifestyle factors, comorbidities, e.g., HPV status.

The method can include assembling training data from the sequencing data and/or patient health data using a computer system. This step may include assembling the sequencing data and/or into an appropriate data structure on which the machine learning algorithm, model, or program can be trained. Assembling the training data may include assembling sequencing data, subject health data, and other relevant data. For instance, assembling the training data may include generating labeled data and including the labeled data in the training data. Labeled data may include sequencing data or other relevant data that have been labeled as belonging to, or otherwise being associated with, one or more different classifications or categories.

In some embodiments, computing device 204 and/or server 216 can be any suitable computing device or combination of devices, such as a desktop computer, a laptop computer, a smartphone, a tablet computer, a wearable computer, a server computer, a virtual machine being executed by a physical computing device, etc. As described herein, system 200 can present information about the characterized protein to a user (e.g., a researcher and/or a physician).

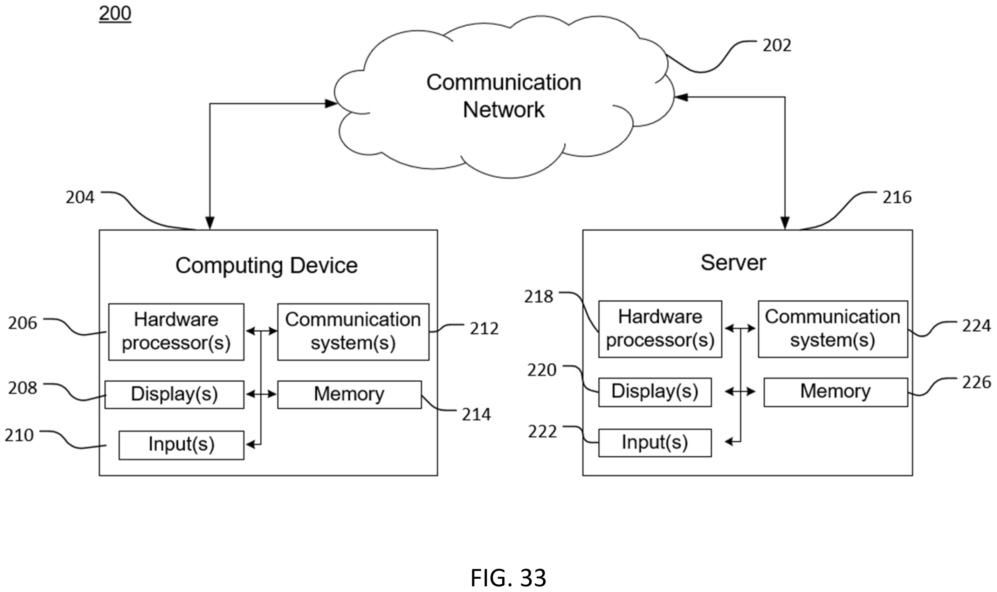

In some embodiments, communication network 202 can be any suitable communication network or combination of communication networks. In some embodiments, communication network 202 can be any suitable communication network or combination of communication networks. For example, communication network 202 can include a Wi-Fi network (which can include one or more wireless routers, one or more switches, etc.), a peer-to-peer network (e.g., a Bluetooth network), a cellular network (e.g., a 4G network, a 5G network, etc., complying with any suitable standard, such as CDMA, GSM, LTE, LTE Advanced, WiMAX, etc.), a wired network, etc. In some embodiments, communication network 202 can be a local area network, a wide area network, a public network (e.g., the Internet), a private or semi-private network (e.g., a corporate or university intranet), any other suitable type of network, or any suitable combination of networks. Communications links shown in FIG. 33 can each be any suitable communications link or combination of communications links, such as wired links, fiber optic links, Wi-Fi links, Bluetooth links, cellular links, etc.

FIG. 33 additionally shows an example of hardware that can be used to implement computing device 204 and server 216 in accordance with some embodiments of the disclosed subject matter. In some embodiments, computing device 204 can be used to execute one or more set of instructions to identify a cancer subtype. In other embodiments, computing device 204 can be used to identify a cancer subtype and information regarding the cancer subtype, associated characteristics, and molecular profile. In still other embodiments, computing device 204 can be used to identify a recommended treatment regimen.

As shown in FIG. 33, computing device 204 can include one or more hardware processor 206, one or more displays 208, one or more inputs 210, one or more communications 212, and/or memory 214. In some embodiments, processor 206 can be any suitable hardware processor or combination of processors, such as central processing unit, a graphics processing unit, etc. In some embodiments, display 208 can include any suitable display devices, such as a computer monitor, a touchscreen, a television, etc. In some embodiments, inputs 210 can include any suitable input device and/or sensors that can be used to receive user input, such as a keyboard, a mouse, a touchscreen, a microphone, etc.

In some embodiments, communication systems 212 can include any suitable hardware, firmware, and/or software for communicating information over communication network 202 and/or any other suitable communication networks. For example, communications systems 212 can include one or more transceivers, one or more communication chips and/or chip sets, etc. In a more particular example, communications systems 212 can include hardware, firmware and/or software that can be used to establish a Wi-Fi connection, a Bluetooth connection, a cellular connection, an Ethernet connection, etc.

In some embodiments, memory 214 can include any suitable storage device or devices that can be used to store instructions, values, etc., that can be used, for example, by processor 206 to present content using display 208, to communicate with server 216 via communications system(s) 212, etc.

Memory 214 can include any suitable volatile memory, non-volatile memory, storage, or any suitable combination thereof. For example, memory 214 can include RAM, ROM, EEPROM, one or more flash drives, one or more hard disks, one or more solid state drives, one or more optical drives, etc. In some embodiments, memory 214 can have encoded thereon a computer program for controlling operation of computing device 204. In such embodiments, processor 206 can execute at least a portion of the computer program to present content (e.g., images, user interfaces, graphics, tables, etc.), receive content from server 216, transmit information to server 216, etc.

In some embodiments, server 216 can include a processor 218, a display 220, one or more inputs 222, one or more communications systems 224, and/or memory 226. In some embodiments, processor 218 can be any suitable hardware processor or combination of processors, such as a central processing unit, a graphics processing unit, etc. In some embodiments, display 220 can include any suitable display devices, such as a computer monitor, a touchscreen, a television, etc. In some embodiments, inputs 222 can include any suitable input devices and/or sensors that can be used to receive user input, such as a keyboard, a mouse, a touchscreen, a microphone, etc.

In some embodiments, communications systems 224 can include any suitable hardware, firmware, and/or software for communicating information over communication network 202 and/or any other suitable communication networks. For example, communications systems 224 can include one or more transceivers, one or more communication chips and/or chip sets, etc. In a more particular example, communications systems 224 can include hardware, firmware and/or software that can be used to establish a Wi-Fi connection, a Bluetooth connection, a cellular connection, an Ethernet connection, etc.

In some embodiments, memory 226 can include any suitable storage device or devices that can be used to store instructions, values, etc., that can be used, for example, by processor 218 to present content using display 220, to communicate with one or more computing devices 204, etc. Memory 226 can include any suitable volatile memory, non-volatile memory, storage, or any suitable combination thereof. For example, memory 226 can include RAM, ROM, EEPROM, one or more flash drives, one or more hard disks, one or more solid state drives, one or more optical drives, etc. In some embodiments, memory 226 can have encoded thereon a server program for controlling operation of server 216. In such embodiments, processor 218 can execute at least a portion of the server program to transmit information and/or content (e.g., results of a tissue identification and/or classification, a user interface, etc.) to one or more computing devices 204, receive information and/or content from one or more computing devices 204, receive instructions from one or more devices (e.g., a personal computer, a laptop computer, a tablet computer, a smartphone, etc.).

In some embodiments, any suitable computer readable media can be used for storing instructions for performing the functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

### Clustering cancer subtypes

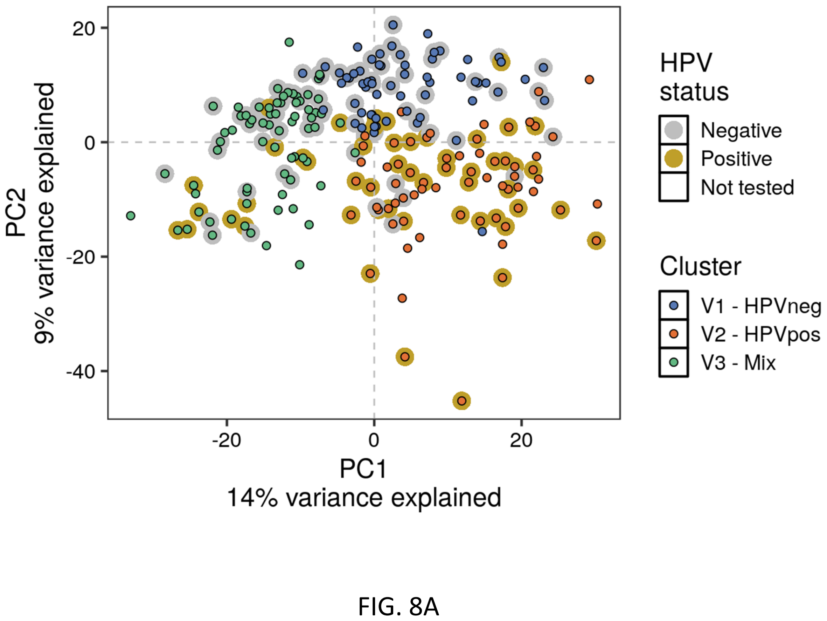

The inventor performed parallel analyses using data from cohorts of subjects that were (1) all diagnosed with the same type of cancer, vulvar squamous cell carcinoma (vSCC) and (2) diagnosed with a variety of different cancers. The inventor discovered that there was variability of molecular profiles within the vSCC cohort which, by reducing the dimensionality of the data, can be expressed as three subtypes vSCC - subtypes 1, 2, and 3 (FIGs. 1A and 8A, FIG. 8A shows the principal component analysis for the clustering of the vSCC subtypes). The inventor analyzed molecular data from a cohort of subject diagnosed with squamous cell carcinoma and discovered that the cohort could be expressed as 5 or 6 different clusters, depending on the cohort data, where data from a larger cohort resulted in 6 different clusters (FIG. 1A). Surprisingly, the vSCC subtypes each generally corresponded to certain pan-SCC subtypes and different SCCs did not cluster by tissue source, e.g., lung, skin, etc. (FIG. 13A).

In some embodiments, cohorts of subjects are clustered to identify cancer subtypes. Any suitable clustering algorithm may be used. The clustering algorithm may be biased or unbiased. Clustering algorithms can include, but are not limited to, k-means clustering, hierarchical clustering, centroid models, Gaussian models, affinity propagation, DBSCAN, density-based clustering, and spectral clustering.

Clustering algorithms may be used on a cohort of subjects with a common cancer; this would result in cancer-specific subtypes. Additionally or alternatively, clustering algorithms may be used on a cohort of subjects diagnosed with multiple cancers: this would result in pan-cancer subtypes.

The terms "cluster" and "subtype" can be used interchangeably. A cancer-specific subtype, as used herein, may be referred to as (cancer name)-subtype (e.g., vulvar SCC is notated as vSCC-subtype). A pan-cancer subtype may be notated as pan-cancer NS, where N is the number of subtypes (e.g., pan-SCC 5S refers to the result of clustering a cohort of subjects with multiple SCCs, which resulted in 5 subtypes and pan-SCC 6S refers to the result of clustering a cohort of subjects with multiple SCCs, which resulted in 6 subtypes).

The pan-cancer subtypes may comprise 5, 6, 7, 8, 9, 10, 11, 12, 13, 14, 15, 16, 17, 18, 19, 20, 21, 22, 23, 24, 25 or more subtypes. The pan-cancer subtypes may comprise data from a cohort of subjects with a total of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 96, 97, 98, 99, 100, or more types of cancers represented in the cohort. The cohort may comprise every known type of cancer. The cohort may comprise all of the subjects, or a subset of the subjects, from a publicly available data set, e.g., the cancer genome atlas (TCGA).

In some cases, cancer subtypes can be used to train machine learning algorithms, such that subjects that were not included in the cohort used for clustering can be identified as belonging to an identified subtype.

### Mapping cancer-specific subtypes to pan-cancer subtypes

In some cases, cancer-specific clusters can be related to pan-cancer models (e.g., a vSCC subtype can be mapped to a pan-SCC , *see* FIG. 13A). The number of cancer-specific clusters may be different from the number of pan-cancer subtypes. There may be a strong relationship between a cancer-specific cluster and a pan-cancer cluster (e.g., the profile of the cancer-specific cluster is highly similar to the pan-cancer cluster; a subject who was classified as belonging to the cancer-specific cluster is highly likely to belong to the corresponding pan-cancer cluster). In other cases, there may be a mixed relationship between a cancer-specific cluster and multiple pan-cancer clusters (e.g., a cancer-specific subtype shares similar features with 2 or more pan-cancer subtypes; a subject with the cancer-specific subtype may associate more strongly with a specific pan-cancer subtype).

In some embodiments, a subject may only be evaluated for a cancer-specific subtype. In some embodiments, a subject may only be evaluated for a pan-cancer subtype. In some embodiments, a subject may be evaluated for a cancer-specific subtype and a pan-cancer specific subtype. There are several potential benefits to determining a cancer-specific subtype and a pan-cancer subtype for a subject. A cancer-specific subtype can provide accurate predictions of who will respond to specific treatments, such as checkpoint inhibitors. Mapping the cancer-specific subtype to a pan-cancer subtype may lead to increased therapeutic options.

### Reports

In some embodiments, the trained algorithm produces a report that may be provided to a user. The report may include the predicted cancer-subtype and associated confidence or likelihood in the prediction. The report may further include a molecular profile of the sample. The report may include a detailed characterization of the cancer subtype a subject is predicted to have. This may include a list of other cancers that belong to the cancer-subtype. The detailed characterization may include a molecular profile or genetic profile the subjects in the subtype share. The detailed characterization may include shared phenotypes or other similarities among the cancers in the cancer subtype.

In some embodiments, the information provided by the trained algorithm can include matched treatment options for a subject based on which treatment options are predicted to be most effective for the subject's predicted subtype. In some embodiments, the treatment efficacy prediction is based on historical treatment response data from other patients having the same subtype. In some embodiments, the matched treatment options could include matched methods (e.g., tests, associated frequencies, etc.) to monitor the progression of the subject's cancer. In some embodiments, the matched treatment options have not been approved or indicated for the patient's cancer type (for example, without the methods disclosed herein, a clinician may not have any rationale for prescribing the treatment). In some embodiments, the matched treatment options could include drugs that are predicted to be effective in treating or preventing the subject's cancer, or drugs that are predicted to be ineffective in treating or preventing the subject's cancer.

At 106, process 100 can generate a report indicative of the predicted cancer subtype of the subject, or can otherwise display or output by the trained machine learning algorithm, model, or program.

The report may include: the molecular profile of the subject's cancer, a list of other cancer/cancer subtypes with similar molecular profiles, cancers or cancer subtypes that do not have similar molecular profiles, a list of treatments that are predicted to be effective for the subject's cancer based on the classification and/or the molecularly similar cancers, therapies that are not predicted to be effective for the subject's cancer based on the classification and/or the molecularly similar cancers, recommendations to a physician for monitoring the subject for cancer progression, e.g., guidance on whether the subject is likely to experience a progression event when treated with a particular treatment, based on the classification of the subjects cancer.

A subject that is likely to experience a progression event may warrant increased radiological assessment or increased frequency of radiological assessment. Further, a subject not likely to experience a progression event may experience immune cell infiltration into a tumor site following certain treatments, e.g., immunotherapies, that may appear to be a progression event. For the subject not likely to experience a progression event, this may be attributed to response to the therapy and not to a progression event, thereby assisting a physician in guiding the course of the subject's treatment.

### Molecular profiles

A cancer subtype may be characterized by a molecular profile (e.g., a plurality of signature genes). The signature genes can each have a corresponding score or weight. The signature genes can include at least 15,000 genes, at least 10,000 genes, at least 5000 genes, at least 4000 genes, at least 3000 genes, at least 2000 genes, at least 1500 genes, at least 1250 genes, at least 1000 genes, at least 900 genes, at least 800 genes, at least 700 genes, at least 600 genes, at least 500 genes, at least 400 genes, at least 300 genes, at least 250 genes, at least 200 genes, at least 150 genes, at least 100 genes, at least 75 genes, at least 50 genes, at least 25 genes, at least 10 genes, at least 9 genes, at least 8 genes, at least 7 genes, at least 6 genes, at least 5 genes, at least 4 genes, at least 3 genes, at least 2 genes, or at least 1 gene. The molecular profile may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, or more signature genes. Genes can be ranked based on their relative importance for a cancer subtype or their association with a cancer subtype. For instance, genes can be ranked based on the absolute value of their score; scores with a larger absolute value may be more important, relative to scores with smaller absolute values. A subtype can be characterized by the top 500 genes, 400 genes, 300 genes, 250 genes, 200 genes, 190 genes, 180 genes, 170 genes, 160 genes, 150 genes, 140 genes, 130 genes, 120 genes, 110 genes, 100 genes, 90 genes, 80 genes, 70 genes, 60 genes, 50 genes, 40 genes, 30 genes, 25 genes, 20 genes, 15 genes, 10 genes, 9 genes, 8 genes, 7 genes, 6 genes, 5 genes, 4 genes, 3 genes, 2 genes, or a top gene. The cancer may be classified based on the expression of the signature genes and/or their associated score or weight, e.g., as shown in Tables 8-13.

Tables 8-13 show the top 100 signature genes for pan-cancer subtypes 1-6, based on the absolute value of the score and ordered from highest absolute score to lowest absolute score.

A subtype may be characterized by signature genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 8, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 8. The signature genes may be selected from any of the genes listed in Table 8 in any order or combination.

A subtype may be characterized by signature genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 9, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 9. The signature genes may be selected from any of the genes listed in Table 9 in any order or combination.

A subtype may be characterized by signature genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 10, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 10. The signature genes may be selected from any of the genes listed in Table 10 in any order or combination.

A subtype may be characterized by signature genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 11, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 11. The signature genes may be selected from any of the genes listed in Table 11 in any order or combination.

A subtype may be characterized by signature genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 12, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 12. The signature genes may be selected from any of the genes listed in Table 12 in any order or combination.

A subtype may be characterized by signature genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 13, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 13. The signature genes may be selected from any of the genes listed in Table 13 in any order or combination.

A subtype may be characterized by signature genes comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 14, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 14. The signature genes may be selected from any of the genes listed in Table 14 in any order or combination.

Thus, the disclosed methods and systems may classify a cancer from a subject based, in whole or in part, on the signature genes described herein.

### EXAMPLES

### Example 1 - Pan-SCC 5S subtypes and vSCC mapping - 5 clusters

### Determining a cancer-specific profile: vSCC clustering

Vulvar squamous cell carcinoma (vSCC) is a rare cancer (FIG. 2A-2B), with approximately 6,500 new cases each year. Rare cancers generally have less research and clinical trials, and have limited treatment options. vSCC has two main causes: HPV, and chronic inflammatory processes such as lichen sclerosus (for example, mutated TP53). vSCC with different causes confer different prognoses and treatment sensitivities, but are currently treated the same clinically. Stage III and IV vSCC have poor prognosis, leading to 47% and 23% 5-year survival respectively (see FIG. 2C). To better understand vSCCs molecularly, unbiased subtyping of vSCC tumors were mapped to a large cohort of multiple SCC types.

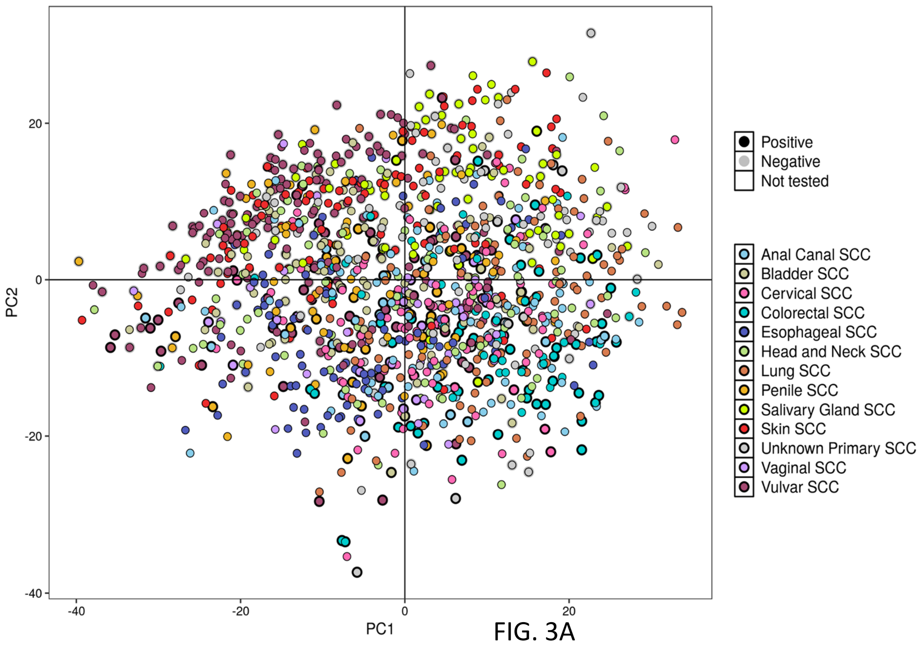

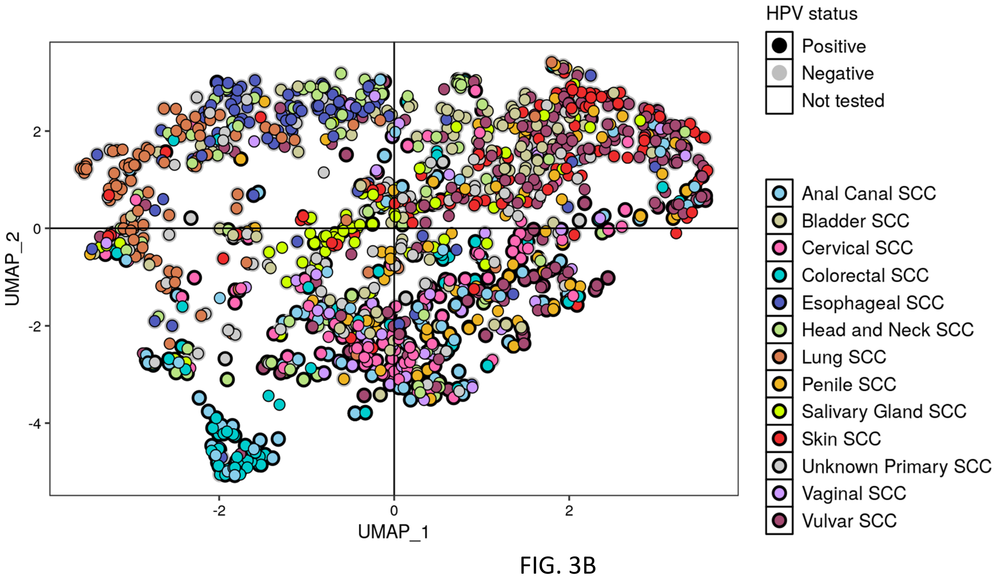

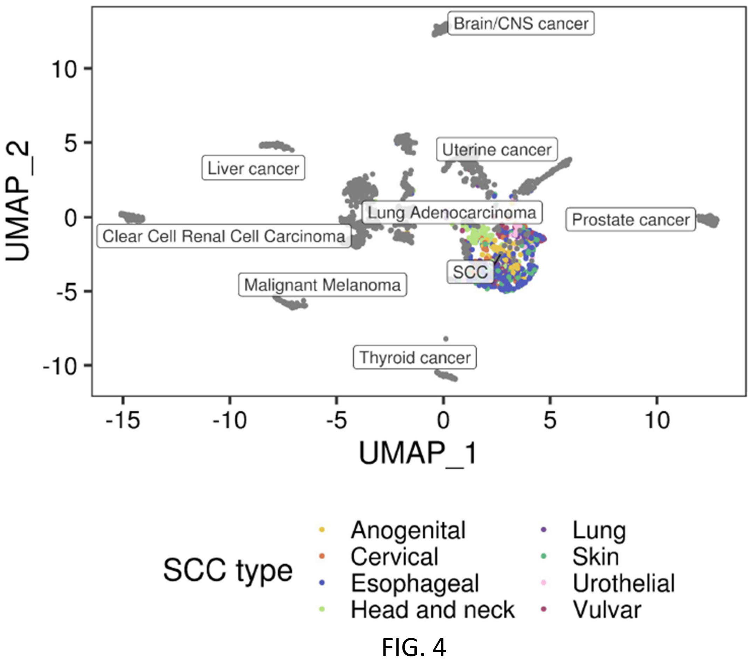

Squamous cell carcinoma (SCCs) are defined by cancer of the squamous cells, which are flat cells in the epidermis. SCCs arise from different tissue sites, including lung, head and neck, skin, cervical, urothelial, esophageal, anogenital (including anal, penile, and vulvar), and colorectal. SCCs have different etiologies, such as smoking/non-smoking, alcohol intake, HPV status, and UV exposure. SCCs have strong gene expression similarities, resulting in tissue site-independent molecular signatures. PCA was completed across SCC cancer types, and the first two principal components were plotted (FIG. 3A). The samples clustered by expression not by cancer type (e.g., tissue type). UMAP analysis was also completed across SCC cancer types, showing slightly more distinct clusters. Notably, the clusters were not always specific to one cancer type (e.g., tissue type) (FIG. 3B). FIG. 4 shows a gene expression UMAP dimension reduction of SCC and non-SCC subjects; SCC cluster together (in color), while other cancers cluster by tissue or origin (gray).

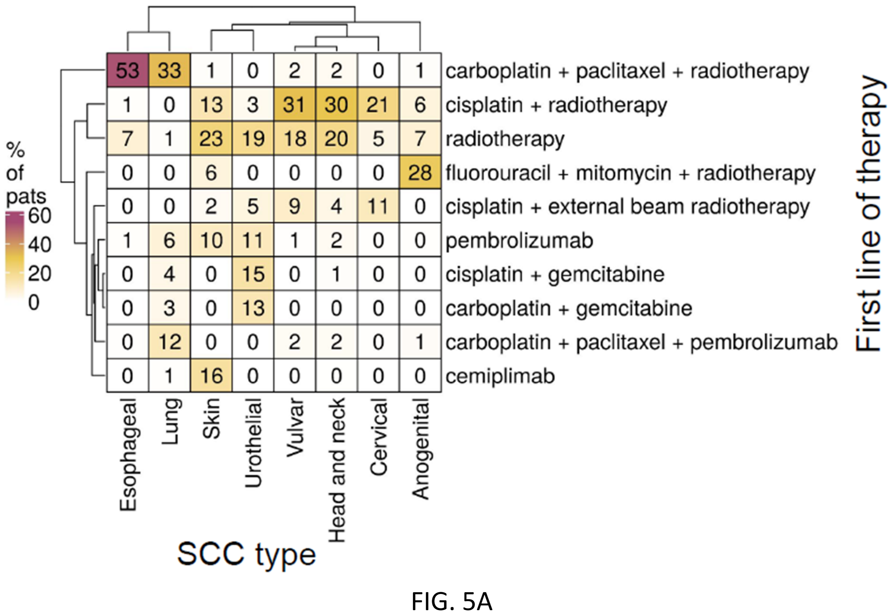

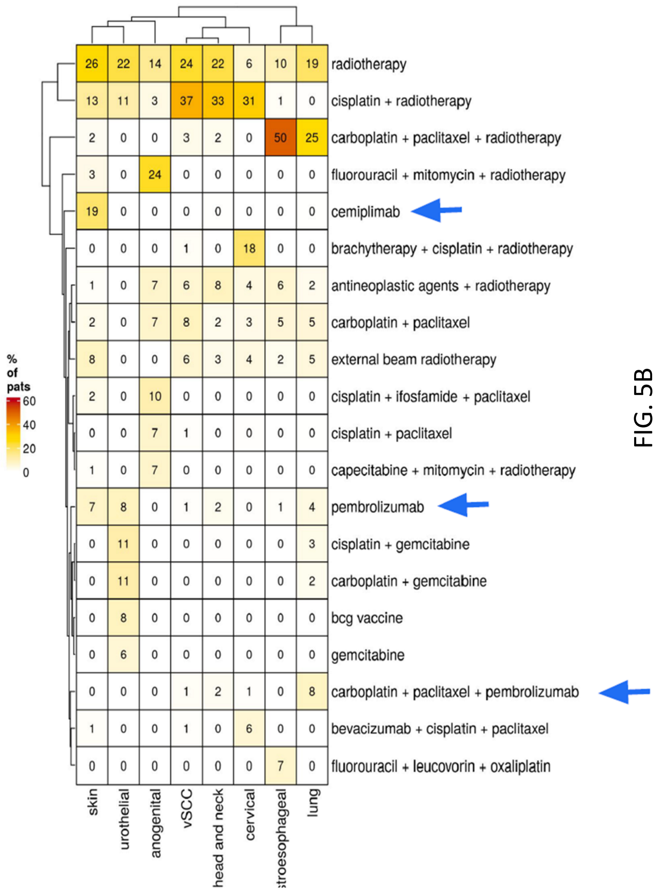

SCCs vary in frequencies. Lung, head and neck, and skin SCC are frequently occurring cancer types, while vulvar SCC is far more rare (see FIG. 2A). SCC treatments are heterogenous, with combinations of chemotherapy and immune checkpoint inhibitors acting as first-line treatment (FIGS. 5A-5B).

One aim is to leverage pan-SCC analysis to learn more about vSCC. This allows us to take advantage of the greater availability of information on common SCCs (e.g., lung SCC) to characterize rare SCCs (e.g., vSCC). This is made possible because SCCs have very similar morphologies and gene expression. By relating a rare tissue specific cancer to a pan-SCC subtype (e.g., a subtype of SCCs across tissue types with common characteristics), it is possible to determine a more comprehensive view of the rare tissue specific cancer.

A general procedure to identify tissue specific subtypes and pan-SCC subtypes can occur as follows (see FIG. 1A). 1) Cohort selection; vSCC criteria may be that a subject has vulvar cancer, the cancer has squamous cell histology, and there are no lung or liver mets for the gene expression samples. Pan-SCC criteria may be subsampled tumor types had more than 100 samples. 2) Molecular profiling: determining the molecular profile of subject in the vSCC cohort and pan-SCC cohort. 3) Identifying cohort subtypes. Subtypes can be identified via any clustering technique. 4) Mapping vSCC subtypes to pan-SCC subtypes. This mapping allows one to take advantage of the greater amount of data in the pan-SCC cohort and leverage it to learn more about vSCC subjects.

### vSCC Characterization

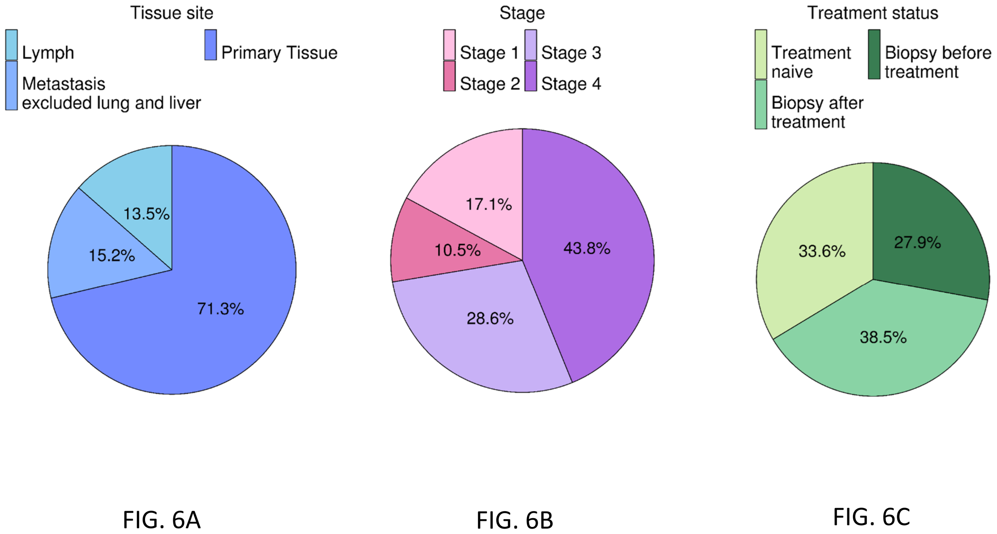

A cohort for vSCC characterization includes 215 samples sequenced with RNA-seq, 219 with tumor DNA-seq (218 from a targeted panel and 1 from whole exome), 208 with both tumor RNA and DNA-seq, and 52 cell free DNA, 40 of which had a match of cell free DNA and tumor DNA. These samples corresponded to 230 unique patients. When a patient had multiple DNA samples, we first prioritized the primary site, then higher tumor purity, and lastly an earlier sample collection date in order to select at maximum one tumor DNA and one cell free DNA sample per patient. FIG. 6A describes the tissue sites from which samples were collected. For cohort-level characterizations, the tumor DNA sample was preferred over the cell free DNA sample.

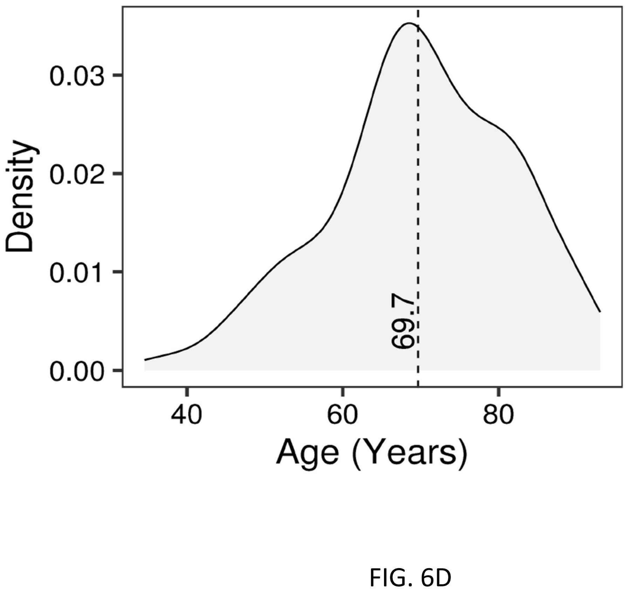

Treatment naive samples accounted for 42% (60/142) of patients with treatment data, whereas treatment exposed samples (have received at least one previous treatment) accounted for 58% of patients (FIG. 6C, Table 1). 134 patients had HPV status determined using a combination of IHC and DNA-seq (details in Methods); 80 negative and 54 positive. The mean patient age was 67; the youngest patient included in this analysis was 27 and the oldest was 89 (see FIG. 6D). The cohort was enriched for later-stage samples, with 63% of the patients with known stage data derived from stage 3 or 4 (FIG. 6B, Table 1). Additionally, 76% of samples with known grade data were Grade 1 (well differentiated) or Grade 2 (moderately differentiated).

**Table 1: Summary of subjects in vSCC cohort.**

| | Level | Overall |
|---|---|---|
| N | | 230 |
| HPV status (%) | Positive | 54 (40.3) |
| | Negative | 80 (59.7) |
| Age (mean (SD)) | | 67.03 (12.44) |
| Biopsy site (%) | primary tissue | 131 (62.4) |
| | lymph involvement | 36 (17.1) |
| | Non-primary tissue | 43 (20.5) |
| DNA Final Tumor Percentage (mean (SD)) | | 56.52 (18.25) |
| Grade (%) | Grade 1 (well differentiated) | 31 (24.2) |
| | Grade 2 (moderately differentiated) | 66 (51.6) |
| | Grade 3 (poorly differentiated) | 31 (24.2) |
| Stage (%) | Stage 1 | 27 (24.8) |
| | Stage 2 | 13 (11.9) |
| | Stage 3 | 36 (33.0) |
| | Stage 4 | 33 (30.3) |
| Treatment group (%) | Treatment Naive | 60 (42.3) |
| | Treatment Exposed | 82 (57.7) |
| Smoking status (%) | Never-smoker | 70 (52.2) |
| | Ex-smoker | 39 (29.1) |
| | Current-smoker | 25 (18.7) |

### vSCC Mutational profile

212 patients had solid tumor DNA sequencing results, with 87% of these samples corresponding to the same gene panel (Table 1). Of these 212 patients, only 5 had no genomic alterations (e.g., somatic pathogenic SNV/indels, amplifications [CN>=8], deletions [CN=0], or fusions) found (2%); 1 patient was HPV+ while the other 4 had unknown HPV status. Since HPV calls were only present in 66% of the solid tumor DNA-seq cohort, we assessed the mutations in our cohort first without considering HPV status. The most frequent mutations observed were *TP53* SNV/indels (59%), *TERT* promoter mutations (50%), *CDKN2A* SNV/indels (27%), *FAT1* SNV/indels (20%) and *PIK3CA* SNV/indels (18%) (Table 2). The most frequent copy number changes were *FGF3*/*4* amplification (12% and *10%), CCND1* amplification (10%), and *EGFR* amplification (8%). *TP53, TERT,* and *CDKN2A* mutations all significantly co-occurred (P < 1x10⁻¹¹, for all Fisher's exact test) as well as *PIK3CA* and *KMT2C* mutations (P=1.9x10⁻³), *TP53* mutations and *FGF3*/*4* amplification (P<1x10⁻³), and *TP53* and *FAT1* mutations (P=9.0x10⁻⁴). 77% (95/124) of *TP53* mutated samples also had a *TERT* promoter mutation (p=3.1x10⁻²⁰). In contrast, *TP53* mutations were significantly exclusive with *KMT2D* (P=3.0x10⁻⁴) and *ZNF750* (P=7.3x10⁻³) mutations.

**Table 2: Top gene prevalence in vSCC cohort**

| Gene | Prevalence |
|---|---|
| TP53 SNV/indel | 58.50% |
| TERT promoter | 49.50% |
| CDKN2A SNV/indel | 26.90% |
| FAT1 SNV/indel | 19.80% |
| PIK3CA SNV/indel | 17.50% |
| FGF3 Amplification | 12.30% |
| KMT2D SNV/indel | 11.30% |
| CCND1 Amplification | 10.40% |
| FGF4 Amplification | 10.40% |
| EGFR Amplification | 8% |
| NOTCH1 SNV/indel | 8% |
| ZNF750 SNV/indel | 8% |
| CASP8 SNV/indel | 7.50% |
| UGT1A1 Deletion | 7.50% |
| SEC61G Amplification | 7.10% |
| MYL1 Deletion | 6.60% |
| CYP1B1 Deletion | 6.10% |
| ERBB4 Deletion | 5.70% |
| KMT2C SNV/indel | 5.70% |
| NTRK3 Deletion | 5.70% |
| HRAS SNV/indel | 5.20% |
| CDKN2B Deletion | 4.70% |
| PTEN SNV/indel | 4.70% |
| AJUBA SNV/indel | 4.20% |
| CDKN2A Deletion | 4.20% |
| EP300 SNV/indel | 4.20% |
| FBXW7 SNV/indel | 4.20% |
| ARID2 SNV/indel | 3.80% |
| GRM3 Deletion | 3.80% |
| MAPK1 SNV/indel | 3.80% |

*TP53* mutation and HPV presence were mutually exclusive (p=6.7x10⁻²¹, Fisher's exact test)Table 3). 49/53 HPV positive samples were *TP53* WT and 72/80 samples were HPV negative and *TP53* mutated. We assessed the mutations in the 8 samples that were *TP53* negative and HPV negative; three had *TERT* promoter mutations. *TP53, TERT, CDKN2A, FAT1* mutations and *FGF3* amplification were all mutually exclusive with HPV presence whereas *KMT2C*/*D* and *ZNF750* mutations were enriched in HPV positive samples (P<0.05, all tests) (Table 3), consistent with previous characterization of vSCC cohorts. Due to the significant overlap between the *TP53* mutant/HPV- and *TP53* WT/HPV+ cohorts, similar enriched mutations were observed when splitting the cohort by *TP53* mutant status (Table 4). *TP53* mutations were previously found to be associated with HPV- vSCC as well as HPV- Oral Cavity Squamous Cell Carcinoma. Moreover, TERT promoter mutations have also previously been linked to HPV- vSCC and penile carcinoma, consistent with our results.

**Table 3: hpv_significant_mutations**

| gene | HPV+/WT | HPV+/mutant | HPV-/WT | HPV-/mutant | p_value |
|---|---|---|---|---|---|
| TP53 SNV/indel | 36.80% | 3% | 6% | 54.10% | 6.73E-21 |
| TERT promoter | 36.80% | 3% | 15% | 45.10% | 3.64E-14 |
| TERT c.-124C>T | 38.30% | 1.50% | 30.80% | 29.30% | 1.13E-06 |
| CDKN2A SNV/indel | 39.10% | 0.80% | 36.10% | 24.10% | 7.86E-07 |
| ZNF750 SNV/indel | 30.80% | 9% | 59.40% | 0.80% | 0.000317 |
| KMT2D SNV/indel | 30.10% | 9.80% | 58.60% | 1.50% | 0.00053 |
| FAT1 SNV/indel | 37.60% | 2.30% | 40.60% | 19.50% | 0.000811 |
| FGF3 Amplification | 39.10% | 0.80% | 48.90% | 11.30% | 0.007492 |
| KMT2C SNV/indel | 34.60% | 5.30% | 59.40% | 0.80% | 0.017203 |

**Table 4: tp53_significant_mutations**

| gene | TP53 WT/WT | TP53 WT/mutant | TP53 mutant/WT | TP53 mutant/mutant | p_value |
|---|---|---|---|---|---|
| TERT promoter | 36.80% | 4.70% | 13.70% | 44.80% | 1.80E-20 |
| TERT c.-124C>T | 38.20% | 3.30% | 29.20% | 29.20% | 4.46E-09 |
| TERT c.-146C>T | 40.60% | 0.90% | 45.30% | 13.20% | 0.001499 |
| CDKN2A SNV/indel | 40.60% | 0.90% | 32.50% | 25.90% | 1.89E-12 |
| CDKN2A p.R80* | 41.50% | 0% | 50.50% | 8% | 0.009753 |
| KMT2D SNV/indel | 32.10% | 9.40% | 56.60% | 1.90% | 7.52E-05 |
| FAT1 SNV/indel | 38.70% | 2.80% | 41.50% | 17% | 0.000236 |
| FGF3 Amplification | 40.60% | 0.90% | 47.20% | 11.30% | 0.000425 |
| ZNF750 SNV/indel | 34.90% | 6.60% | 57.10% | 1.40% | 0.00189 |
| PIK3CA SNV/indel | 31.10% | 10.40% | 51.40% | 7.10% | 0.039091 |

Tumor Mutation Burden (TMB) was low in the vSCC cohort, with a median of 3.1 and a maximum of 35.0; for reference, melanoma had a median TMB of 6.50 and NSCLC had a median TMB of 5.71. Only 9 out of 212 (4%) samples were TMB High, defined as >=10, and only 2 samples were MSI-High, consistent with previous characterizations of vSCC cohorts. Of the 170 samples scored for PD-L1 IHC 22c3 TPS, 65% (111/170) were PD-L1 Positive when using a >=1 TPS cutoff and 14% (24/170) of those samples were High Positive (>=50 TPS). When using PD-L1 IHC 22c3 CPS as a metric, 45% (64/141) were PD-L1 Positive (>=10 CPS). Discrepant proportions of PD-L1 positive vSCC tumor cells have previously been described, possibly due to differing antibodies and cutoffs utilized. Nonetheless, there was a high correlation observed between the PD-L1 TPS and CPS IHC categories (p=3.17x10⁻⁹).

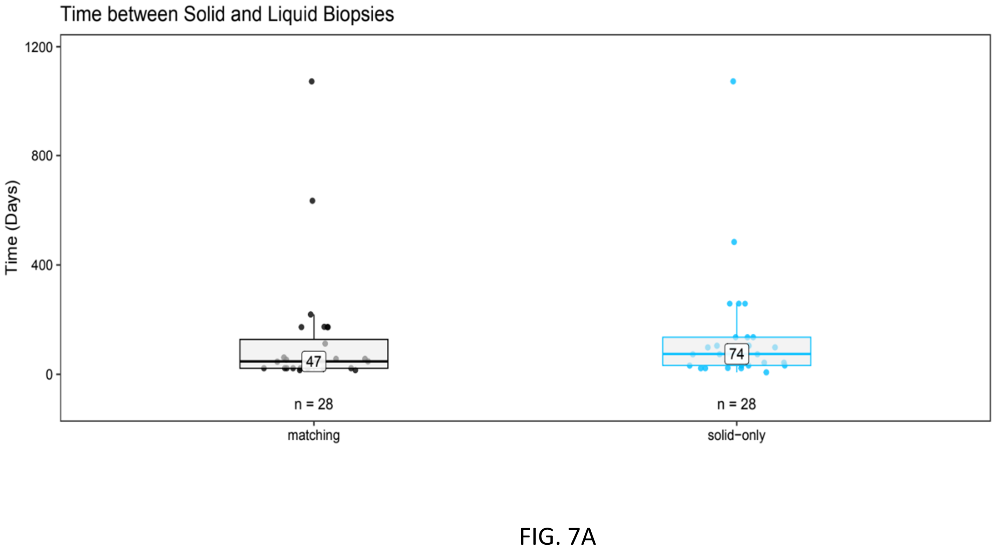

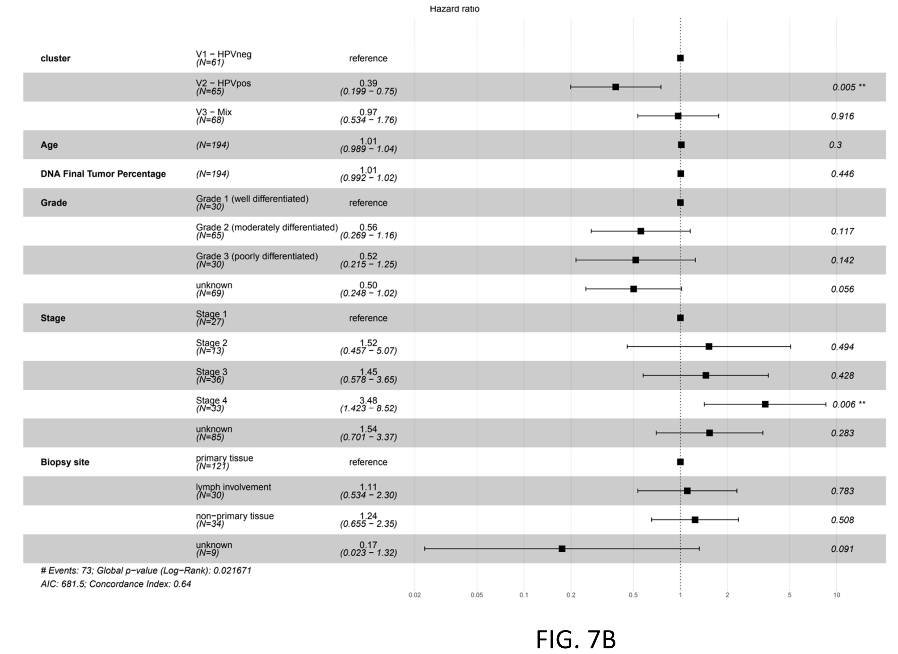

49 patients had cell-free DNA data, while 40 patients had matched tumor DNA (solid biopsy) and cell-free DNA (liquid biopsy) samples, with a median time of 74.5 days between solid and liquid biopsies, a minimum of 7 days, and a maximum of 1,092 days. Out of these 40 patients, 20 patients showed at least one somatic pathogenic genomic alteration that was present in both the solid and liquid biopsies. For the mutations with the highest prevalence in this cohort of patients (*TP53, PIK3CA, TERT, and CDKN2A*)*,* we examined the prevalence of patients with a mutation found in the solid tumor, then identified in the cell free DNA assay. 47.6% (10/21) of patients had matching *TP53* mutations, 47.1% (8/17) patients had matching *TERT* mutations, 62.5% (5/8) patients had matching *PIK3CA* mutations, and 50% (5/10) patients had matching *CDKN2A* mutations; the timing between the solid and liquid biopsies may contribute to matching results (FIG. 7A). These results are relatively consistent with previous results analyzing the sensitivity and specificity of matched samples from these solid and liquid biopsy methods in NSCLC, CRC, and Breast Cancer, which found an overall sensitivity of liquid relative to solid of 68.18% for SNVs and INDELs, and 57.89% for CNVs. FIG. 7B shows a table summarizing the hazard ratio of vSCC subjects stratified by different characteristics.

### vSCC Unsupervised gene expression subtyping

Consensus clustering (CC) algorithms compute probabilities of cluster assignment and produce robust and reproducible clusters. The rapid increase in dataset sizes from bulk RNA-seq and single cell has made CC algorithms computationally prohibitive. As a result, we developed FastPG-CC, an ultra-fast CC tool for highly scalable clustering for high-dimensional and large sample datasets.

Using FastPG-CC, we identified three vulvar cancer clusters; an HPV- cluster (**V1** - **HPVneg,** 91% HPV- and 91% TP53 mutated), HPV+ cluster (V2 - **HPVpos,** 78% HPV+ and 82% TP53 WT), and a cluster which contained both HPV+ and HPV- samples **(V3** - **Mix,** 24% HPV+ and 33% TP53 WT). Interestingly, the Mix cluster accounted for the first largest source of variation in the data determined using Principal Component Analysis, whereas HPV status accounted for the second largest source of variation (FIG. 8A). To assess the biological differences between subtypes, we assessed the gene expression-based pathway analysis and the cell deconvolution method, *xCell* (Methods).

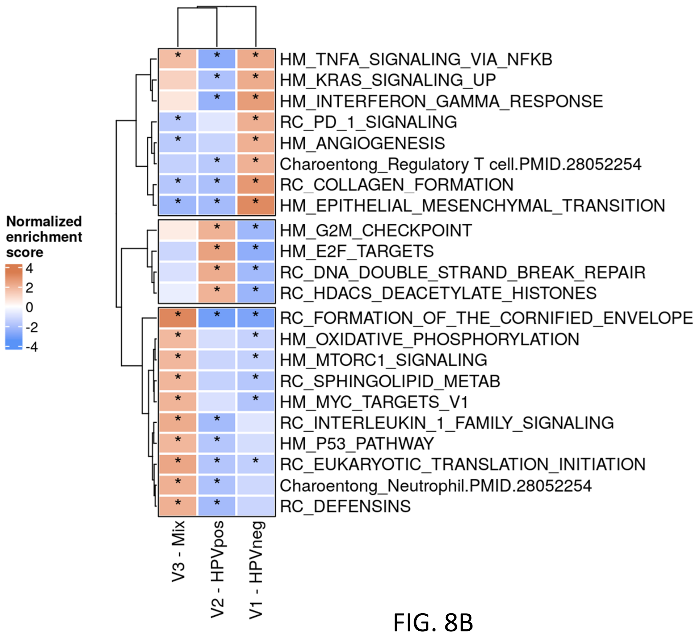

**V1** - **HPVneg** was enriched in pathways associated with epithelial-mesenchymal transition (EMT, Q = 3x10⁻⁴⁶), KRAS signaling (Q = 2x10⁻¹¹), inflammatory response (Q = 4x10⁻¹⁶), and a large tumor microenvironment fraction indicative of a immunosuppressive environment, with strong enrichment for cancer associated fibroblasts (Q = 1x10⁻⁶), and T-regulatory cells (Q = 3x10⁻⁴) (FIG. 8B). **V2 - HPVpos** was enriched in tumor proliferation and cell cycle related pathways: E2F targets (Q = 2x10⁻¹⁷) and G2M checkpoint (Q = 4x10⁻⁸). **V3 - Mix** was enriched in RNA, protein, and fatty acid metabolism (Q = 1x10⁻⁸, 5x10⁻⁷, 1x10⁻³ respectively), and neutrophil expression (Q = 7x10⁻⁶).

### vSCC Clinical parameter comparison by cluster

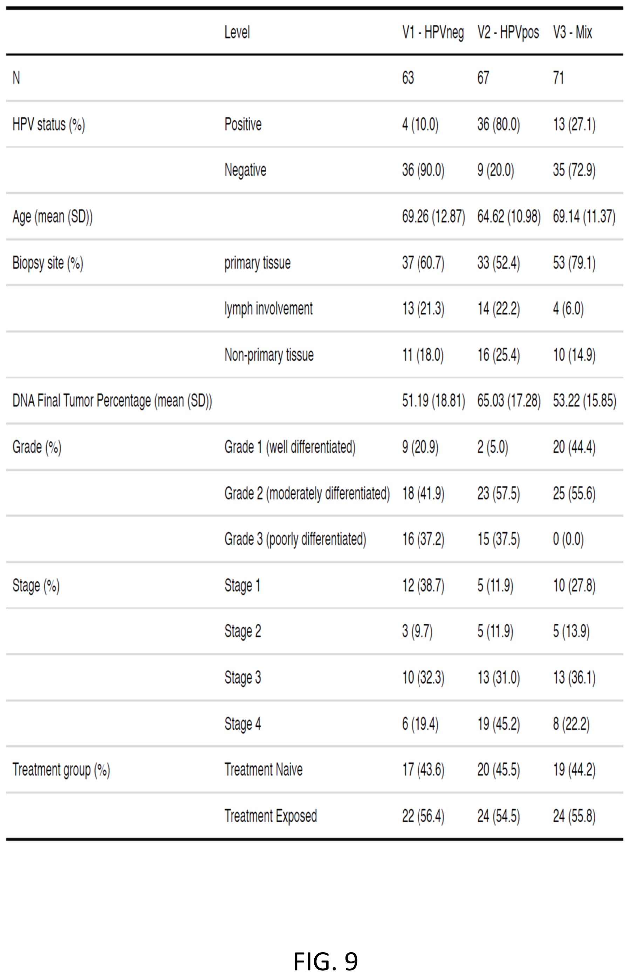

**V1** - **HPVneg** and **V3 - Mix** had lower bioinformatics-derived tumor purity (median of 51% and 53% respectively) which was significantly lower compared to **V2 - HPVpos** (Wilcox test, P = 2.6x10⁻⁵ and P = 8.3x10⁻⁵ respectively, (FIG. 9),which may account for the increased evidence of tumor microenvironment infiltration observed in gene expression for V1 and V3 compared to V2. **V3 - Mix** had the highest proportion of moderate and well-differentiated samples with no poorly differentiated samples annotated, whereas 37.2% and 37.5% of the samples were poorly differentiated in **V1** - **HPVneg** and **V2 - HPVpos,** respectively (P=2.0x10⁻⁶ V1 vs. V3, P=3.7x10⁻⁷ V2 vs. V3). The Mix-V3 cluster also had the higher proportion of primary tumor derived samples (P=3.2x10⁻³ compared to V1 and P=0.014 compared to V2, Chi-Squared test).

All three clusters displayed similar proportions of treatment naive and treatment exposed samples (all clusters between 43.6% and 45.5% treatment naive) . Interestingly, **V2** - **HPVpos** had the lowest pathology-derived differentiation scores and the cluster with the highest stage 3 and 4 proportions. HPV positive vSCCs have previously been reported to have better outcomes and the lower differentiation and higher proportion of late stage samples may be due to the sampling bias inherent in the clinically-derived sequencing dataset.

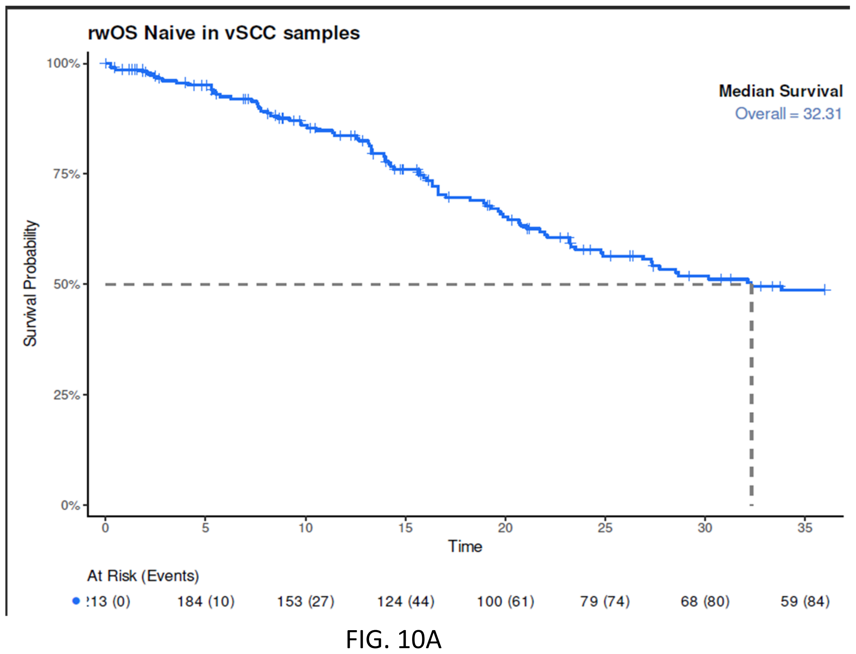

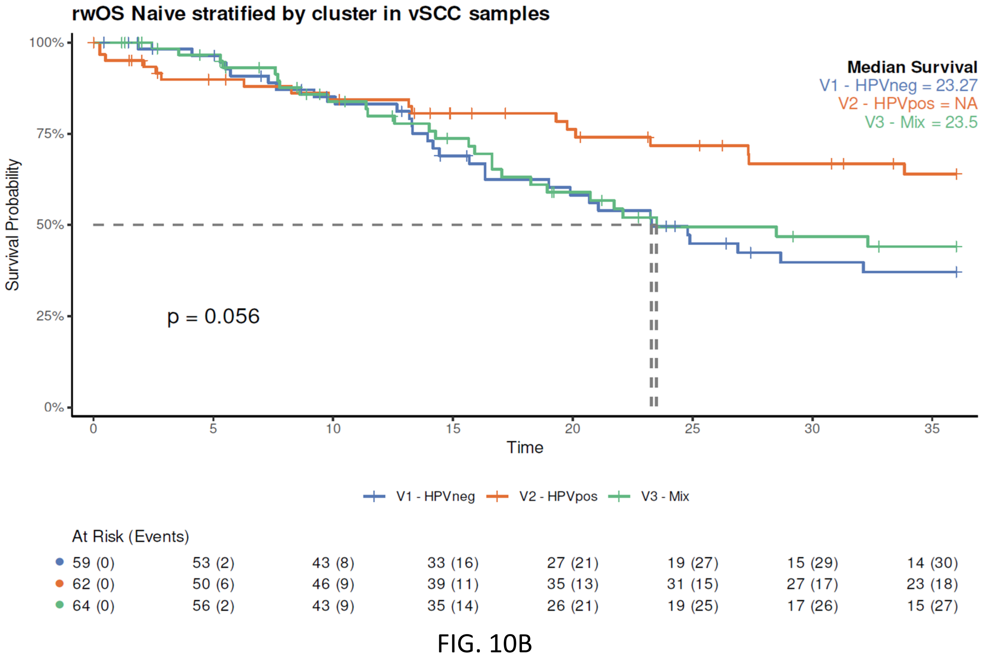

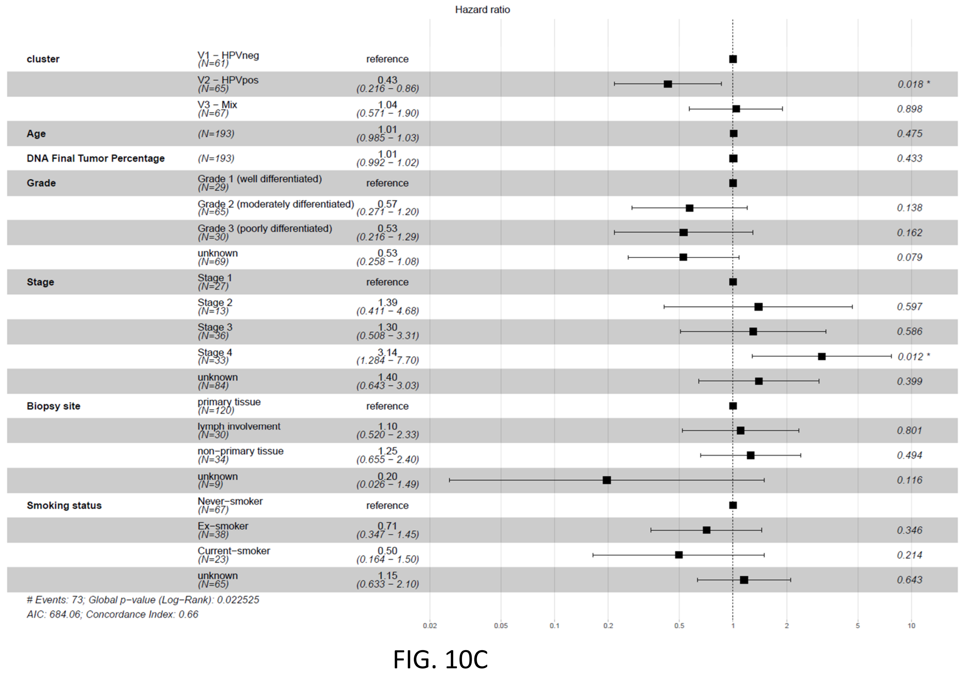

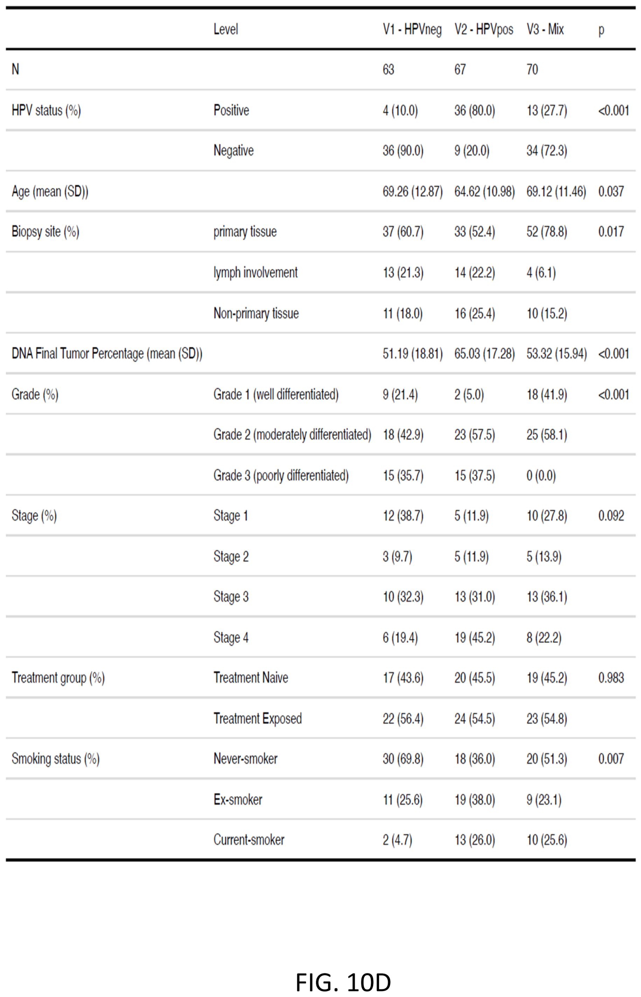

rwOS (real-world overall survival) was assessed for 229 patients with outcomes data (FIG. 10D, FIG. 10C). The vSCC cohort showed a median overall survival of 32.3 months (FIG. 10A). When stratifying by cluster assignment, V2 - HPVpos displayed an undefined median overall survival, with greater than 50% survival probability at 36 months, while V1 - HPVneg showed a median OS of 23.27 months and V3 - Mix showed a median overall survival of 23.5 months (P=0.06, univariate model) (FIG. 10B). We fit a multivariate CoxPH model with cluster, age, tumor purity, grade, stage, and biopsy site as covariates, and found a significant difference in rwOS between V2 and V1 (P=0.005) (FIG. 10C).

While fusions were assessed, they were only present in 12 patients (2 from cluster V1 and 10 from cluster V2) and no fusions were found among the most prevalent mutations observed. 3/12 (25%) of these fusions were *FGFR3-TACC3* fusions; all of these fusions were found in cluster V2. 3p loss was positively associated with TP53 mutations (p=0.031), while 3q gain was negatively associated with TP53 (p=2.7x10⁻³), TERT promoter (p=6.5x10⁻⁶), and CDKN2A mutations (p=0.015). Similarly, 11q loss was negatively associated with TP53 (p=0.046) and TERT promoter mutations (p=1.9x10⁻⁴), but positively associated with PIK3CA (p=0.024), KMT2D (p=3.1x10⁻³), and ZFN750 mutations (p=5.3x10⁻³).

### Alterations by vSCC subtype

On the whole, enriched and depleted mutations in V1 and V2 demonstrated significant overlap and similar changes in proportion when compared to the HPV positive and negative enriched mutations; in contrast, there were no significant mutations in V3, potentially due to the mixed HPV status of this cluster. Of the HPV-associated mutations, *FGF4* amplification was the only mutation not significant in either V1 or V2 (Table 5, Table 6). In addition to the HPV-associated mutations, *CYP1B1* deletions were significantly enriched in V1 (P=0.019), while *PIK3CA* SNV/indels and *KMT2C* SNV/indels were significantly enriched in V2 (P=0.00022 and P=0.014, respectively) (Table 7).

**Table 5: vSCC subtype 1 significant mutations**

| gene | Cluster 1/WT | Cluster 1/mutant | Not Cluster 1/WT | Not Cluster 1/mutant | p_value |
|---|---|---|---|---|---|
| TP53 SNV/indel | 3% | 28.40% | 38.80% | 29.90% | 7.86E-10 |
| TERT promoter | 7% | 24.40% | 43.80% | 24.90% | 5.64E-07 |
| TERT c.-124C>T | 14.90% | 16.40% | 51.70% | 16.90% | 0.042833 |
| CDKN2A SNV/indel | 15.90% | 15.40% | 56.70% | 11.90% | 4.22E-05 |
| CYP1B1 Deletion | 27.40% | 4% | 67.70% | 1% | 0.005287 |
| KMT2D SNV/indel | 30.80% | 0.50% | 58.20% | 10.40% | 0.008123 |
| PIK3CA SNV/indel | 28.90% | 2.50% | 53.20% | 15.40% | 0.037174 |
| KMT2C SNV/indel | 31.30% | 0% | 63.20% | 5.50% | 0.041372 |
| FGF3 Amplification | 24.90% | 6.50% | 62.70% | 6% | 0.0475 |

**Table 6: vSCC subtype 2 significant mutations**

| gene | Cluster 2/WT | Cluster 2/mutant | Not Cluster 2/WT | Not Cluster 2/mutant | p_value |
|---|---|---|---|---|---|
| TERT promoter | 30.30% | 3% | 20.40% | 46.30% | 1.66E-15 |
| TERT c.-124C>T | 30.30% | 3% | 36.30% | 30.30% | 1.87E-05 |
| TERT c.-146C>T | 33.30% | 0% | 53.70% | 12.90% | 0.002496 |
| TP53 SNV/indel | 27.40% | 6% | 14.40% | 52.20% | 3.67E-15 |
| CDKN2A SNV/indel | 33.30% | 0% | 39.30% | 27.40% | 3.90E-11 |
| PIK3CA SNV/indel | 21.40% | 11.90% | 60.70% | 6% | 5.37E-05 |
| KMT2D SNV/indel | 24.90% | 8.50% | 64.20% | 2.50% | 6.34E-05 |
| ZNF750 SNV/indel | 26.90% | 6.50% | 65.20% | 1.50% | 0.000312 |
| FAT1 SNV/indel | 31.80% | 1.50% | 48.80% | 17.90% | 0.000473 |
| KMT2C SNV/indel | 28.90% | 4.50% | 65.70% | 1% | 0.003369 |
| FGF3 Amplification | 32.30% | 1% | 55.20% | 11.40% | 0.008359 |

**Table 7: HPV significant mutations**

| gene | HPV+/WT | HPV+/mutant | HPV-/WT | HPV-/mutant | p_value |
|---|---|---|---|---|---|
| TP53 SNV/indel | 36.80% | 3% | 6% | 54.10% | 6.73E-21 |
| TERT promoter | 36.80% | 3% | 15% | 45.10% | 3.64E-14 |
| TERT c.-124C>T | 38.30% | 1.50% | 30.80% | 29.30% | 1.13E-06 |
| CDKN2A SNV/indel | 39.10% | 0.80% | 36.10% | 24.10% | 7.86E-07 |
| ZNF750 SNV/indel | 30.80% | 9% | 59.40% | 0.80% | 0.000317 |
| KMT2D SNV/indel | 30.10% | 9.80% | 58.60% | 1.50% | 0.00053 |
| FAT1 SNV/indel | 37.60% | 2.30% | 40.60% | 19.50% | 0.000811 |
| FGF3 Amplification | 39.10% | 0.80% | 48.90% | 11.30% | 0.007492 |
| KMT2C SNV/indel | 34.60% | 5.30% | 59.40% | 0.80% | 0.017203 |

In various embodiments, features that define subgroups can include molecular data in addition to or instead of transcriptomic data. Molecular data can include genomic, epigenomic, proteomic, peptidomic, and/or metabolomic data.

### Pan-SCC cancer type comparison

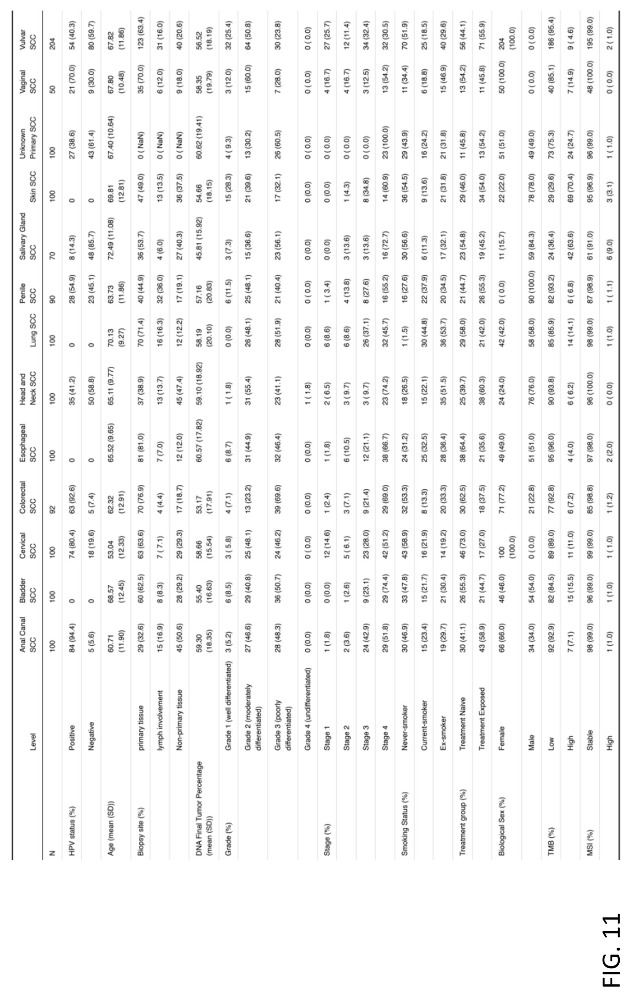

In order to understand the molecular profiling of vSCCs and to see if vSCCs have similarity to other SCCs for potential additional treatment options, we aggregated a subset of all the SCC samples in the Tempus RW database; a pan-SCC cohort which is composed of 13 different SCC cancer types (FIG. 11A) including: anal canal, bladder, cervical, colorectal, esophageal, head and neck, lung, salivary gland, penile, unknown primary, skin, vaginal, and vulvar SCC. We excluded SCC types with <50 samples with both DNA and RNA sequencing, and for cancer types with >100 samples in the proprietary database, we prioritized tumor purity, primary tumor biopsy site, and assay. With the SCC cohorts with >50 and <100 samples with both DNA and RNA sequencing, we included all samples. We deduplicated the data so there was at maximum 1 sample per patient, using tumor purity, primary tumor biopsy site, and assay for deduplication

### Pan-SCC cancer type clinical characteristics

There were 1,306 samples sequenced from 13 different SCC cancer types (FIG. 11). We assigned HPV status for those cancer types known to be HPV-associated: Anal Canal SCC, Cervical SCC, Colorectal SCC, Head and Neck SCC, Penile SCC, Salivary Gland SCC, Unknown Primary SCC, Vaginal SCC, and Vulvar SCC (FIG. 11A). Anal Canal SCC, Cervical SCC, and Colorectal SCC were identified to have the highest proportions of HPV positive samples (94%, 80%, and 93%, respectively), while Head and Neck SCC, Salivary Gland SCC, Vulvar SCC, and Unknown Primary SCC display higher proportions of HPV negative samples (59%, 86%, 60%, and 61% respectively). Treatment naive samples comprised 40% to 73% of samples within each indication. The median patient age was between 53 and 73 years for all indications, with the lowest median in Cervical SCC and the highest median in Salivary Gland SCC (FIG. 11A).

Similar to the vSCC cohort, the pan-SCC cohort was also enriched for later stage samples, with 83% (498/603) of the patients with known stage data derived from stage 3 or 4 (FIG. 11). The majority of samples were assigned Grade 2 or Grade 3, with an enrichment in higher grade samples observed in Colorectal SCC (70% Grade 3) (FIG. 11). The majority of samples with known biopsy site data (59%, 691/1164) were biopsied from primary tissue (FIG. 11). While the majority of indications displayed less than 25% High TMB samples, the majority of Skin SCC and Salivary Gland SCC samples had high TMB (70% and 64%, respectively) (FIG. 11). 98% (1251/1271) of samples with known MSI data were stable (FIG. 11). The lowest proportion of never-smoker patients was observed in Lung SCC (1.5%, 1/67), while the highest proportions of never-smoker patients were observed in Cervical SCC (58.9%, 43/73) and Salivary Gland SCC (56.6%, 30/53) (FIG. 11).

### Pan-SCC cancer type alteration comparison

FIG. 12A shows the mutational patterns stratified by SCC type. The left column represents the proportion of mutation. Since HPV status had a strong association with alterations, for the cancer types which are known to be HPV driven, we split them into HPV- and HPV+ and only included cohorts with >20 samples (vulvar, cervical, head and neck, colorectal, anal cancel, and unknown primary SCC, and penile), we split those cancer types by HPV status. Across all cancer types, there were four main mutational patterns (FIG. 12B Supplemental table): (1) HPV+ group, (2) mostly TP53 mutated and TERT WT, (3) bladder SCC, and 4 mostly TP53 AND TERT mutated.

The **(1) HPV+ group** is composed HPV+ cancers. This group tended to have a higher frequency of PIK3CA (26-52%) and KMT2D (14-32%) and almost no TP53 mutations, which unknown primary SCC with the highest frequency (0-36%). CDKN2A alterations, FGF3/4 amplifications, and CCND1 amplifications, and NFE2L2 SNPs had very low frequencies. FIG. 12C shows UMAP analysis stratified by HPV status, colored by SCC type.

The **(2) mostly TP53 mutated and TERT WT group** contained esophageal, lung, head and neck HPV-, and unknown primary SCC HPV-. This group had a very high frequency of TP53 mutations, ranging from 72% to 94%. TERT mutations had <1% frequency in lung and esophageal SCC and were more frequent in H&N and unknown primary (33 and 43% respectively) H&N and esophageal had higher frequencies of copy number changes with CDKN2A deep deletion occurring in 33% and 40% respectively, and FGF3 amplification occurring in 29% and 34% respectively.

The **(4) mostly TPS3/TERT** mutated group was composed of salivary gland, skin, bladder, penile, and vulvar SCC. This group had a high frequency of TP53 mutations (ranging from 56%- 96%) and a high frequency of TERT promoter mutations (50-77%). This group also had a higher frequency of CDKN2A, FAT1, and NOTCH1 alterations. Vulvar SCC HPV- had the most frequent TERT promoter mutations, less NOTCH1, and higher FGF3/4 and CCND1 amplifications compared to the other cancer types in this group.

Lastly, **bladder SCC** had a unique mutational profile, with some features from each of the other three classes; a higher frequency of PIK3CA and KMT2D mutants similarly to the HPV+ samples (41 and 29% respectively), but also had higher frequencies of TP53 and TERT promoter mutations (66% and 62% respectively). Bladder SCC had the highest frequency of MTAP deep deletions (32%).

The other alteration type to consider was fusions. The most recurring fusions was FGFR3-TACC3.

Copy number alterations description. 3q and 3p are the most characteristic CN changes for SCC. In this dataset, we find those altered as well, but independent of each other (TEST). There were significantly more 3q gains in HPV+ compared to HPV-, with Lung SCC having the highest 3q gains and skin SCC the least (FIG. 12A. Interestingly, 3p CN was not associated with HPV (P-value). Vulvar SCC had the highest 3p loss (FIG. 12A), followed by esophageal. Lastly, we assessed the 11q.13.3 gain which was also characteristic of SCCs. This cytoband contains FGF3, FGF4, and CCND1, which were amplified in many SCCs (FIG. 12A). Esophageal had the highest proportion of gains followed by HNSC and vulvar SCC. 11q 13.3 was strongly associated with HPV status (P-value, ), enriched in HPV negative samples.

### Classifying Pan-SCC cancer type relationship based on gene expression and pan-SCC cohort relatedness

PCA was computed across SCC cancer types, and the first two principle components were plotted. (FIG. 3A). The different cancer types largely overlapped. Next, the UMAP scores were calculated across cancer type (FIG. 3B). This showed somewhat more clear differentiation across SCC cancer types. For instance, colorectal SCC was largely clustered together, slightly separated from other cancer types. Next, subjects were divided based on HPV status, and UMAP analysis was completed on the HPV negative and HPV positive subtypes (FIG. 12C).

We calculated three different metrics using gene expression to better understand the relationships between and across SCC cancer types. (1) Computed the centroids within each cancer type and compared the Euclidean distance of all the cancer type's centroids. This metric allows an understanding of how similar an approximate middle of each of the cancer types are compared to each other. (2) Computed the sum of squares within cancer types, which measures how similar each of the samples within a group are to each other. The larger the sum of squares, the more spread there is within a cancer type. (3) Lastly, we calculated the Silhouette widths. Silhouette width calculates the cluster cohesion vs. the cluster separation. Higher the value, more strongly the sample belongs to the assigned cancer type, while the negative values represent samples which map closer to another tissue (FIG. 12D). This measurement enables us to determine if a sample belongs most strongly to its assigned cancer type or has a closer distance to another cancer type.

The Silhouette measurement outputs the next nearest cancer type assignment. For example, the samples with the most negative head and neck SCC Silhouette widths group more similarly to vulvar SCC (FIG. 12D).

As expected, unknown primary SCC had the largest within-cancer type sum of squares and the lowest silhouette width, meaning this cancer type assignment had the most differences amongst samples within a cancer type (FIG. 12E), followed by bladder, head and neck, penile and skin. The cancer types with the lowest sum of square values and highest silhouette widths were salivary gland, cervical, colorectal, vulvar and esophageal (FIG. 12E, FIG. 12H, FIG. 12I). The SCC types with the worst cluster cohesion were, as expected, SCC of unknown primary, with all samples having a negative Silhouette width, followed by head and neck, bladder, vaginal, and penile.

From the pan-SCC cohort, the cancer types that had the smallest centroid distance compared to vulvar SCC were penile SCC (distance = 33.8), skin SCC (distance = 38.6), bladder SCC (distance = 39.2), and head and neck (distance = 39.4). The cancer types that overall had the closest centroids by Euclidean distance were vaginal and cervical cancers (distance = 25.9), followed by anal canal and cervical (distance = 30.1) and anal canal and colorectal (distance = 30.3); all of these cancer types are mostly HPV+. Interestingly, despite both colorectal and cervical being close to anal canal, they are more distant from each other (distance = 41) (FIG. 12F). Lung SCC and salivary gland SCC were the most distant from the other SCCs, with mean distances of 59 and 60 respectively, while the next largest mean was vaginal SCC with a mean distance of 45.2 (FIG. 12F). FIG. 12G shows a schematic representation of SCC cancer type.

### pan-SCC subtypes and vSCC mapping: pan-SCC 5S (five subtypes/clusters)

In a particular example, clustering of the pan-SCC cohort led to five subtypes with robust group membership (referred to as pan-SCC 5S). Lung, cervical, anogenital, and esophageal SCCs had strong gene expression-based tissue type cohesion, meaning samples within cancer types were more similar to each other than to other SCC types. Head and neck, skin, and especially vulvar SCCs were heterogeneous; samples within these tumor types were more similar to SCC types. Silhouette width (SW) plots display the cluster cohesion vs. the cluster separation. Higher values represent samples that belong more strongly to the assigned cluster, while the negative values represent sample which map closer to another tissue. FIG. 13A shows the Silhouette widths by SCC tissue type, while FIG. 13B shows the SW by pan-SCC 5S clusters. FIG. 13C shows a barplot representing the proportion of each cancer type belonging to each of the pan-SCC 5S subtypes. FIG. 13D shows tumor origin analysis stratified by pan-SCC 5S clusters.

The pan-SCC 5S subtypes (SCC1, SCC2, SCC3, SCC4, SCC5, SCC6) can be generally characterized as follows. SCC1 is dominated by esophageal SCC. SCC2 is characterized by EMT and suppressive immune microenvironment and includes primarily vSCC, skin, and head and neck cancers. SCC3 is characterized by HPV positivity (92%). SCC4 is dominated by lung SCC. SCC5 includes skin and vSCC characterized by metabolic and neutrophil related genes.

The three vSCC subtypes (V1, V2, V3) mapped strongly with pan-SCC 5S subtypes (FIG. 13E). V1 is largely HPV negative mapped strongly to SCC2. V2 is largely HPV positive, and mapped strongly to SCC3. V3 is a mix of HPV positive and negative, and mapped strongly to SCC5. FIG. 13F shows the specificity and sensitivity of each pan-SCC 5S subtype. FIG. 13G shows the number of samples in each pan-SCC 5S subtype.

vSCCs were almost indistinguishable from skin SCC by gene expression. pan-SCC 5S subtypes 2, 3, and 5 were analyzed to investigate gene expression analyses of vSCC within these subtypes (in which vSCCs were mostly found) (see FIGS. 14A-14C). Within pan-SCC 5S subtype 2, vSCC only had 3 differentially expressed genes (DEGs). Within pan-SCC 5S subtype 5, vSCC vs. skin only had 11 DEGs.

SCC membership is associated with overall survival. SCC2 assignment and SCC2 probability were both associated with worse overall survival (OS). Using multinomial ElasticNet, SCC subtypes were applied to a larger SCC cohort from the Tempus clinic-genomic database (n=2,763) for outcomes analyses. Patients with samples in pan-SCC 5S subtype 2 had consistently worse outcomes compared to patients with samples from pan-SCC 5S subtype 5 in cancer types with >50 samples in each: vSCC (p=0.06, HR = 1.7, Cox PH), head and neck (p = 0.01, HR = 1.5), skin squamous (p = 0.05, HR = 1.9). FIGS. 15A-15C show Kaplan-Meier plots displaying the overall survival probability for the cancer types with >50 samples in pan-SCC 5S subtype 2 and 5. FIG. 15D shows that SCC probability was associated with OS after accounting for clinical covariates. FIG. 15E and FIG. 15F summarizes average OS of pan-SCC 5S subtype 2, stratified by SCC type.

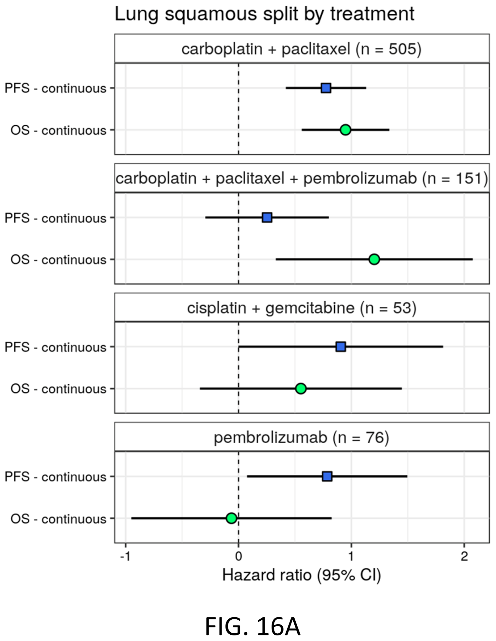

Subtype 2 probability is predictive across lung SCC for multiple regimens. All treatment was completed using naive primary samples. FIG. 16A shows lung squamous cells split by treatment. FIG. 16B shows survival probability based on treating lung SCC subjects with LUSC carboplatin and paclitaxel, stratified based on pan-SCC 5S subtype 2 status. Subjects who are assigned pan-SCC 5S subtype 2 have a lower survival probability. FIG. 16C shows number at risk over time, stratified based on pan-SCC 5S subtype 2 status. FIG. 16D shows different treatment regiments.

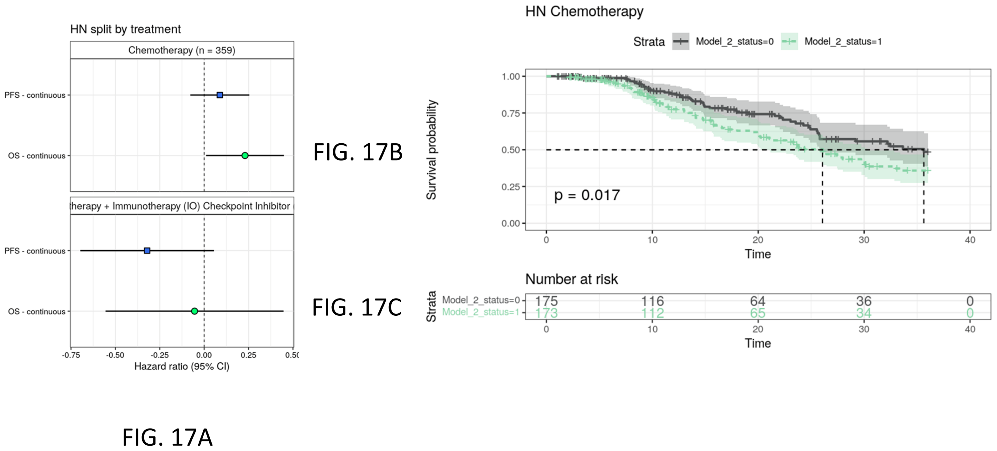

Subtype 2 probability is predictive for chemotherapy in head and neck. All pre-treatment samples, first line treatment for each of the regimen listen. FIG. 17A shows H&N split by treatment. FIG. 17B shows survival probability based on treating H&N SCC subjects with chemotherapy, stratified by pan-SCC 5S subtype 2 status. Subjects who are assigned pan-SCC 5S subtype 2 have a lower probability. FIG. 17C shows the numbers at risk over time stratified on pan-SCC 5S subtype 2.

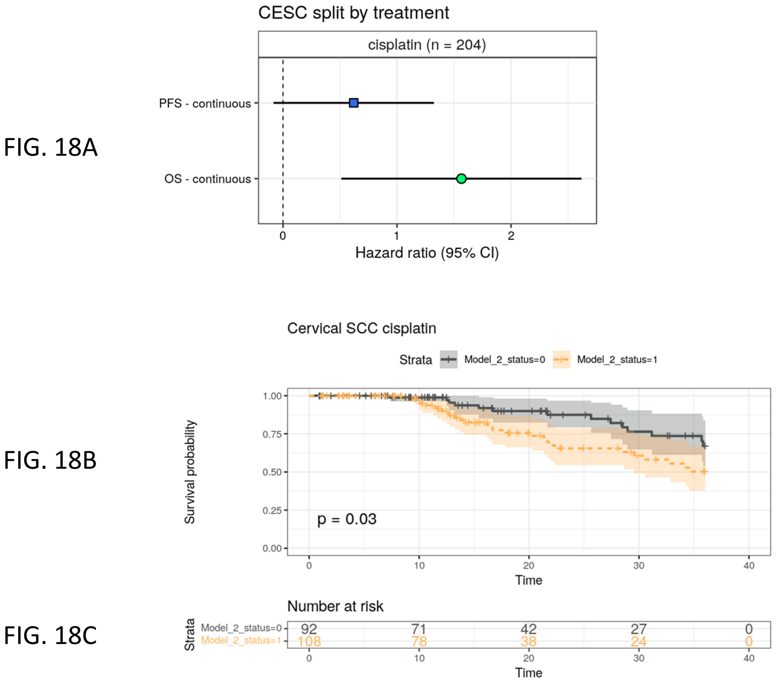

Subtype 2 probability is predictive for cisplatin in cervical SCC. All pre-treatment samples, first line treatment for each of the regiment listed. FIG. 18A shows CESC survivability based on treatment with cisplatin. FIG. 18B shows survivability probability of cervical SCC treated with cisplatin, stratified based on pan-SCC 5S subtype 2 status. Subjects who are assigned pan-SCC 5S subtype two have lower survival probability. FIG. 18C shows number at risk over time stratified by pan-SCC 5S subtype 2.

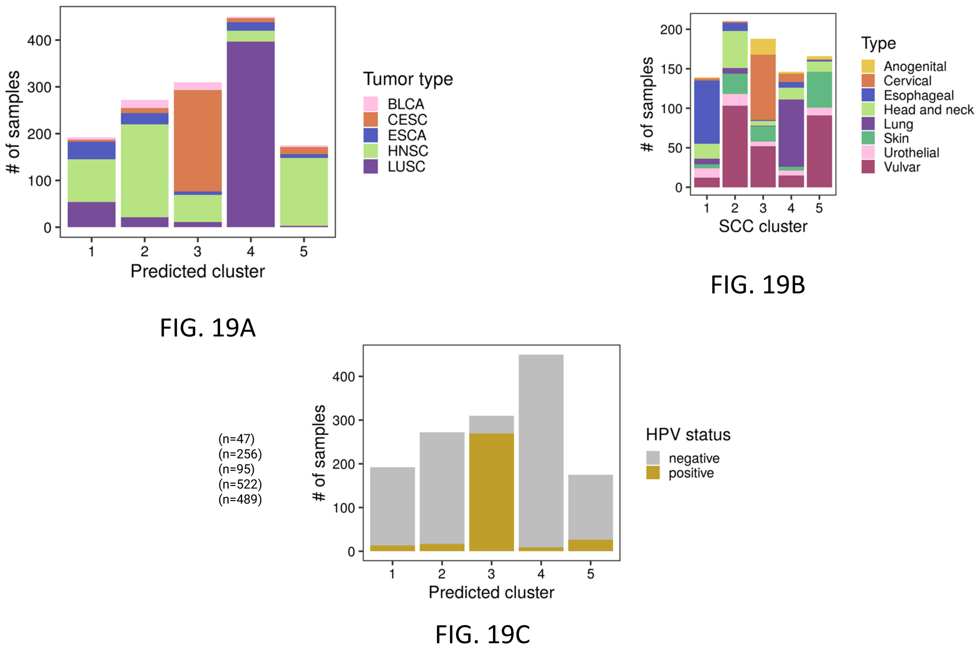

Next, the classifier was applied to The Cancer Genome Altas Program (TCGAf). TCGA verified SCC samples based on pathology confirmation was used for this analysis. FIG. 19A shows the number of samples in the TCGA database belonging to each pan-SCC 5S subtype, colored by tumor type. For comparison, FIG. 19B shows the number of SCC type samples in the pan-SCC 5S subtypes. HNSC in pan-SCC 5S subtype 1 were primarily from larynx/hypopharynx, physically closer to the esophagus. FIG. 19C shows the HPV status (by number of samples) stratified by pan-SCC 5S subtype.

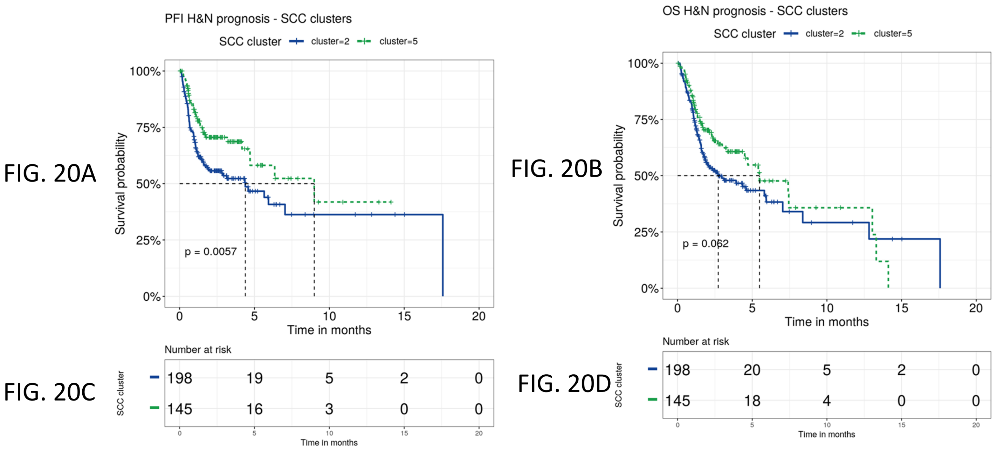

We then focused analysis on H&N samples from the TCGA database that were assigned pan-SCC 5S subtype 2 or subtype 5. FIG. 20A shows the survivability probability (PFI) stratified by pan-SCC 5S subtype, and FIG. 20C shows the corresponding number at risk. FIG. 20B shows the survivability probability (OS) stratified by pan-SCC 5S subtype, and FIG. 20D shows the corresponding number at risk. Using both PFI and OS as metrics, pan-SCC 5S subtype 2 has worse survival probability.

### Example 2 - Pan-SCC 6S subtypes and vSCC mapping - 6 clusters

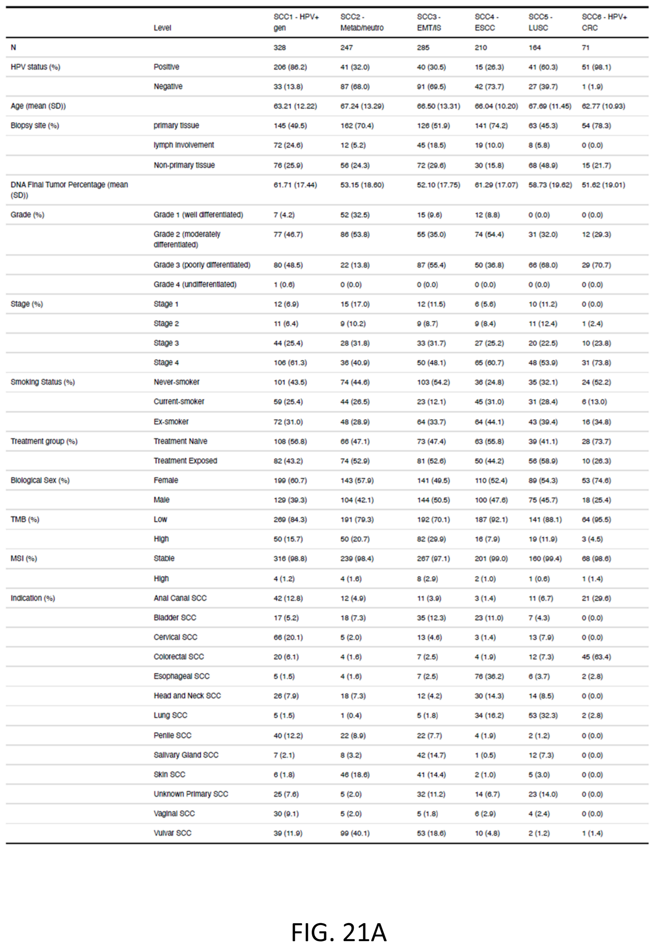

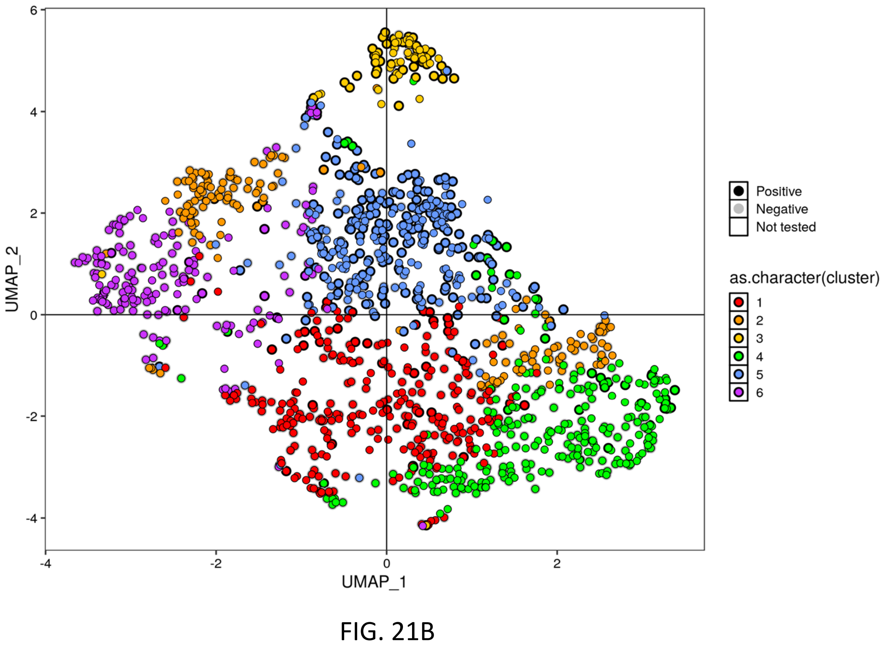

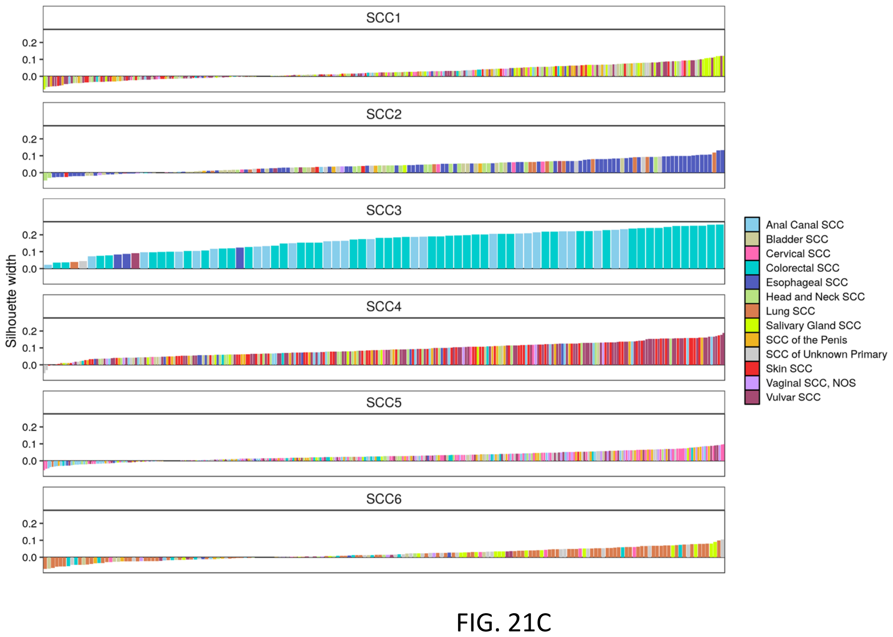

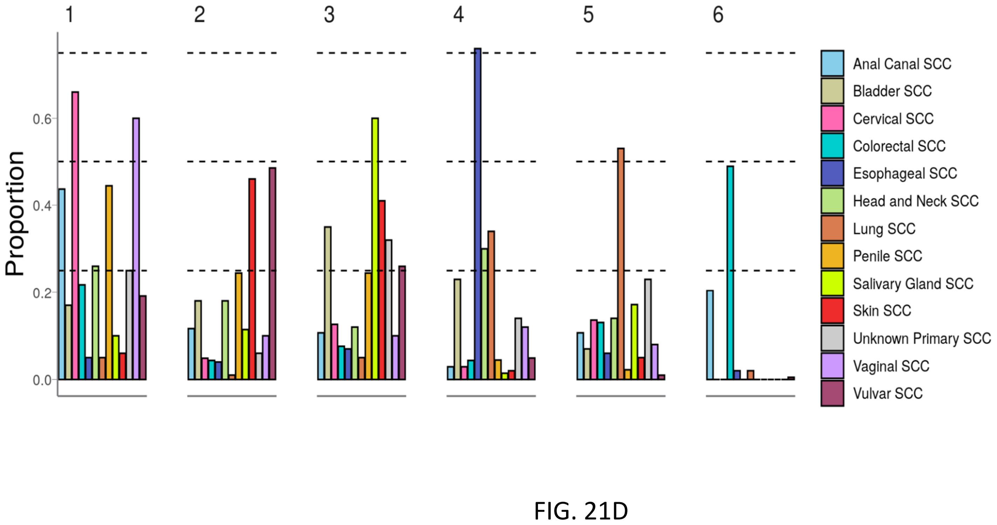

Unbiased graph-based clustering of transcriptomic data identified six clusters from the pan-SCC cohort (see FIG. 21A and FIG. 21B, *see Methods*)*.* The pan-SCC subtyping led to stronger cluster cohesion than the SCC tissue types by mean Silhouette width (cancer type = -0.0129, cluster = 0.04). The Silhouette widths went from a negative mean value to a positive, indicating that clustering led to more cohesive groups (FIG. 21C). The vSCC samples were split mostly across three subtypes; *SCC1, SCC2,* and *SCC3,* with the majority of vSCC samples (99/204) assigned to *SCC2* (FIG. 21A, FIG. 21D). Similarly, Skin SCC was also predominantly assigned to *SCC2.* In contrast, Anal Canal, Cervical, Penile, and Vaginal SCC were most frequently assigned to *SCC1,* while Bladder and Salivary Gland SCC were most frequently assigned to *SCC3* (FIG. 21A, 21C). The majority of esophageal SCC samples (76/100) corresponded to *SCC4,* Lung SCC predominantly corresponded to *SCC* (53/100) and Colorectal SCC corresponded to *SCC6* (45/92)*.* 98% (51/52) of SCC6 and 86% (206/239) of SCC1 samples were HPV positive, while the majority of SCC2, SCC3, and SCC4 samples were HPV negative (FIG. 21A).

*SCC1 - HPV+ genital*/*anal* was one of two HPV+ clusters, with 86% (n=206/239) of the samples being HPV+; including 66% of the cervical cancer samples, 60% of vaginal cancer, 44% of penile and anal canal SCCs, 26% of head and neck, 25% of the SCCs of unknown primary, and 22% of colorectal SCC. This subtype was enriched in cell proliferation related modules (HALLMARK_E2F_TARGETS: Q= 2x10⁻²⁷, HALLMARK_G2M_CHECKPOINT: Q= 1x10⁻¹²) and was enriched in B-cell expression (Q=5x10⁻⁵).

**Table 8 shows the top 100 genes in the pan-SCC 6S subtype 1.**

| Table 8: pan-SCC 6S Subtype 1 | | | | |
|---|---|---|---|---|
| | | | | |

| Gene | Score | | ACTBL2 | -0.015738663 |
|---|---|---|---|---|
| CRACDL | 0.017993792 | | TCEA3 | 0.015704859 |
| DPF1 | -0.017746989 | | EPB41L4B | -0.01559809 |
| RAX | 0.017569328 | | CT62 | -0.015493089 |
| GATM | 0.016715792 | | DKK3 | -0.015441422 |
| KLHL35 | 0.016469693 | | FJX1 | -0.015179696 |
| TMEM236 | -0.016356504 | | CASP5 | -0.015166602 |
| MANEAL | 0.014589354 | | ANKRD65 | 0.011807574 |
| NUP210 | 0.013736357 | | ZDHHC19 | -0.011765721 |
| RPL10L | -0.013473221 | | IGF2BP2 | -0.011719201 |
| FOXF2 | -0.013439383 | | KLF8 | 0.011718834 |
| LIPG | -0.013367577 | | TACSTD2 | 0.011702321 |
| GRID2 | 0.01330491 | | CCDC166 | 0.011643321 |
| C2orf48 | 0.013208988 | | TRIL | 0.011576482 |
| SH3TC2 | -0.013148342 | | ZP4 | -0.01154551 |
| MECOM | 0.013014394 | | SHISAL2A | 0.011526069 |
| SPACA5 | 0.012961891 | | TMT1B | -0.011492571 |
| SHC4 | -0.012924398 | | ADGRE1 | -0.011481807 |
| R3HDML | -0.012860867 | | OCM | 0.011474119 |
| BRME1 | 0.012815564 | | PIWIL2 | 0.011457887 |
| L1TD1 | -0.012761385 | | SNCB | -0.011434885 |
| ZAR1 | 0.012675172 | | PDPN | -0.01135823 |
| SLC28A1 | 0.012670819 | | RASD2 | -0.011332543 |
| FAM169A | -0.012633846 | | NICOL1 | -0.011306238 |
| FEV | -0.012595604 | | COLEC10 | -0.011303649 |
| SPMIP11 | 0.012552449 | | GJE1 | 0.011286532 |
| GLI1 | -0.012526138 | | EGR3 | -0.011230959 |
| CRYBB2 | -0.012524522 | | RIBC2 | 0.011217705 |
| KIRREL3 | -0.012517271 | | SLC26A5 | 0.011214708 |
| PI15 | -0.012396998 | | SLC2A12 | -0.011193868 |
| FEZ1 | -0.01236558 | | GABRB1 | -0.011167396 |
| C2CD4B | 0.012344215 | | SGCG | -0.011147746 |
| PLEKHG4 | 0.012331033 | | GABRA2 | -0.011139656 |
| GOLGA6L10 | 0.012294369 | | FAM81A | 0.011136079 |
| GRIN2C | 0.012270933 | | ATP8A2 | -0.011038323 |
| CELF5 | -0.012247513 | | USP2 | -0.011036264 |
| TSPAN18 | -0.012185604 | | RAPGEFL1 | 0.01103572 |
| CARD10 | -0.01216493 | | NAALADL2 | 0.010983501 |
| ACOD1 | -0.012113965 | | CCDC185 | 0.010980679 |
| PLCH1 | -0.012095758 | | NANOG | 0.010977772 |
| AR | 0.01204578 | | HTR2C | -0.010960212 |
| MTNR1A | -0.012024977 | | SLC10A4 | 0.010956826 |
| PPP1R14C | -0.012024064 | | PHACTR3 | 0.010877195 |
| B4GALNT3 | -0.012016956 | | NPSR1 | -0.010875009 |
| ESR1 | 0.011989555 | | TRH | 0.01086772 |
| PITX1 | 0.011962525 | | PMP2 | -0.010864153 |
| PRSS46P | 0.011942653 | | HBEGF | -0.010836783 |
| CHRNA3 | 0.011915187 | | C22orf31 | 0.010803649 |
| DNAJB13 | 0.011912478 | | LVRN | -0.010798711 |
| RET | -0.011899689 | | ZSWIM5 | 0.010751326 |
| PAX8 | 0.011820482 | | | |

*SCC2 - Metab*/*neutro (metabolism*/*neutrophils)* was composed of 49% of the vulvar SCC, 46% of the vSCC samples, and 24% of the penile SCC. SCC2 was mostly strongly enriched for TNFa signaling (Q= 9.5x10⁻⁶), P53 pathway (Q= 6x10⁻⁵), metabolism of RNA (Q= 8x10⁻⁴) and fatty acids (REACTOME_PHOSPHOLIPID_METABOLISM: Q= 2x10⁻², REACTOME_SPHINGOLIPID: Q= 3x10⁻²), and expression of neutrophils (Q= 1x10⁻³).

**Table 9 shows the top 100 genes in the pan-SCC 6S subtype 2.**

| Table 9: pan-SCC 6S Subtype 2 | | | TUBB2A | 0.0139617 |
|---|---|---|---|---|
| | Score | | MMP27 | 0.013959335 |
| ARG1 | 0.020273448 | | HOPX | 0.013728285 |
| TREX2 | 0.019640277 | | MS4A2 | 0.013691067 |
| CMA1 | 0.019291295 | | KRT33B | 0.013493095 |
| KRTAP5-4 | 0.018531438 | | ESYT3 | 0.013481988 |
| LIPM | 0.018256754 | | GALNT6 | 0.013450421 |
| SPTLC3 | 0.017894902 | | DEGS2 | 0.013334907 |
| GCSAML | 0.017407909 | | LIPN | 0.013247409 |
| HAL | 0.017397364 | | IL37 | 0.013137718 |
| LGALSL | 0.017212258 | | ACKR2 | 0.013100571 |
| VSIG8 | 0.017161992 | | LCE1D | 0.013097837 |
| TMC4 | -0.017056414 | | HTR3A | 0.013028445 |
| ELMOD1 | 0.016881342 | | DCT | 0.012872085 |
| SMPD3 | 0.016799544 | | RARB | -0.012808705 |
| ACER1 | 0.016309421 | | OPN1MW | 0.012724486 |
| ABCG4 | 0.016256501 | | SPAG11B | 0.012709755 |
| ATP6V1C2 | 0.016046397 | | FLG2 | 0.012593798 |
| TPPP2 | 0.016035344 | | DEFB105B | 0.012573452 |
| DCD | 0.015955599 | | VIPR1 | 0.012562262 |
| ELOVL4 | 0.01578585 | | LCE1A | 0.012438538 |
| KRT25 | 0.015656766 | | SPACA5 | -0.012438478 |
| RNF222 | 0.015635131 | | SCGB1D2 | 0.012432681 |
| ACSBG1 | 0.015407411 | | GLB1L3 | 0.012412967 |
| ANKRD31 | 0.015361124 | | TEX28P2 | 0.012403744 |
| MELTF | -0.015334556 | | HDC | 0.012302431 |
| NPM2 | -0.01529908 | | PTGS 1 | 0.012260221 |
| FRMPD1 | 0.015291 | | RDH16 | 0.012246258 |
| ENDOU | 0.015243143 | | KRT80 | 0.012243612 |
| LCE5A | 0.015188853 | | CIDEA | 0.012115824 |
| USP2 | 0.015117458 | | SCN4B | 0.012090058 |
| LCE1B | 0.015019388 | | HYAL4 | 0.012072735 |
| DGAT2 | 0.015010188 | | CTSG | 0.012071695 |
| LCE1E | 0.014974665 | | GPR63 | -0.012025843 |
| PNPLA1 | 0.014802223 | | TYR | 0.012015414 |
| SERPINA12 | 0.014772134 | | LELP1 | 0.012015154 |
| SYT17 | -0.014734624 | | LYPD5 | 0.011984487 |
| TMEM45A | 0.014642944 | | SCGB2A2 | 0.01197324 |
| CCL27 | 0.014535421 | | HOXD1 | -0.011964317 |
| LCE6A | 0.014314033 | | TEX28P1 | 0.011955493 |
| RDH12 | 0.014212621 | | RHBG | 0.011933557 |
| ASPRV1 | 0.014055934 | | FLG | 0.011862803 |
| XKRX | 0.014047841 | | AADACL3 | 0.011838878 |
| BPIFC | 0.011833437 | | TEX28 | 0.011573153 |
| TRPM1 | 0.011782294 | | IL1F10 | 0.011485111 |
| OPN1LW | 0.0117086 | | LORICRIN | 0.011447853 |
| NEU2 | 0.011708249 | | GATA3 | 0.011444512 |
| NSG1 | 0.011696716 | | PTPN5 | 0.01137705 |
| MECOM | -0.01169315 | | NWD2 | 0.011376454 |
| GALNT12 | -0.01166234 | | KRT84 | 0.011359274 |
| COX8C | -0.011582045 | | WNT16 | 0.011333459 |

*SCC3 - EMT*/*IS (Epithelial*/*mesenchymal transition*/*immunosuppressive)* was the most heterogeneous by cancer type, comprising 60% of the included salivary gland SCCs, and 41% of skin, 35% of bladder, 32% of SCC of unknown primary, 26% of vulvar SCC. SCC2 had a very similar gene expression profile compared to ***V1** - **HPVneg***; with the strongest signal for EMT (Q = 1x10⁻⁴⁸), TNFa signaling via NFkB (Q = 4x10⁻³⁰), IFNg (Q=8x10⁻²⁸), cancer associated fibroblasts (Q = 4x10⁻¹¹) and T-regulatory cells (Q = 3x10⁻⁶), as well as significantly enriched for KRAS signaling, JAK/STAT signaling, and apoptosis (Q<1x10⁻⁶).

**Table 10 shows the top 100 genes in the pan-SCC 6S subtype 3.**

| Table 10: pan-SCC 6S Subtype 3 | | | RNF183 | -0.013149855 |
|---|---|---|---|---|
| | pan-SCC subtype 3 | | VSIG8 | -0.013089037 |
| RAB25 | -0.018793723 | | DNAI7 | -0.012866875 |
| TTLL10 | -0.017807636 | | C22orf31 | -0.012853555 |
| SGPP2 | -0.017796372 | | FAM181A | -0.01283432 |
| SPINK9 | -0.016776743 | | GSTA4 | -0.012810787 |
| IGSF9 | -0.016526871 | | ALG1L2 | -0.012788087 |
| ARHGEF26 | -0.015888365 | | PLS1 | -0.012783174 |
| PIR | -0.015301937 | | BMP7 | -0.012720584 |
| RAPGEFL1 | -0.015164893 | | CFAP73 | -0.012699186 |
| CIMAP2 | -0.015083112 | | EFCC1 | -0.012668817 |
| SCNN1A | -0.014565503 | | ISL2 | -0.012483916 |
| ZBTB7C | -0.014436749 | | ENDOU | -0.012441141 |
| BDNF | -0.014148643 | | L1CAM | 0.012358909 |
| ACSBG1 | -0.01414026 | | CYP4X1 | -0.01231455 |
| PGAP4 | -0.014109064 | | GPX2 | -0.012314351 |
| ZNF711 | -0.013999547 | | IL20RA | -0.012261997 |
| ACP3 | -0.013876261 | | COMMD5P1 | -0.012161256 |
| TMEM125 | -0.013709537 | | SOX1 | -0.012157489 |
| CLDN4 | -0.013654373 | | PCP4L1 | -0.012120902 |
| GGT6 | -0.013579215 | | KRTAP5-2 | -0.011952429 |
| P2RY1 | -0.013562493 | | FA2H | -0.011928996 |
| C1orf210 | -0.013512019 | | SAMD12 | -0.011889457 |
| OTX1 | -0.013499078 | | SRXN1 | -0.011870607 |
| CSN3 | 0.013284493 | | GRID2 | -0.011805714 |
| ESYT3 | -0.013271244 | | TRH | -0.011790048 |
| TTC39A | -0.01323696 | | TLCD4-RWDD3 | -0.011722939 |
| RNF225 | -0.011606693 | | ACTBL2 | 0.010910553 |
| MCIDAS | -0.011579822 | | VAX2 | -0.010873775 |
| NDRG4 | -0.011568187 | | ZDHHC11 | -0.010854473 |
| PRR35 | -0.011500991 | | ART3 | 0.010832572 |
| CCN3 | -0.011499872 | | MYH14 | -0.01081514 |
| LIPM | -0.011490576 | | TGFBI | 0.010785928 |
| OVOL2 | -0.011478764 | | C2orf48 | -0.010782586 |
| CGN | -0.011428174 | | LINC02898 | -0.010776047 |
| POU2F3 | -0.011426005 | | CFAP276 | -0.010772134 |
| HOPX | -0.011424843 | | PLA2G3 | -0.010740208 |
| DOC2B | -0.011384264 | | GCSAML | -0.010722378 |
| RBBP8NL | -0.011382497 | | MYOM3 | 0.010721528 |
| B4GALNT3 | -0.011267556 | | FGFR2 | -0.010720817 |
| SPOCK1 | 0.011201232 | | ALG1L1P | -0.010715599 |
| GLYATL1 | -0.011189577 | | KLHDC7A | -0.010699049 |
| SRRM3 | -0.011149924 | | OPRK1 | -0.010676626 |
| BSPRY | -0.011096108 | | POF1B | -0.01066549 |
| CACNA2D3 | -0.011092193 | | CBX2 | -0.010574471 |
| PHGDH | -0.011021991 | | CEACAM1 | -0.010570965 |
| BCL2L15 | -0.011018168 | | THBS1 | 0.010550241 |
| B3GNT6 | -0.010993343 | | NEBL | -0.010540636 |
| ZNF385C | -0.010962648 | | CCDC185 | -0.010468631 |
| VEGFC | 0.010960509 | | C20orf144 | -0.01045251 |
| EBF3 | 0.010914584 | | CHODL | -0.010439461 |

**SCC4 - ESCC** was composed of 76% of the esophageal SCCs, 34% of the lung SCC, and 30% of the head and neck, and 23% of bladder SCCs. SCC4 had the highest enrichment of MTOR and MYC pathways (Q = 1x10⁻⁶ and Q= 2x10⁻⁶ respectively), glycolysis (Q = 2x10⁻⁴), and similarly to SCC2 but with higher enrichment scores, this subtype was enriched in metabolism; protein, RNA, and cholesterol metabolism (Q < 1x10⁻²).

**Table 11 shows the top 100 genes in the pan-SCC 6S subtype 4.**

| Table 11: pan-SCC 6S Subtype 4 | | | BDNF | 0.015512692 |
|---|---|---|---|---|
| | pan-SCC subtype 4 | | PIR | 0.015424356 |
| OSGIN1 | 0.01953795 | | OR6C2 | 0.015336466 |
| SRXN1 | 0.018887271 | | ME1 | 0.015144018 |
| G6PD | 0.017731886 | | GPAT3 | 0.014986886 |
| ETNK2 | 0.01767256 | | NQO1 | 0.014827457 |
| DGKG | 0.017117811 | | TRIM16L | 0.01426287 |
| MDGAl | 0.016312847 | | JAKMIP3 | 0.014041993 |
| ODC1 | 0.016298614 | | NECAB2 | 0.013874729 |
| RAB3B | 0.0162786 | | GLI2 | 0.013656823 |
| GATA3 | -0.016219797 | | SLC38A8 | 0.013652881 |
| PLCXD2 | 0.015853888 | | CYP2S1 | 0.013346484 |
| GSTM2 | 0.015635841 | | GSTM3 | 0.013326654 |
| WNT5A | 0.015597092 | | CCL28 | -0.013156004 |
| GPX2 | 0.012948595 | | RND2 | 0.010943444 |
| NOG | -0.012886984 | | SGCZ | 0.01094297 |
| C1QTNF12 | 0.012815433 | | SAMD12 | 0.010917281 |
| TSPAN7 | 0.012647893 | | HAP1 | 0.010914323 |
| OR56B4 | 0.012624203 | | BRD2 | 0.010893447 |
| SCN9A | 0.012613168 | | DAZ3 | -0.010830938 |
| NKX6-1 | 0.012582504 | | AKR1C3 | 0.010825829 |
| GLI1 | 0.012472652 | | ENPP3 | -0.010784913 |
| PANX2 | 0.012423211 | | ANO1 | 0.010783773 |
| CFAP20DC | 0.012362456 | | MACROD2 | -0.010752357 |
| C1orf226 | 0.0123006 | | UPKlB | 0.010748313 |
| ENTHD1 | 0.012232437 | | JAKMIP2 | 0.010717644 |
| SLC7A11 | 0.012190303 | | AKR1C4 | 0.010660345 |
| UGT1A1 | 0.012185849 | | ETNPPL | -0.010644107 |
| MST1R | -0.012105614 | | PFN2 | 0.010624474 |
| AKR1C1 | 0.012010662 | | ANXA10 | 0.010615149 |
| RAB6B | 0.011952934 | | LRRC2 | -0.010613753 |
| H4C9 | -0.011932446 | | ZDHHC2 | 0.01061364 |
| CCDC125 | -0.011648477 | | NUDT11 | 0.010562052 |
| VPS37D | 0.01159562 | | CNTN6 | -0.01049453 |
| DPF1 | 0.011579642 | | SLC4A3 | 0.010454108 |
| SLC6A13 | 0.011557387 | | ALDH3A1 | 0.010447198 |
| B4GALNT3 | 0.011541667 | | TMC1 | 0.010437303 |
| GCNT2 | 0.011436105 | | OR6C70 | 0.010437128 |
| GASK1A | -0.011382211 | | DLG2 | -0.010413941 |
| CCL26 | 0.011361519 | | CIMAP2 | 0.010412149 |
| NR0B1 | 0.011279877 | | VIPR1 | -0.010401568 |
| KLRG1 | -0.011256115 | | SPTLC3 | -0.010349558 |
| ARTN | 0.011251865 | | KIT | -0.010346439 |
| NRCAM | 0.011202357 | | CYP26A1 | 0.010329867 |
| ELAPOR2 | 0.011134441 | | ROR1 | -0.010326915 |
| KCND3 | -0.011104544 | | PMP2 | 0.01031163 |
| TPRG1 | 0.011085586 | | NYAP1 | 0.010309255 |
| ZMAT1 | -0.011071624 | | FGF 13 | 0.010304782 |
| OTOP2 | 0.011049401 | | SAMD3 | -0.010233198 |
| RORC | -0.011009673 | | S100A5 | 0.010210074 |
| PCYT1B | 0.010981078 | | LGSN | 0.010187559 |

**SCCS - LUSC** 53% of the lung SCC, 25% of the salivary gland tumors, and 24% of the SCCs of unknown primary. SCC5 had limited gene set enrichment compared to the other SCC subtypes, but was significant for metabolism of steroid hormones (Q=0.1). This may indicate that the gene expression modules chosen did not accurately capture the biology of this subtype.

**Table 12 shows the top 100 genes in the pan-SCC 6S subtype 5.**

| Table 12: pan-SCC 6S Subtype 5 | | | | pan-SCC subtype 5 |
|---|---|---|---|---|
| SFTA3 | 0.021704573 | | ABCC6 | 0.011491362 |
| GGTLC1 | 0.018284353 | | KHDRBS2 | 0.011490811 |
| NAPSA | 0.018174679 | | PLA2GlB | 0.011484964 |
| SFTPD | 0.017551136 | | SPEF2 | 0.011454388 |
| MS4A15 | 0.017184186 | | SCN1A | 0.011420704 |
| VWA3A | 0.017003737 | | CFAP276 | 0.011326516 |
| ANKRD66 | 0.01621871 | | WFDC6 | 0.011290785 |
| HABP2 | 0.016152903 | | SLC22A31 | 0.011283049 |
| CPAMD8 | 0.016123286 | | RGPD3 | 0.011279808 |
| KCNK3 | 0.016056604 | | KRTAP10-9 | 0.01127418 |
| CFAP95 | 0.015925169 | | DNAI1 | 0.011064357 |
| CFAP43 | 0.015080993 | | ACSM1 | 0.011034976 |
| CFAP221 | 0.015057014 | | RAB6C | 0.011012979 |
| NKX2-1 | 0.014791401 | | CFAP65 | 0.011001811 |
| FOXB1 | 0.014629798 | | MARCHF10 | 0.01099464 |
| C16orf89 | 0.014536098 | | CDHR3 | 0.0109832 |
| C8B | 0.014208973 | | FRMPD2 | 0.010968648 |
| NEK5 | 0.014165826 | | DNAI7 | 0.010853065 |
| LRP2 | 0.014131549 | | ERICH2 | 0.010850967 |
| AQP4 | 0.014083154 | | DNAH12 | 0.010783332 |
| SLC9C2 | 0.013869371 | | ZNF648 | 0.010779431 |
| C4BPA | 0.013831717 | | CIMIP1 | 0.010778808 |
| TMEM212 | 0.013693109 | | GARIN6 | 0.010745369 |
| STOML3 | 0.013568523 | | ARMC3 | 0.010737985 |
| CDH7 | 0.013441726 | | HOATZ | 0.010734372 |
| KIAA2012 | 0.013180225 | | C2orf73 | 0.010702068 |
| DLG2 | 0.013120128 | | Clorf222 | 0.010676122 |
| TTC29 | 0.013119626 | | TEKT2 | 0.010636536 |
| USP44 | 0.012991212 | | CFAP90 | 0.010635709 |
| F11 | 0.01292816 | | AGBL1 | 0.010600467 |
| PPM1H | 0.012925318 | | SNTN | 0.010571056 |
| PGC | 0.012900569 | | DRC1 | 0.010534955 |
| SFTPB | 0.012825687 | | MIA2 | 0.010524184 |
| ODAD1 | 0.012812767 | | C4A | 0.0105 |
| CATSPERD | 0.012399384 | | RSPH1 | 0.010498379 |
| PEBP4 | 0.012330352 | | ASB4 | 0.010438101 |
| PLCH1 | 0.012295948 | | STMND1 | 0.01038919 |
| ZBBX | 0.012234549 | | DNAH5 | 0.010359067 |
| CFAP107 | 0.012233547 | | CABCOCO1 | 0.010358975 |
| C1orf87 | 0.012154978 | | NME5 | 0.010344941 |
| DAW1 | 0.012050804 | | HP | 0.010334063 |
| ROPN1L | 0.011941913 | | TSPAN19 | 0.010330369 |
| FYB2 | 0.011935711 | | CGNL1 | 0.010264974 |
| KCTD16 | 0.011836619 | | MALRD1 | 0.010242065 |
| C8orf34 | 0.011794874 | | SHISA3 | 0.01020754 |
| PCDHAC2 | 0.011695932 | | CNTN6 | 0.010166277 |
| CP | 0.011637337 | | SCGB3A2 | 0.010153208 |
| ERICH3 | 0.011538644 | | NRGN | 0.010150074 |
| RP1 | 0.011519613 | | XAGE1C | 0.010136624 |
| ABCA3 | 0.010133132 | | HYDIN | 0.01006243 |

Interestingly, **SCC6 - HPV+ CRC/anal** was also an HPV+ cluster (98% HPV+, n=51/52), but contained almost only colorectal SCCs and anal canal SCCs (49 and 20% respectively). This subtype was closer to the SCC1 HPV+ cluster. SCC6 had the highest enrichment of B-cell and activated B-cell modules (Q = 5x10⁻³ and 0.01 respectively) and fatty acid metabolism (Q = 0.02), and Th17 cells (Q=0.04).

**Table 13 shows the top 100 genes in the pan-SCC 6S subtype 6.**

| | | | | |
|---|---|---|---|---|
| Table 13: pan-SCC 6S Subtype 6 | | | MEP1A | 0.013356867 |
| | pan-SCC subtype 6 | | GCG | 0.013278899 |
| RNF186 | 0.020300491 | | CDHR2 | 0.01319159 |
| CCL15 | 0.020102327 | | CHST5 | 0.01309814 |
| TMIGD1 | 0.019139275 | | B3GNT7 | 0.012986436 |
| RPL10L | 0.017833975 | | ZG16 | 0.01295507 |
| ATOH1 | 0.01733829 | | GALNT8 | 0.01292738 |
| ANKS4B | 0.017177862 | | EFNA2 | 0.012829469 |
| ALPI | 0.016971098 | | TINAG | 0.012666181 |
| SLC17A4 | 0.016934882 | | LYPD8 | 0.012607134 |
| B3GNT6 | 0.016166541 | | SLC51B | 0.012522815 |
| MOGAT3 | 0.015974437 | | FABP2 | 0.01249894 |
| NR1I2 | 0.015783877 | | LEFTY1 | 0.012298619 |
| IHH | 0.015568939 | | HTR4 | 0.012261985 |
| MS4A12 | 0.015566887 | | CHGA | 0.012228712 |
| A1CF | 0.015512256 | | TM4SF5 | 0.012218846 |
| FEV | 0.015331799 | | MYO7B | 0.012147503 |
| CLRN3 | 0.015295512 | | LGALS4 | 0.012076676 |
| NHERF4 | 0.015059684 | | SLC6A19 | 0.012043497 |
| INSL5 | 0.015037448 | | CDX1 | 0.011995973 |
| R3HDML | 0.014969376 | | SI | 0.011965966 |
| GUCA2B | 0.014884757 | | RETNLB | 0.01196104 |
| NXPE1 | 0.014802388 | | PLA2G10 | 0.011902417 |
| MYO1A | 0.014519019 | | BCL2L15 | 0.011872343 |
| HNF1A | 0.014306873 | | TMEM236 | 0.011819566 |
| NAT2 | 0.014278278 | | SLC18A1 | 0.011799818 |
| PYY | 0.014266755 | | SAMD13 | 0.011773589 |
| NXPE4 | 0.014128069 | | CA7 | 0.011753975 |
| AQP8 | 0.014091187 | | HHLA2 | 0.011750988 |
| NOX1 | 0.014088032 | | SULT1B1 | 0.011735539 |
| REG3A | 0.014048101 | | C5orf52 | 0.011730596 |
| UGT2A3 | 0.014026692 | | GPA33 | 0.011714595 |
| TRIM15 | 0.013916026 | | REG1B | 0.011654382 |
| B3GALT1 | 0.013743354 | | GP9 | 0.011607045 |
| ISX | 0.013678394 | | HEPACAM2 | 0.011592709 |
| CDH17 | 0.013440282 | | LRRC31 | 0.011574343 |
| NXPE2 | 0.013382652 | | GUCA2A | 0.01153351 |
| REG4 | 0.011519951 | | AOC1 | 0.010781119 |
| VSIG2 | 0.011505245 | | ANXA13 | 0.010731299 |
| CLCA1 | 0.011418761 | | GUCY2C | 0.010652496 |
| SLC26A3 | 0.01139984 | | FAM135B | 0.010616392 |
| IYD | 0.01136244 | | CA1 | 0.01058997 |
| BNIP5 | 0.011321629 | | CAPN9 | 0.010546151 |
| GREM2 | 0.011294286 | | GABRA2 | 0.010542146 |
| SGK2 | 0.011277782 | | ALDOB | 0.010529747 |
| HGD | 0.01124772 | | SULT1C3 | 0.01051233 |
| VIL1 | 0.011221742 | | HNF4A | 0.010444489 |
| VSTM2A | 0.011076605 | | MUC12 | 0.010288634 |
| KRT20 | 0.010953411 | | PPP1R14D | 0.010223541 |
| SPMIP10 | 0.010935924 | | SPINK4 | 0.01021422 |
| SLC28A2 | 0.010827179 | | BTNL3 | 0.010189862 |

The mutation distribution fell similarly to what was observed by cancer type: HPV+ cluster (SCC1 and SCC6) had very similar mutational profiles, while the HPV negative were split into TP53 mutated (SCC4 and SCC4) vs. TP53/TERT mutated (SCC2 and SCC3). The HPV+ clusters, SCC1 and SCC6 had very low frequencies of TP53 (21 and 15% respectively), and high PIK3CA (41 and 35%), and KMT2D (23 and 26%). SCC1 and SCC6 differed in the frequency of TERT (0 and 14%, P=0.0006, one-sided Fisher's exact test), ZNF740 (19 and 6%, P=0.01). SCC2 and SCC3 had very similar mutation profiles, but had significantly different RET deep deletions (6 and 2% respectively, P=0.02). SCC4 and SCC5 both had high frequencies of TP53 (84 and 65% respectively) and similar frequencies of PIK3CA mutations (18 and 19% respectively), but had significantly different frequencies of NFE2L2, TP53, CCND1/FGF3/FGF4 amplification, and CDKN2A/CDKN2B/MTAP deletion (P<0.001).

The median tumor purity for all clusters ranged between 52% and 62% (FIG. 21A), with the lowest purity in *SCC2, SCC3, and SCC6. SCC6* displayed the most Stage 3 and 4 samples, with 97.6% (41/42) samples corresponding to these stages. In contrast, *SCC2* displayed the lowest proportion of Stage 3 and 4 samples, with 72% (64/88), and the lowest proportion of Grade 3 and 4 samples (13%, 22/160) (FIG. 21A). Moreover, *SCC6* contained the highest percentage of treatment naive samples (74%, n=28/38), while the other clusters contained between 41% and 57% treatment naive samples (FIG. 21A). *SCC2*, *SCC4*, and *SCC6* were predominantly collected from primary tissue (70.4%, 74.2%, and 78.3%, respectively) (FIG. 21A). Over 97% of samples in all six clusters were MSS, while over 80% of samples in most clusters were TMB low (FIG. 21A). The highest proportion of TMB high samples (30%, 82/274) and MSI (3%, 8/275) were observed in SCC3. Finally, the lowest proportion of never-smoker samples were observed in SCC4 - ESCC (24.8%, 36/145) and SCC5 - LUSC (32.1%, 35/109), and the highest proportion of never-smoker samples were found in SCC3 - EMT/IS (54.2%, 103/190) and SCC6 - HPV+ CRC (52.2%, 24/46) (FIG. 21A).

### pan-SCC 6S subtype relatedness

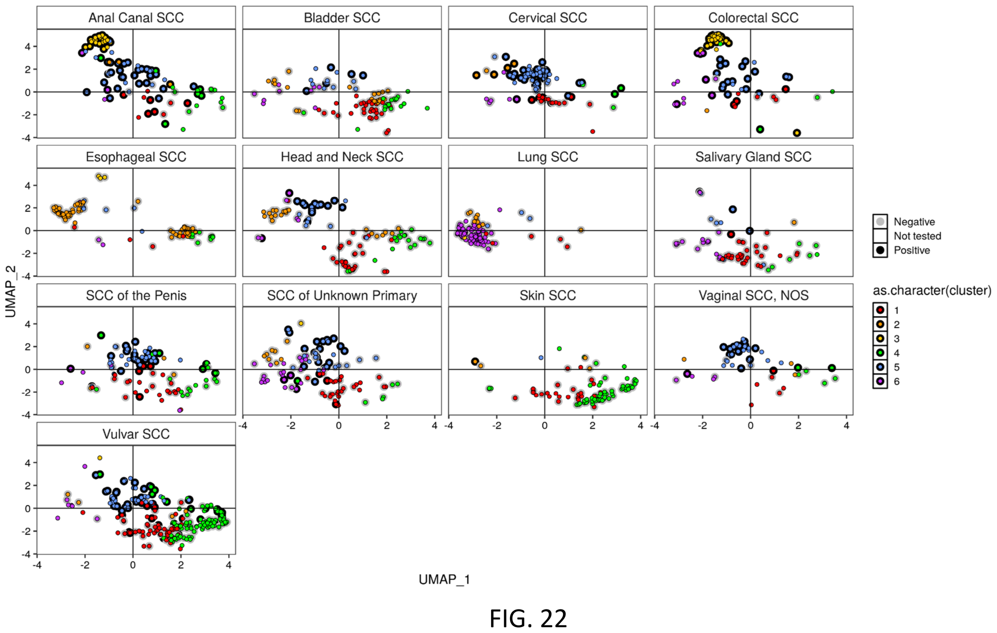

We characterized the genetic similarity of the pan-SCC 6S clusters . UMAP analysis was completed, and UMAP 1 and UMAP 2 were plotted against one another, grouped by pan-SCC 6S subtype. We plotted the UMAP1 and UMAP 2 of each SCC type, colored by the sample tissue source match. We then plotted the UMAP1 and UMAP2 of each SCC type, colored by 6 pan-SCC subtype (FIG. 22). FIG. 21C shows the proportion of each SCC type in each pan-SCC 6S subtype.

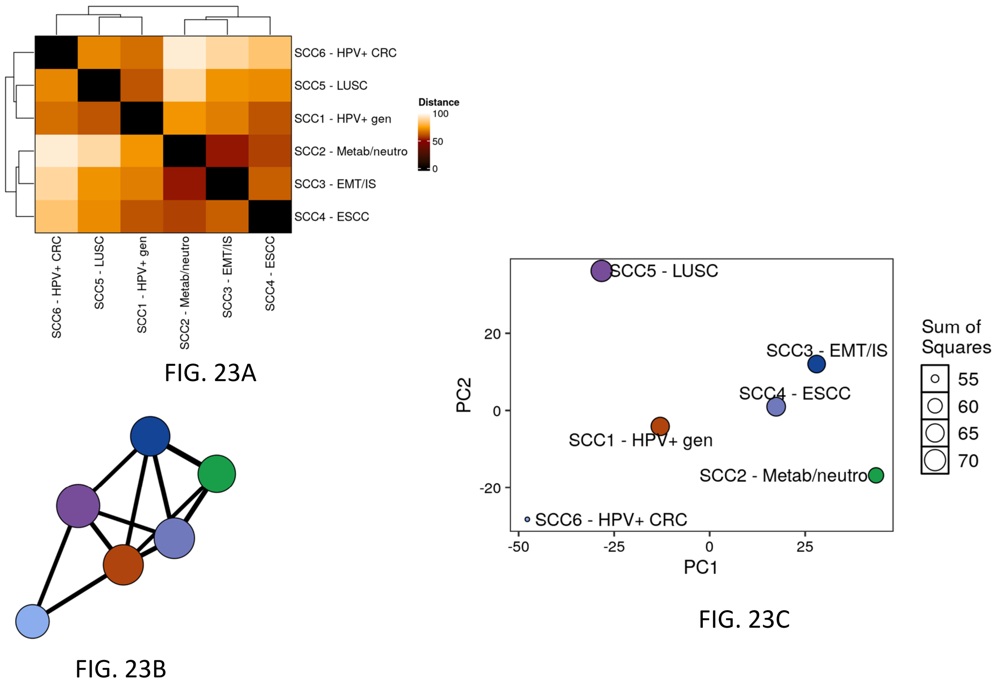

*SCC2 - Metab*/*neutro* and *SCC3 - EMT*/*suppre* were the closest by Euclidean distance (FIG. 23A, distance = 52), followed by *SCC2 - Metab*/*neutro* and *SCC4 - ESCC* (distance = 54). Interestingly, *SCC6 - HPV+ CRC* which contains mostly colorectal SCCs was closest to *SCC1 - HPVpos gen*, the other HPV cluster (distance=66), but overall had the highest distance mean between it and the other clusters (mean distance = 67). *SCC1 - HPVpos gen* had the lowest mean distance (mean distance = 54), meaning it was the cluster which was closest to the other clusters, followed by *SCC4 - ESCC* (mean distance = 56). FIG. 23B shows a graphical representation of the relations between pan-SCC 6S subtypes based on Euclidean distance. The nodes connected based on Euclidean distances, and the edges were pruned if > 75^{th} quartile of the distance. The weight of the edges represents the distance. Size of vertex is based on sum of squares. PCA was done on the pan-SCC 6S subtypes. PC1 and PC2 were plotted against one another, and the subtypes were sized based on the sum of squares (FIG. 23C). Interestingly, *SCC6 - HPV+ CRC* had the lowest sum of squares within the cluster and the highest mean silhouette width, meaning the samples within the cluster were the most similar based on Euclidean distance, whereas the *SCC5 - LUSC* had the largest sum of squares and the lowest silhouette width (FIG. 23C).

### Create a pan-SCC 6S subtype classifier

In order to expand the cohort for outcomes analyses, we created a pan-SCC Subtype Classifier Model using gene expression as the features into multinomial ridge regression, a machine learning method (see FIG. 1B for exemplary steps to train a model). We split the pan-SCC 6S cohort in half for training and testing. Within the test dataset, each model had high sensitivity and specificity (FIG. 24A): SCC1 (94% and 94%, respectively), SCC2 (93% and 98%), SCC3 (87% and 98%), SCC4 (91% and 99%), and SCC5 (84% and 99%), and SCC6 (88% and 100%). From this classifier, each sample had six probabilities calculated, one for each of the subtypes, which summed to one. The subtype assignment was determined using the highest score.

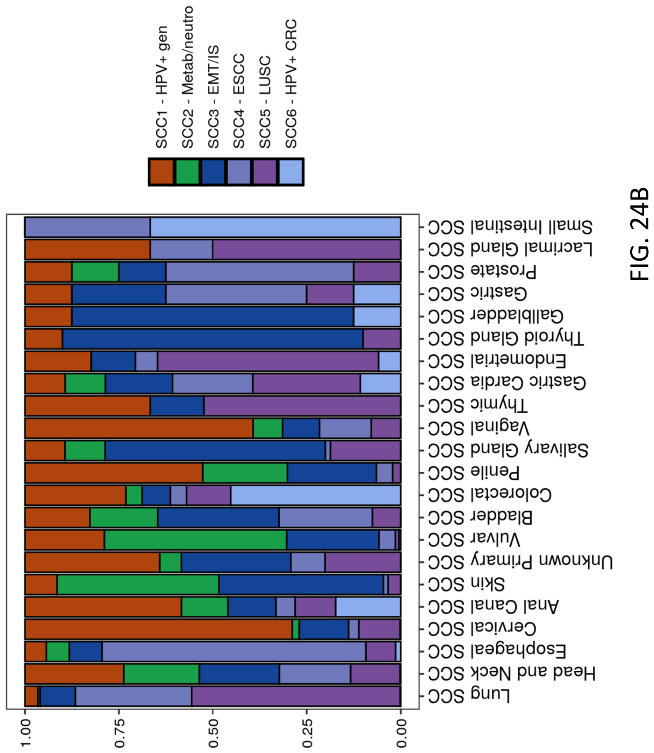

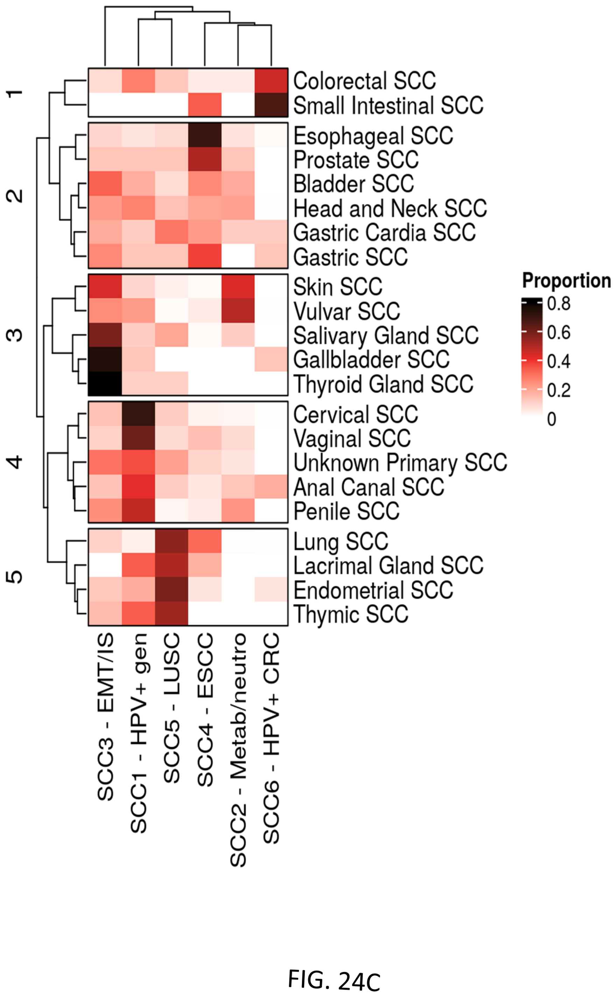

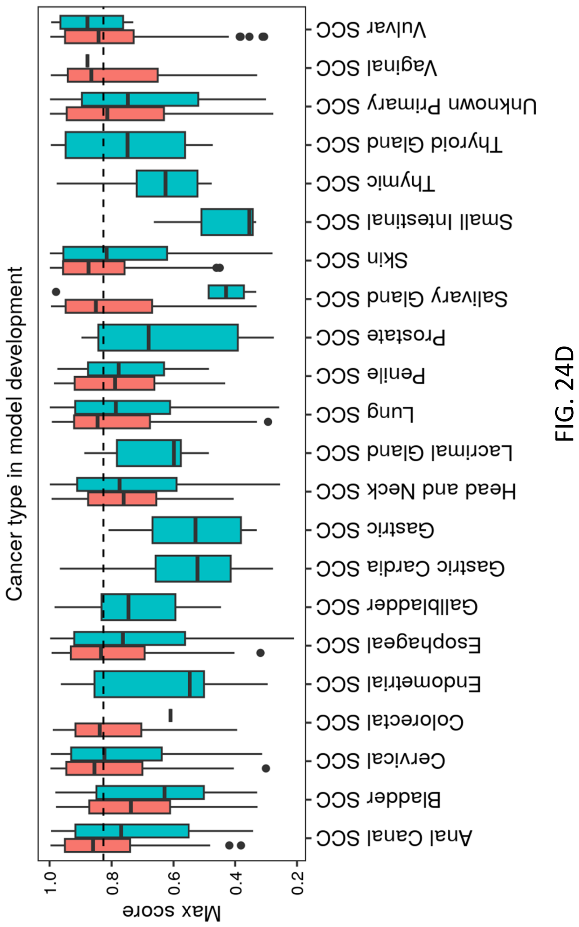

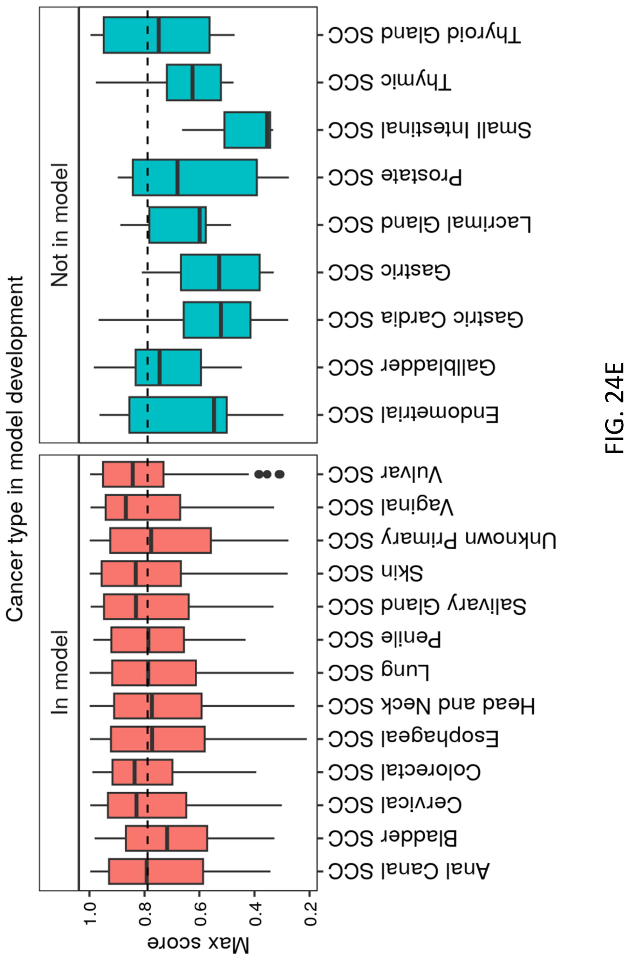

We applied this classifier to additional samples in all the SCC tumor types within the Tempus data (n=14,140), including expression from nine additional SCC cancer types which had <50 samples in the Tempus database. The nine additional cancer types had lower probabilities compared to the cancer types included in the model development as expected, but some cancer types had probabilities comparable to those used in model development, such as gallbladder and thyroid SCC (FIG. 24B, FIG. 24D, FIG. 24E). Samples not included in model development in anal canal, esophageal, salivary gland, and skin SCC had significantly lower probabilities (Wilcox test, P<0.05), however samples chosen for model development (FIG. 24D, FIG. 24E). As expected, the proportions of cancer types were similar to the original cluster assignments (FIG. 24C); the majority of cervical, esophageal, and lung SCC were predicted to be in the same clusters as previously defined (SCC1, SCC4, and SCC5 respectively).

### Pan-SCC 6S classifier associations with outcomes

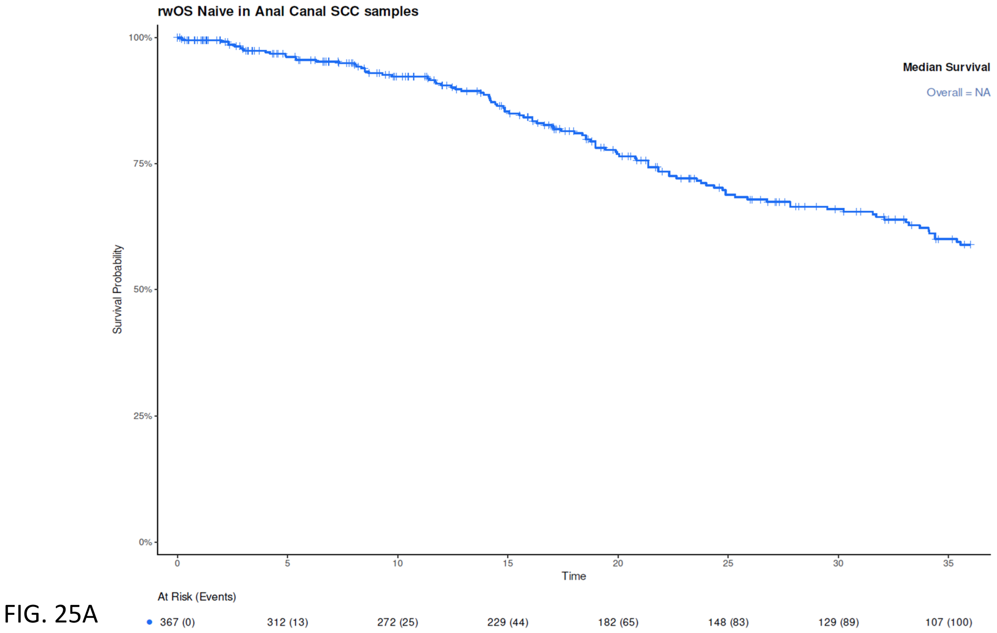

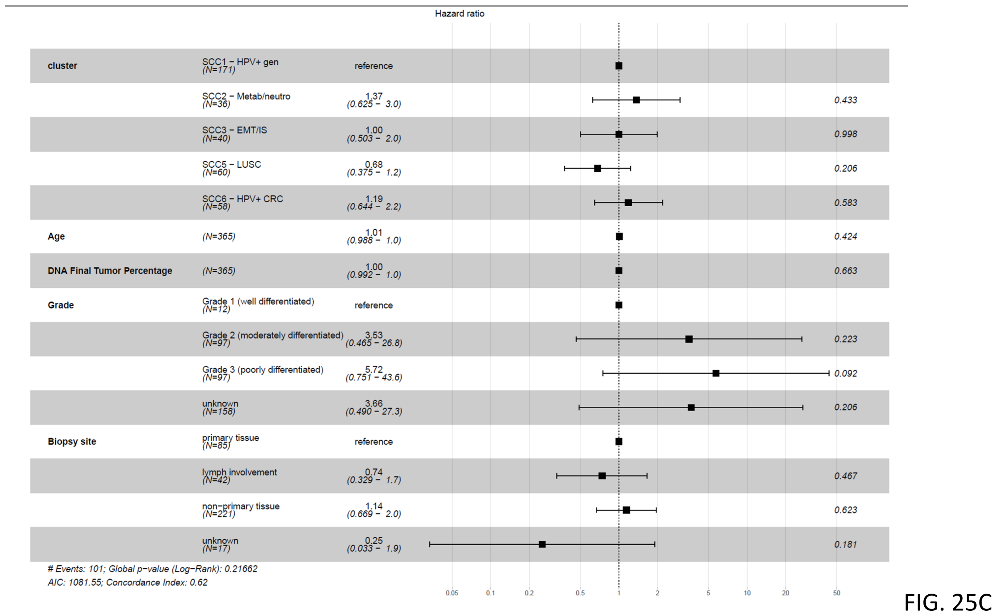

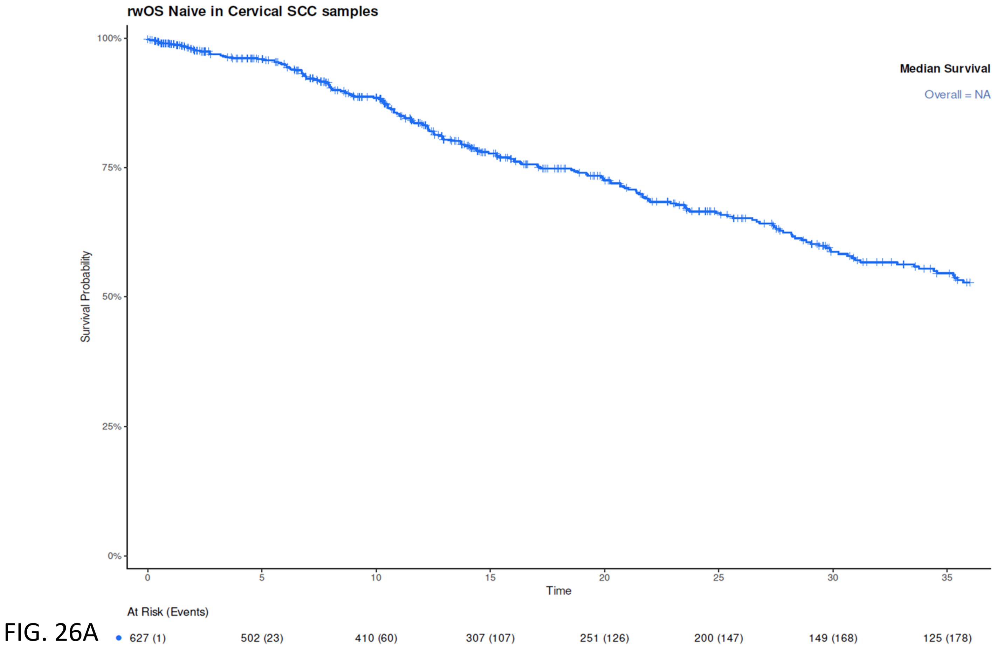

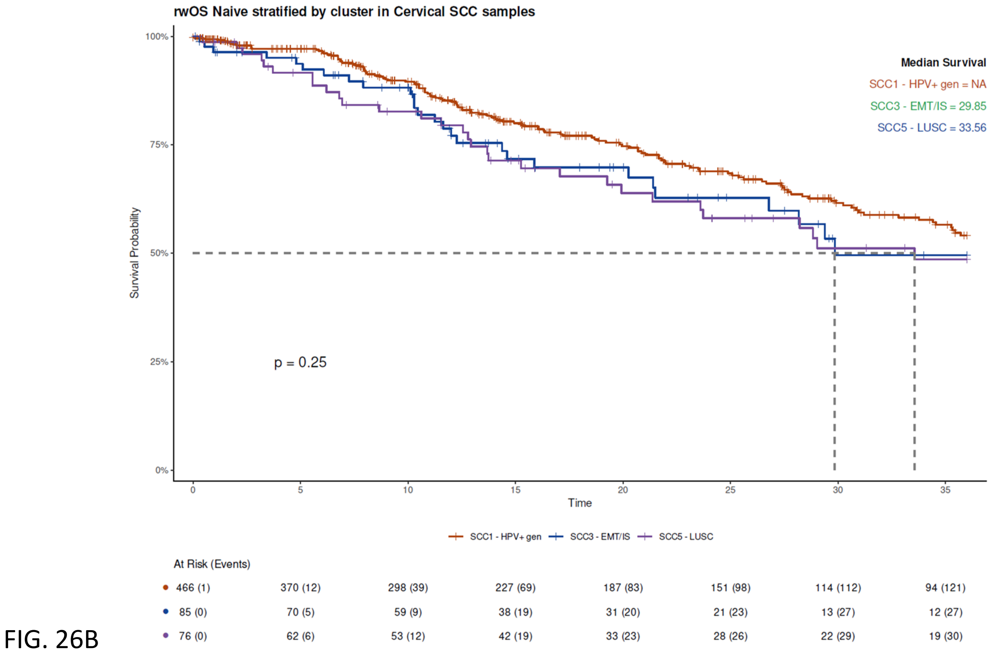

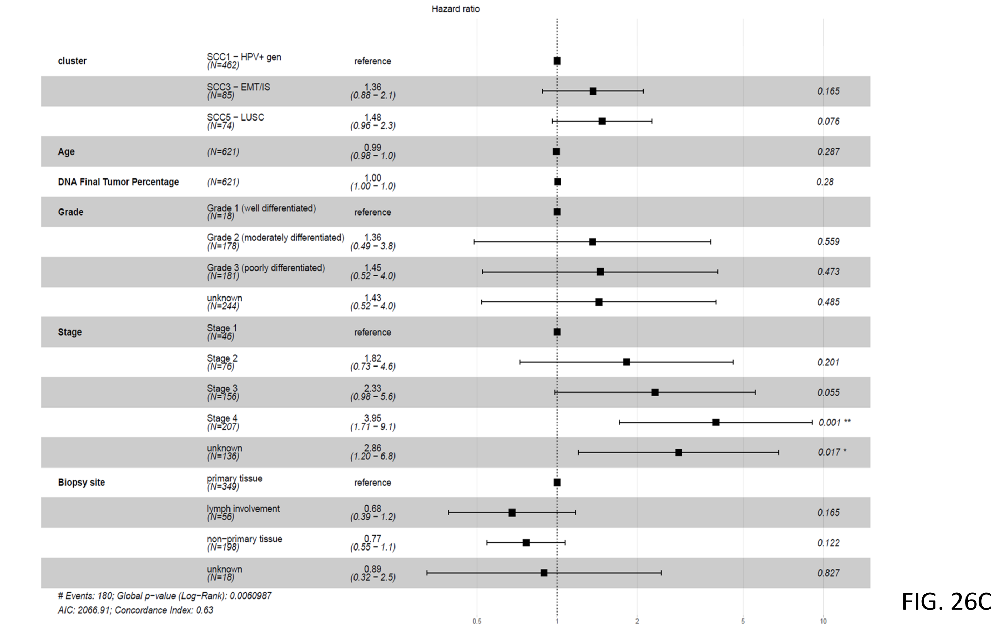

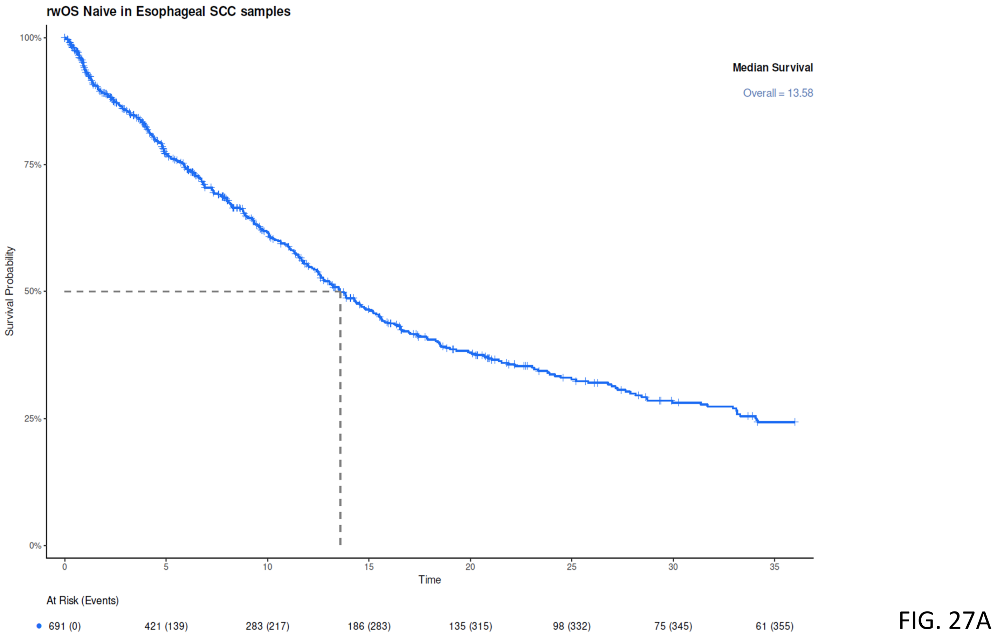

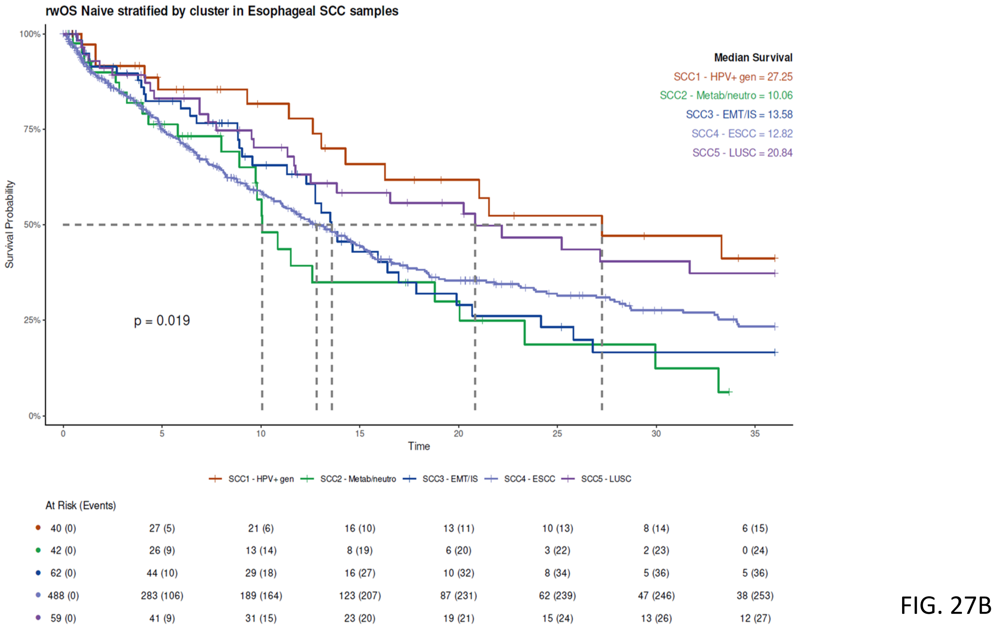

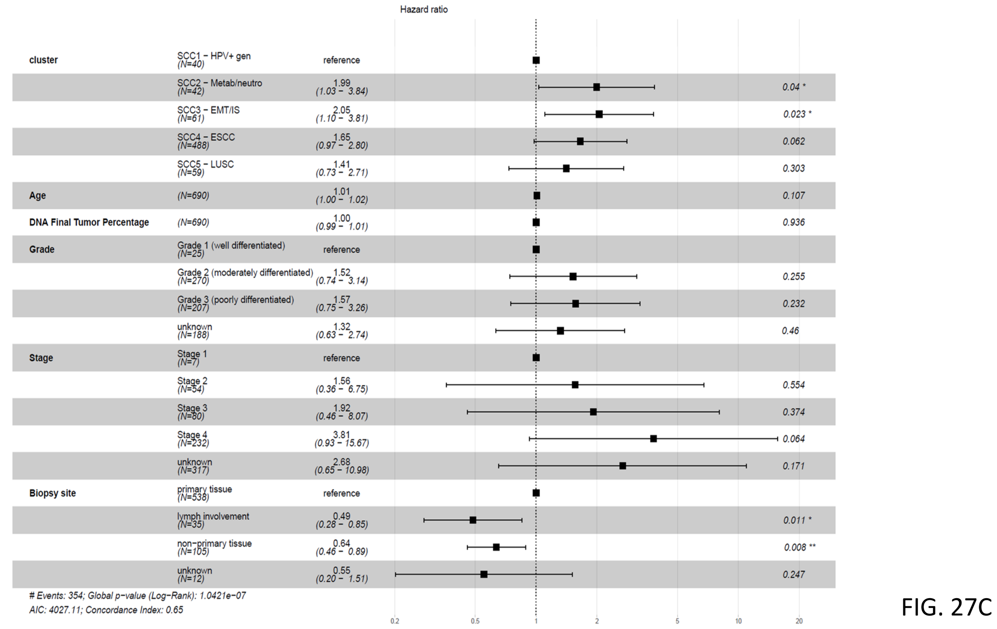

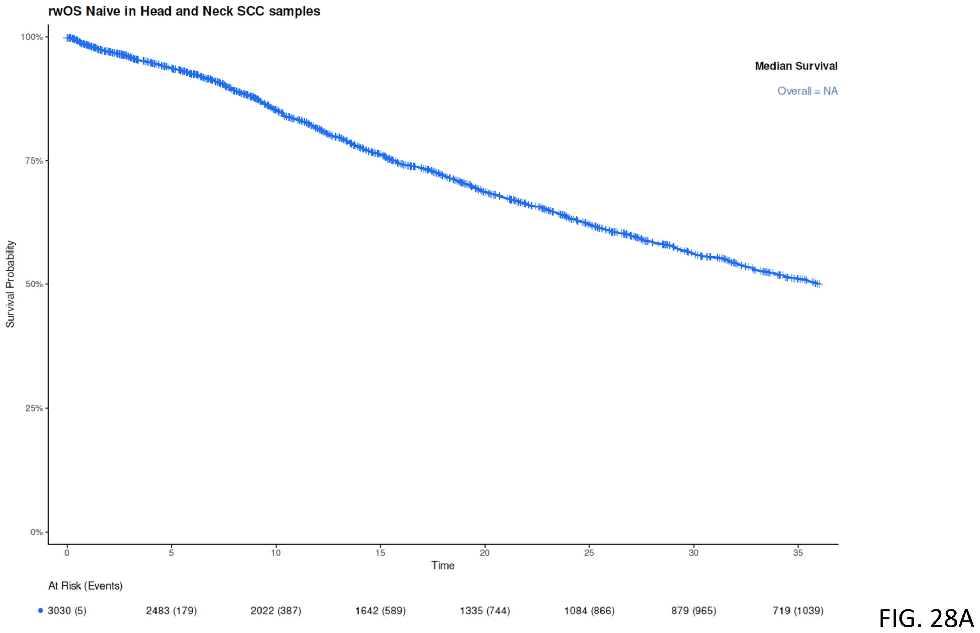

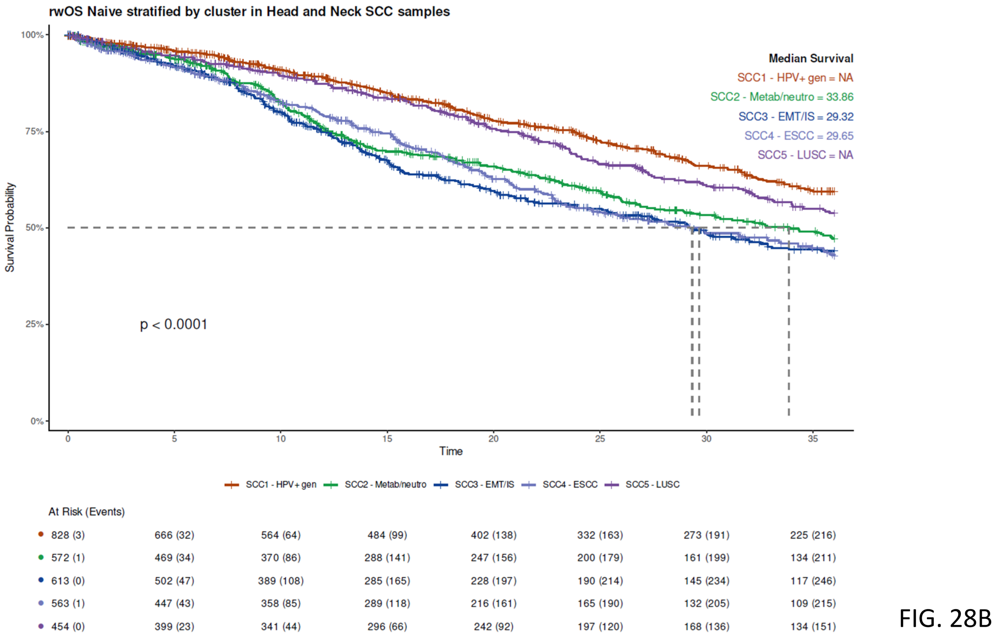

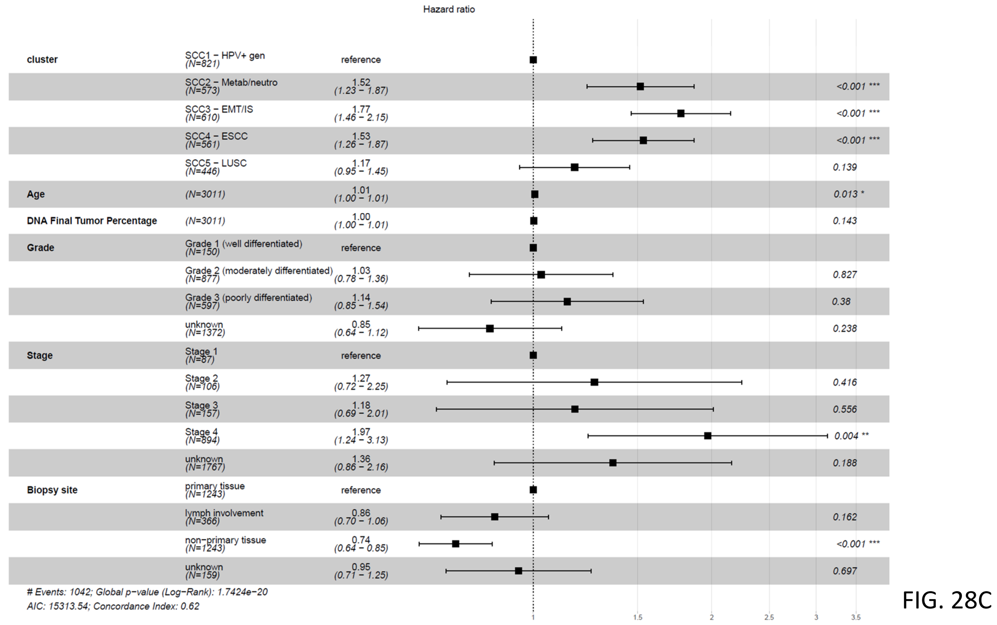

We selected six SCC types to analyze for survivability. For each cancer type, we determined the rwOS survivability over time for all samples, and then the survivability based on stratified data, in which the data was stratified over the pan-SCC 6S subtypes. Finally, we summarized the hazard ratio for a given cancer based on different characteristics, including pan-SCC 6S clusters, age, DNA final tumor percentage, tumor grade, tumor stage, and biopsy site. This was completed for Anal (FIG. 25A-25C), cervical (FIG. 26A-26C), esophageal (FIG. 27A-27C), H&N (FIG. 28A-28C), lung (29A-29C), and vulvar (BKA-BKC).

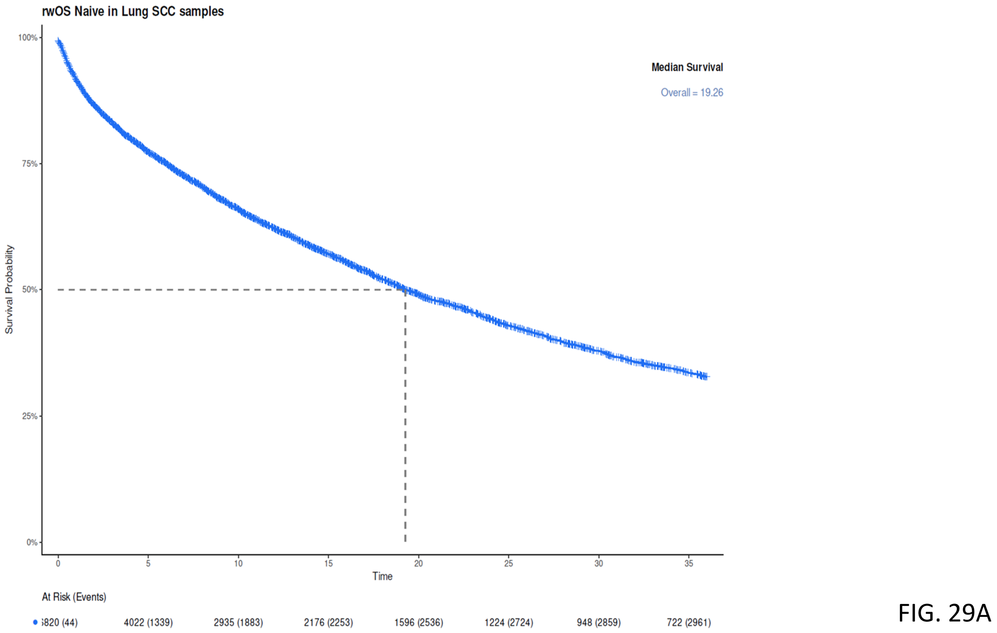

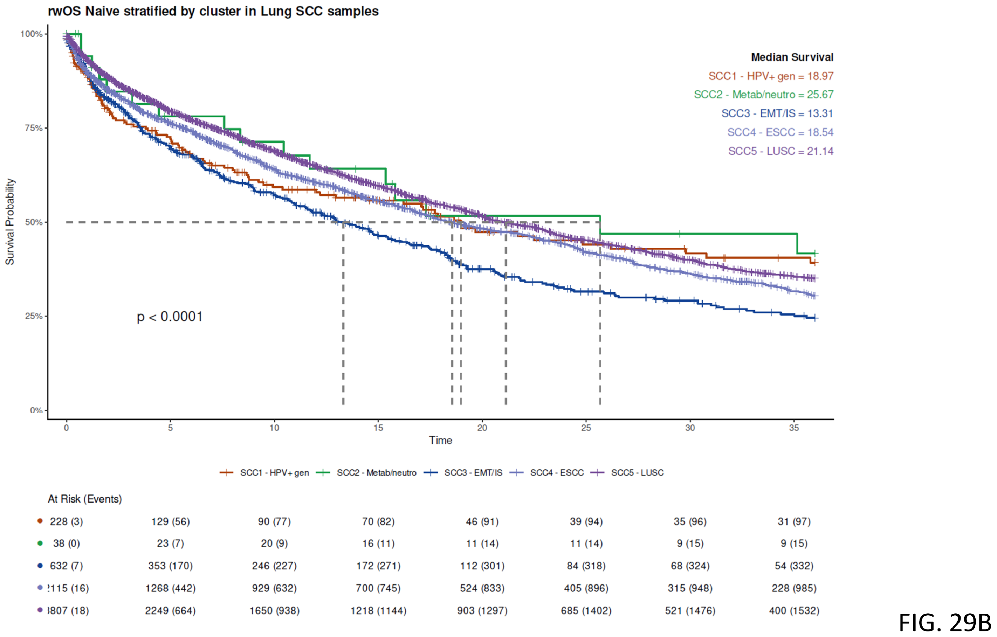

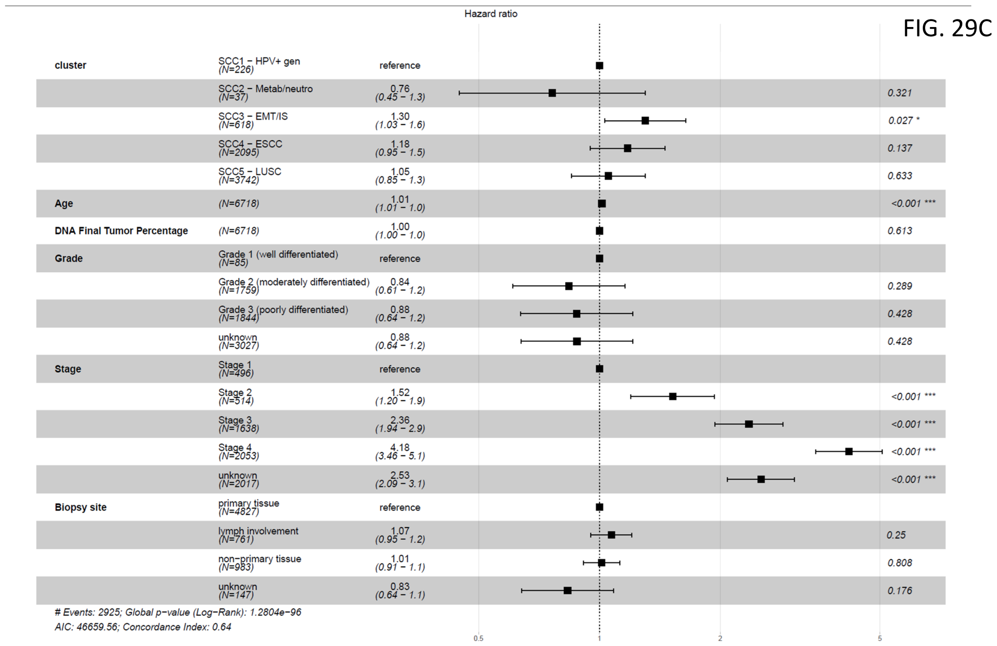

From both the subtype scores and the subtype assignments, we found strong association with outcomes. As expected, in vSCC and head and neck, the patient samples assigned to 6 pan-SCC subtype 3 (SCC3) had a higher overall survival (OS) compared to those in other subtypes (P=X, FIG. 29B). There were three cancer types with >50 samples in both SCC2 and SCC5; vSCC, HN, and skin SCC. For all three of those cancer types, SCC2 had a worse OS compared to SCC5 To ensure that this was not associated with a confounding variable, we checked for age, sex, drug status.

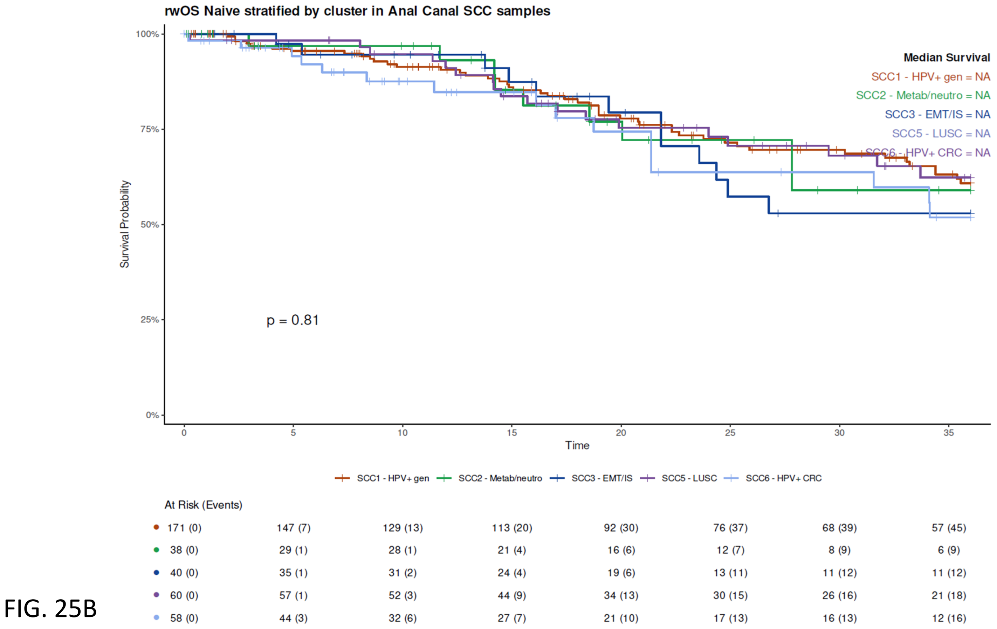

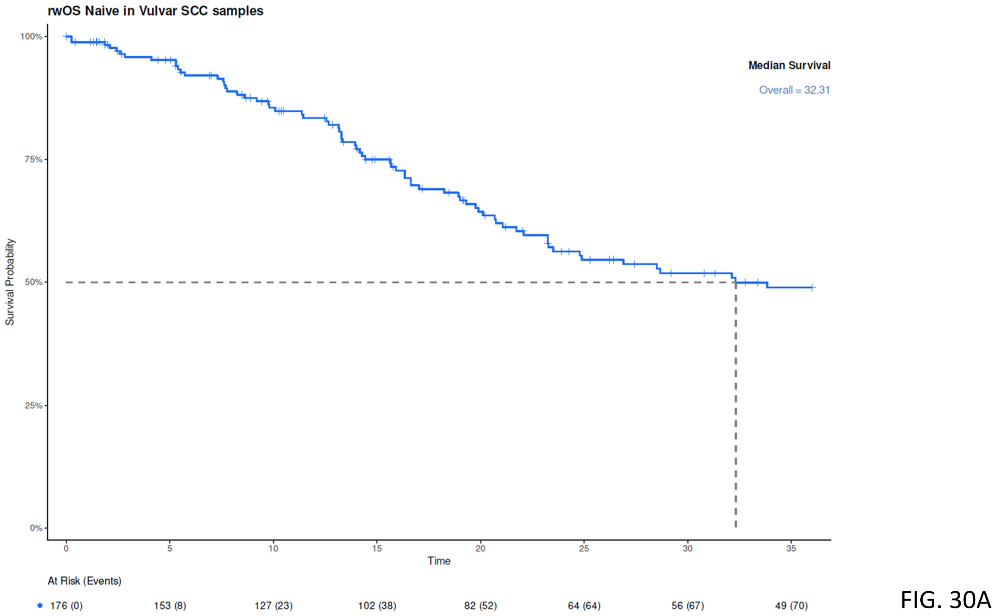

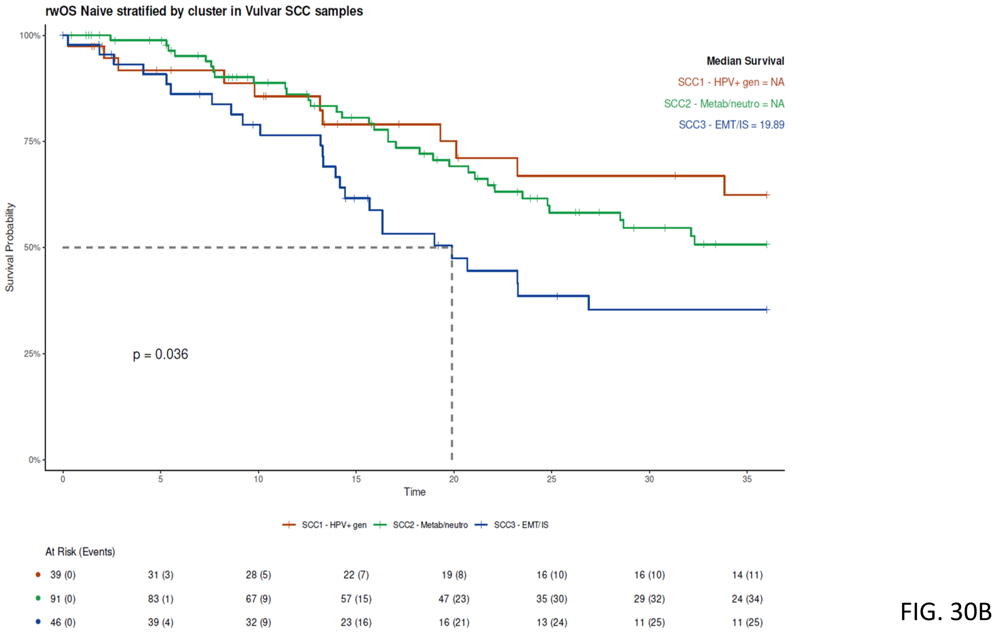

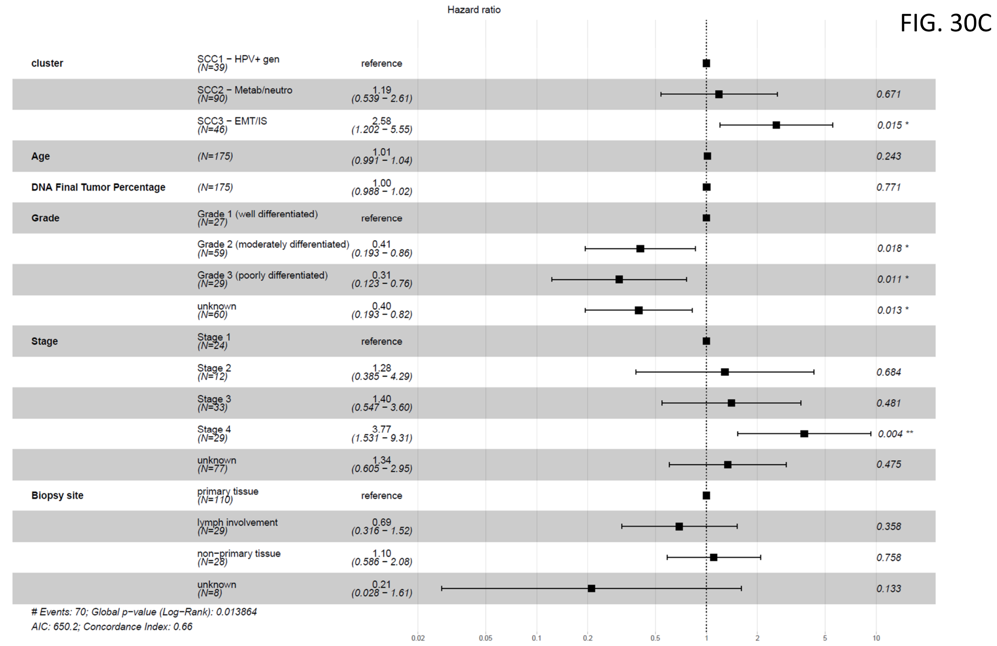

We tested the six SCC subtype probability scores across the different SCC cancer types and found that SCC2 was significantly associated with OS across all the SCC cancer types (FIG. 25B, 26B, 27B, 28B, 29B, 30B). The SCC2 model was most strongly enriched in EMT, tumor specific keratinocytes, inflammatory pathways, and cancer associated fibroblasts. To ensure that the consistent OS was not due to the EMT signature itself, we tested the association of EMT with OS and found inconsistent significance and tested EMT as a covariate with SCC2 and found significance after controlling for that signal.

We next tested if the SCC2 score was associated with OS when limiting to naive treatment patient samples within an individual drug regimen. The cancer type/drug regimen combinations we tested that had high enough power were LUSC, CESC, and HN within chemo treated, and LUSC with chemo + pembro. We found consistently significant OS.

### Apply pan-SCC 6S model to TCGA

We applied the pan-SCC 6S Subtype Classifier Model to TCGA to test model robustness in an independent cohort. TCGA had fewer SCC cancer types compared to the Tempus data, which included lung SCC, head and neck SCC, cervical SCC, esophageal SCC, and bladder cancer. We limited the TCGA samples to the ones pathology-confirmed as being SCC from other studies.

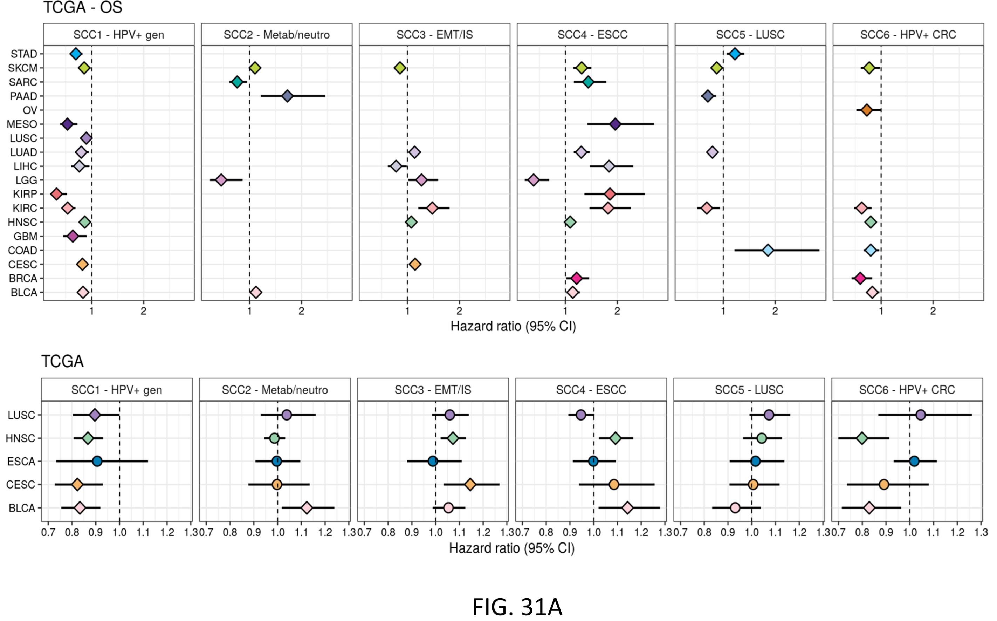

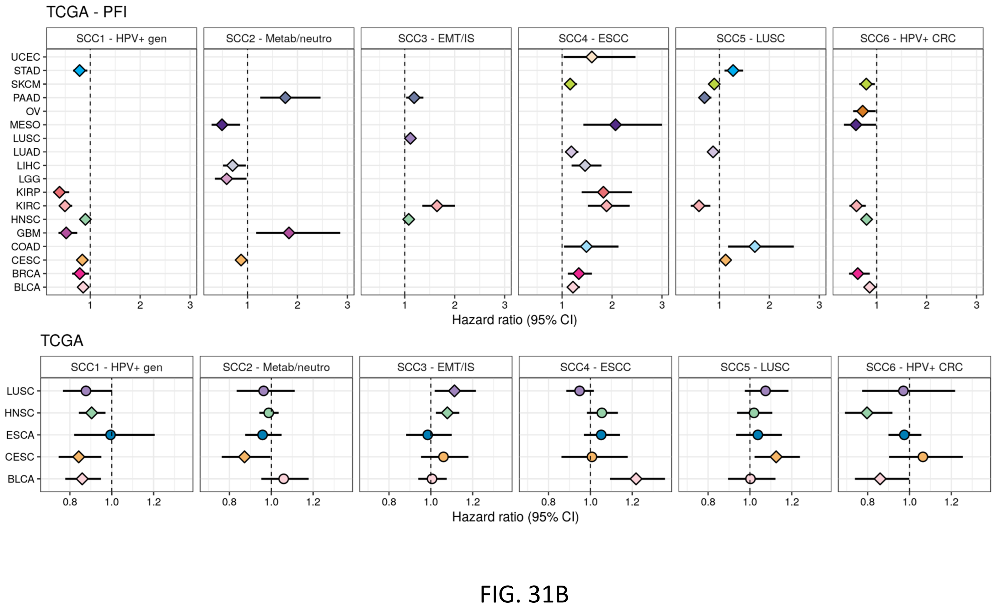

After applying the model, we found a similar tissue-type distribution as Tempus data (FIG. 31A, FIG. 31B).

### Association with outcomes

We tested the association of outcomes measurements from the TCGA clinical paper (OS, PFI, and DFI) with the SCC subtype assignments in HN alone since HN was the only cancer type with enough samples in multiple subtypes. By both PFI and OS, consistent with findings in the Tempus RWD, SCC2 in HN had worse outcomes compared to SCC5.

We next tested the association of each subtype score in the SCC samples only and found X associations.

Lastly, we applied the model to all TCGA and tested the association of outcome scores by cancer types defined by TCGA (some cancer types contained a mixture of adenocarcinoma and squamous cell). We found that eleven cancer types' SCC2 probability scores were associated with OS (P<0.05).

### Methods

### Subject selection

De-identified SCC records were selected from a database, and vulvar cancers annotated with squamous histology and available RNA-seq were selected for analysis. Samples derived from lung and liver metastases were excluded from analysis due to the background effect on gene expression. The pan-SCC cohort was limited to randomly samples (for cohorts >100 samples) primarly, naive to any treatment, female samples with paired RNA- and DNA-seq from 7 additional SCC types. The pan-SCC cohort includes: lung (n=100), head and neck (n=100), skin (n=100), urothelial (n=49), cervical (n=100) anogenital (n=27) esophageal (n=100) and vSCC (n=273).

We analyzed all the available genomics data for vulvar squamous cell carcinoma in the Tempus database. We included samples which were designated as "vulvar neoplasm" from the TMO table and were also defined as "squamous cell carcinoma" in one of several histological, diagnosis, or pathologic data fields. For the RNA analysis alone, we removed samples from distant metastases and only analyzed samples from primary samples or local metastases. For DNA, we kept all samples, regardless of location (except for removing liver metastases), due to the higher stability to alterations.

### Unsupervised clustering

### FastPG-CC

We used unsupervised clustering to identify cancer-specific and pan-cancer subtypes.

The parameters to the trained model are:
- 'k', specifying FastPG's local neighborhood size
- 'iterations', the total number of clusterings to perform on data subsets
- 'percent_feature_subset', the percent of features to randomly sample in each iteration
- 'percent_sample_subset', the percent of samples to randomly sample in each iteration
- 'min_observations', if not collecting a single consensus clustering, this is the number of iterations a clustering (e.g. a 5-cluster arrangement) must appear in to be considered viable; this parameter gives a mechanism for excluding from the final output clusterings which appear only rarely across iterations
- 'single_consensus', boolean parameter specifying whether or not to force the algorithm to select a single "best" clustering; otherwise, all clusterings meeting the requirements of 'min_observations' will be returned

The procedure is as follows. The 'consensus_cluster' function subsamples the data (according to 'percent_feature_subset' and 'percent_sample_subset'), recording which pairs of samples were present in this subset - the set of samples which can co-cluster in this interation. FastPG is used to cluster this subset of the data and then pairs of samples which co-occur in the same cluster are tallied to give a "connectivity" matrix for this iteration. A consensus is built up by summing the connectivity matrices for all iterations and scaling by a second matrix containing the frequency with which samples were jointly subsampled. We generate a stablest "assignment" of each sample to a cluster in a given clustering (e.g. across all clusterings that resulted in 5 clusters) using hierarchical clustering on the consensus matrix. If collecting a single consensus clustering, the algorithm return the stablest "assignment" calculated in the previous step for the single clustering with the highest modularity score. That is, the function chooses a "best" clustering and uses the optimal sample-level assignments calculated for that clustering.

### vSCC subtype identification

We optimized the cluster definition by using the minimum silhouette width of the cluster from the gene expression of the vSCC samples by iterating through several different ks (10, 15, 20, 30, 40, 50, 60, 70, and 80), using different size gene sets (2,500, 5000, and 10,000 most variable gene). Our final set of parameters were as follows: *k*=60, 100 iterations, 100% of features for each iteration, 80% of samples for each iteration, minimum observations = 10.

### Pan-SCC cohort subtype identification

We next accounted for the effect of sex by taking the residuals from the gene expression. This allowed us to determine robust pan-cancer subtypes.

In addition to taking sex into account, we further accounted for pathway enrichment, cell deconvolution, and pan-SCC cohort inclusion. This allowed us to develop a robust pan-SCC subtype classifier model.

### Arm level copy number calls

To assess the presence of arm-level copy number alterations in solid tumor samples sequenced with xT.v4, we applied a machine learning model (next generation karyotyping; NGK). This model consists of a hierarchical, ordinal logistic regression classifier that predicts the probability of three output states (*deletion, neutral, amplification*) and assigns a call to the most probable of these states. NGK was trained using a combination of Tempus-abstracted clinical sequencing results (e.g., FISH, array-CGH) and TCGA-based estimates of prevalence among all cancer types and chromosomal arms. Features in the NGK model include segment-level CNV calls aggregated by and intersected with each arm-level region of interest.

**Table 14 - vSCC subtype weights**

| | | | | | |
|---|---|---|---|---|---|
| gene | weight | CLDN7 | -0.13634 | EVAlA | 0.059922 |
| ELF3 | -0.42838 | CDH2 | 0.132732 | ABI3BP | 0.058403 |
| P2RY1 | -0.34379 | RUFY4 | 0.128315 | MILR1 | 0.057562 |
| MMP13 | 0.255627 | RGS22 | 0.126687 | CSMD2 | 0.053555 |
| CXCL17 | -0.24174 | CYP24A1 | -0.12531 | MAGEA4 | 0.053307 |
| MYL11 | 0.231941 | GOLGA8T | 0.122883 | OTOF | 0.051324 |
| SLIT2 | 0.220927 | IL20RA | -0.11933 | OR2B6 | -0.05128 |
| GABRA3 | -0.21979 | MAJIN | -0.11853 | TTC24 | 0.050829 |
| EPCAM | -0.20952 | TRAT1 | 0.1133 | GPC6 | 0.050167 |
| AMN | -0.20394 | XCL2 | 0.10865 | MGAT5B | 0.048871 |
| MMP2 | 0.19938 | CCHCR1 | -0.10779 | RPS28 | 0.046214 |
| SRPX | 0.19831 | KLRC4-KLRK1 | 0.106669 | CREB3L1 | 0.041111 |
| CCDC8 | 0.196315 | IGFL2 | -0.10541 | MCIDAS | -0.04052 |
| GFAP | 0.196028 | IGFL3 | -0.10459 | ADSS1 | -0.03709 |
| PLS1 | -0.19594 | MAL2 | -0.10317 | OLFM1 | -0.037 |
| NXPH4 | -0.19318 | FN1 | 0.102779 | OBP2A | 0.036525 |
| PRIMA1 | -0.18859 | AMIGO2 | 0.101084 | CGB8 | 0.036348 |
| TMPRSS4 | -0.18616 | ELOVL7 | -0.09942 | SEPTIN3 | -0.03623 |
| CLEC4C | 0.182521 | | | FAP | 0.035073 |
| EOMES | 0.182315 | TTLL10 | -0.09753 | GOLGA6L9 | -0.03453 |
| ZNF98 | 0.179958 | LILRA4 | 0.094267 | HOXB9 | 0.034528 |
| TAS2R46 | 0.176151 | KCNS1 | -0.09016 | XIRP1 | 0.034371 |
| ZNF208 | 0.174222 | MYH13 | 0.08901 | PGAP4 | -0.02932 |
| GZMK | 0.173747 | TLX2 | -0.08687 | BSPRY | -0.0261 |
| GREM1 | 0.173204 | MYH14 | -0.08634 | SLC66A1LP | 0.025242 |
| SEMA3D | 0.162725 | PNCK | -0.08483 | PRH1 | 0.022373 |
| CA5A | 0.157987 | TTC9 | -0.08358 | COL3A1 | 0.017879 |
| LINC03040 | -0.15633 | DAXX | -0.08336 | FCRL1 | 0.017376 |
| DLX6 | -0.15582 | ANO4 | 0.082491 | TAFA5 | 0.016111 |
| SIGLEC11 | 0.154447 | CYP2C19 | -0.08224 | HAS2 | 0.015819 |
| TMC5 | -0.15348 | AKR1B10 | -0.08209 | YBX2 | -0.01542 |
| FOXE1 | -0.14776 | RGS1 | 0.08001 | SYCP2 | -0.01396 |
| SULF1 | 0.147615 | TBX5 | 0.078191 | FCRL3 | 0.013501 |
| CSPG5 | -0.14531 | NMU | -0.07168 | BCL2L10 | -0.01217 |
| MUC1 | -0.14378 | MAGEA5P | -0.06798 | ESYT3 | -0.01182 |
| LSAMP | 0.139154 | ASPG | -0.0653 | LGALS9B | 0.011527 |
| MEDAG | 0.137643 | LAMP5 | 0.064513 | IRX1 | 0.010037 |
| NRTN | -0.13735 | HAP1 | -0.06116 | KCNJ12 | 0.009309 |
| CPNE7 | -0.1371 | CYP4F3 | -0.05993 | ESPN | -0.00924 |
| CGB5 | 0.007397 | DLX5 | -0.00506 | AMPD1 | 0.000271 |
| GSTM3 | -0.00731 | SFRP2 | 0.003179 | PLP1 | 0.000135 |
| PODXL2 | -0.00586 | PNLIPRP3 | 0.003002 | TDO2 | 2.15E-06 |
| ZYG11A | -0.00555 | OR2B2 | -0.00235 | | |
| AKR1B15 | -0.0051 | TNNT2 | -0.00037 | | |

### Example 3 - Detection of improved cancer therapies

In one example, the disclosed methods and systems are used to detect an improved therapy for a subject suffering from a cancer, e.g., a squamous cell carcinoma (SCC). The subject may have been diagnosed with a cancer that has limited treatment options (e.g., treatment options with poor likelihood of response or only palliative treatments) or no treatment options at all. RNA sequencing, and optionally DNA sequencing, is performed on a sample of a tumor from the subject. Alternatively, previously performed RNA sequencing data from a sample of the subject's tumor is electronically received by a computer system equipped to perform the disclosed methods. The disclosed methods are performed to characterize/classify the subject's cancer based on factors comprising the molecular profile of the cancer, e.g., a plurality of signature genes. The subject's cancer is classified as belonging to a subtype including a molecularly similar group of cancers with treatment options that are improved as compared to the treatment options for the subject's cancer as originally diagnosed. Improved treatment options may comprise treatment options that have a higher likelihood of response for the molecularly similar group of cancers. In the case of a lack of treatment options for the subject's cancer, as originally diagnosed, improved treatment options may be any treatment options. The subject may further be administered the improved treatment options, e.g., a therapeutically effective amount of the improved treatment options.

### Example 4 - Identifying subjects with rare cancers for enrollment in clinical trials for treatments for molecularly similar cancers

In one example, a subject is suffering from a rare cancer, e.g., a cancer that affect fewer than 15 out of every 100,000 people each year or fewer than 40,000 people per year in the U.S. The rare cancer may have limited treatment options (e.g., treatment options with poor likelihood of response or only palliative treatments), no treatment options, or no clinical trials enrolling subjects with the rare cancer. RNA sequencing (and optionally DNA sequencing) is performed on a sample of a tumor from the subject. Alternatively, previously performed RNA sequencing data from a sample of the subject's tumor is electronically received by a computer system equipped to perform the disclosed methods. The disclosed methods are performed to characterize the subject's rare cancer based on factors comprising the molecular profile of the rare cancer, e.g., a plurality of signature genes. The subject's rare cancer is classified as belonging to a subtype including a molecularly similar group of cancers with a clinical trial that is enrolling subjects. The clinical trial may be enrolling subjects based on their molecular profile. The subject may further be enrolled in the clinical trial based on the results of the disclosed methods.

It should be understood that the examples given above are illustrative and do not limit the uses of the systems and methods described herein in combination with a digital and laboratory health care platform.

**Illustrative Clauses (that are not claims):**
Clause 1. A method comprising:
   obtaining, with a computer system, sequencing read data collected from a sample from a cancer of a subject, the read data comprising RNA sequencing data;
   classifying, with the computer system, the cancer as a subtype of cancer, using a trained machine learning algorithm,
      wherein the subtype of cancer comprises a plurality of cell proliferative diseases with common characteristics wherein the common characteristics comprise similar molecular profiles,
      wherein the trained machine learning algorithm is trained on a data set of sequencing read data collected from a cohort of subjects suffering from cancer.
Clause 2. A method of classifying a cancer from a subject:
   obtaining, with a computer system, sequencing read data collected from a sample from the cancer of the subject, the read data comprising RNA sequencing data;
   classifying, with the computer system, the cancer as a subtype of cancer, using a trained machine learning algorithm,
      wherein the subtype of cancer comprises a plurality of cell proliferative diseases with common characteristics wherein the common characteristics comprise similar molecular profiles,
      wherein the trained machine learning algorithm is trained on a data set of sequencing read data collected from a cohort of subjects suffering from cancer.
Clause 3. A method of diagnosing a cancer from a subject:
   obtaining, with a computer system, sequencing read data collected from a sample of the cancer, the read data comprising RNA sequencing data;
   classifying, with the computer system, the cancer as a subtype of cancer, using a trained machine learning algorithm,
      wherein the subtype of cancer comprises a plurality of cell proliferative diseases with common characteristics wherein the common characteristics comprise similar molecular profiles,
      wherein the trained machine learning algorithm is trained on a data set of sequencing read data collected from a cohort of subjects suffering from cancer.
Clause 4. A method of identifying treatment options for a subject suffering from a cancer for which there are limited treatments:
   obtaining, with a computer system, sequencing read data collected from a sample of the cancer from the subject, wherein the read data comprising RNA sequencing data;
   classifying, with the computer system, the cancer as a subtype of cancer, using a trained machine learning algorithm,
      wherein the subtype of cancer comprises a plurality of cell proliferative diseases with common characteristics wherein the common characteristics comprise similar molecular profiles,
      wherein the trained machine learning algorithm is trained on a data set of sequencing read data collected from a cohort of subjects suffering from cancer.
Clause 5. The method of any one of clauses 1-4, wherein the sample comprises at least one of a tumor sample, blood sample, or cell free DNA.
Clause 6. The method of any one of clauses 1-5, wherein the plurality of cell proliferative diseases comprises squamous cell carcinomas (SCC).
Clause 7. The method of clause 6, wherein the squamous cell carcinomas comprises anogenital, cervical, esophageal, head and neck, lung, skin, urothelial, colorectal, and vulvar squamous cell carcinomas.
Clause 8. The method of any one of clauses 1-7, wherein the common characteristics further comprises similar phenotypes, prognosis, and predicted responses to treatment.
Clause 9. The method of clause 8, where the similar phenotypes comprise symptoms, comorbidities, and lifestyle habits.
Clause 10. The method of clause 9, wherein the comorbidities comprise HPV status.
Clause 11. The method of any one of clauses 8-10, wherein the prognosis comprises survivability, aggressiveness, and stage.
Clause 12. The method of any one of clauses 8-11, wherein the predicted response to treatment comprises predicted response to chemotherapy.
Clause 13. The method of any one of clauses 8-11, wherein the predicted response to treatment comprises predicted response to an immunotherapy, or a chemotherapy, or targetable mutation small molecule inhibitors, such as PIK3CA inhibitors.
Clause 14. The method of clause 13, wherein the immunotherapy comprises an immune checkpoint inhibitor (ICI).
Clause 15. The method of clause 13 or 14, wherein the chemotherapy comprises a platinum-based therapy or a taxane therapy.
Clause 16. The method of clause 15, wherein the platinum-based therapy comprises carboplatin.
Clause 17. The method of clause 15 or 16, wherein the taxane therapy comprises paclitaxel.
Clause 18. The method of any one of clauses 1-13, wherein the similar molecular profiles comprise expression levels of one or more of RNF186, CCL15, TMIGD1, RPL10L, ATOH1, ANKS4B, ALPI, SCL17A4, B3GNT6, MOGAT3, SFTA3, GGTLCl, NAPSA, SFTPD, MS4A15, VWA3A, ANKRD66, HABP2, CPAMD8, KCNK3, CFAP95, CFAP43, OSGIN1, SRXN1, G6PD, ETNK2, DGKG, NDGA1, LDC1, RAB3B, TAGA3, PLCXD2, GSTM2, WNT5A, RAB25, TTLL10, SGPP2, SPINK9, IGSF9, ARHGEF26, PIR, RAPGEFL1, CIMAP2, SCNN1A, ZBTB7C, BDNF, ARG1, TREX2, CMA1, KRTAP5-4, LIPM, SPTLC3, GCSAML, HAL, LGALSL, VSIG8, TMC4, ELMOD1, SMPD3, GRACDL, DPF1, RAX, GATM, KLHL35, TMEM236, ACTBL2, TCEA3, EPB41LB, CT62, DKK3, FJX1, CASP5, MANEAL, or NUP210.
Clause 19. The method of any one of clauses 1-18, wherein the cohort of subjects comprises subjects diagnosed with at least 5 different types of cancers.
Clause 20. The method of any one of clauses 1-19, wherein each subject in the cohort of subjects has been diagnosed with a squamous cell carcinoma.
Clause 21. The method of any one of clauses 1-20, wherein the trained machine learning algorithm comprises at least one of a gradient boosting model, a random forest model, a neural network, a regression model, ElasticNet, or a Naive Bayes model.
Clause 22. The method of any one of clauses 1-21, wherein the trained machine learning algorithm is ElasticNet.
Clause 23. The method of any one of clauses 1-22, wherein the method further comprises generating a report.
Clause 24. The method of clause 23, wherein the report comprises the subtype of cancer, the plurality of cell proliferative diseases with common characteristics, and the molecular profiles.
Clause 25. The method of any one of clauses 23-24, wherein the report further comprises patient data.
Clause 26. The method of any one of clauses 23-25, wherein the report further comprises a list of treatment options.
Clause 27. The method of clause 3, wherein the diagnosed cancer comprises a squamous cell carcinoma. Clause 28. The method of clause 3, wherein the diagnosed cancer does not comprise a squamous cell carcinoma.
Clause 29. The method of clause 4, wherein limited treatments comprise at least one of ineffective treatments, few treatments, and no known treatments.
Clause 30. The method of clause 4 or 29, wherein the treatment options are identified based on the plurality of cell proliferative diseases with common characteristics and the molecular profile.
Clause 31. The method of any one of clauses 4, 29, or 30, wherein the cancer with limited treatments is vulvar squamous cell carcinoma.
Clause 32. The method of any one of clauses 1-31, wherein the molecular profiles comprise RNA expression data and the computer system classifies the cancer based on expression of a plurality of signature genes in the RNA sequencing data.
Clause 33. The method of clause 32, wherein the plurality of signature genes comprises two or more genes selected from one of (i), (ii), (iii), (iv), (v), or (vi):
   (i) CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, or ZSWIM5;
   (ii) ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, or WNT16;
   (iii) RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRKl, POF1B, CBX2, CEACAM1, THBS1, NEBL, CCDC185, C20orf144, or CHODL;
   (iv) OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQOl, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, Clorf226, ENTHD1, SLC7A11, UGT1A1, MST1R, AKRIC1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, or LGSN;
   (v) SFTA3, GGTLCl, NAPSA, SFTPD, MS4A15, VWA3A, ANKRD66, HABP2, CPAMD8, KCNK3, CFAP95, CFAP43, CFAP221, NKX2-1, FOXB1, C16orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, C1orf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, or HYDIN;
   (vi) RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCAl, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, or BTNL3.
Clause 34. A method of classifying a cancer, the method comprising:
   obtaining, with a computer system, sequencing read data collected from a sample of the cancer, the read data comprising RNA sequencing data;
   classifying, with the computer system, the cancer as a subtype of cancer, using a trained machine learning algorithm,
      wherein the subtype of cancer comprises a plurality of cell proliferative diseases with common characteristics, wherein the common characteristics comprise similar molecular profiles, wherein the molecular profiles comprise RNA expression data and the computer system classifies the cancer based on expression of a plurality of signature genes in the RNA sequencing data, and
      wherein the trained machine learning algorithm is trained on a data set of sequencing read data collected from a cohort of subjects suffering from cancer.
Clause 35. The method of clause 34, wherein the plurality of signature genes comprises two or more genes selected from the group consisting of CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, and ZSWIMS.
Clause 36. The method of clause 34, wherein the plurality of signature genes comprises CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, and ZSWIM5.
Clause 37. The method of clause 34, wherein the plurality of signature genes comprises two or more genes selected from the group consisting of ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, and WNT16.
Clause 38. The method of clause 34, wherein the plurality of signature genes comprises ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, and WNT16.
Clause 39. The method of clause 34, wherein the plurality of signature genes comprises two or more genes selected from the group consisting of RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRKl, POF1B, CBX2, CEACAM1, THBS1, NEBL, CCDC185, C20orf144, and CHODL.
Clause 40. The method of clause 34, wherein the plurality of signature genes comprises RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRKl, POF1B, CBX2, CEACAM1, THBS1, NEBL, CCDC185, C20orf144, and CHODL.
Clause 41. The method of clause 34, wherein the plurality of signature genes comprises two or more genes selected from the group consisting of OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, Clorf226, ENTHD1, SLC7A11, UGT1A1, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, and LGSN.
Clause 42. The method of clause 34, wherein the plurality of signature genes comprises OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQO1, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, C1orf226, ENTHD1, SLC7A11, UGT1A1, MST1R, AKR1C1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, and LGSN.
Clause 43. The method of clause 34, wherein the plurality of signature genes comprises two or more genes selected from the group consisting of SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, C16orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, C1orf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, and HYDIN.
Clause 44. The method of clause 34, wherein the plurality of signature genes comprises SFTA3, GGTLC1, NAPSA, SFTPD, MS4A15, VWA3A, ANKRD66, HABP2, CPAMD8, KCNK3, CFAP95, CFAP43, CFAP221, NKX2-1, FOXB1, C16orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, Clorf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, Clorf222, TEKT2, CFAP90, AGBL1, SNTN, DRC1, MIA2, C4A, RSPH1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, and HYDIN.
Clause 45. The method of clause 34, wherein the plurality of signature genes comprises two or more genes selected from the group consisting of RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, and BTNL3.
Clause 46. The method of clause 34, wherein the plurality of signature genes comprises RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCA1, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, and BTNL3.
Clause 47. The method of any one of clauses 34-46, wherein the sample comprises at least one of a tumor sample, blood sample, or cell free DNA.
Clause 48. The method of any one of clauses 34-47, wherein the plurality of cell proliferative diseases comprises squamous cell carcinomas (SCC).
Clause 49. The method of clause 48, wherein the squamous cell carcinomas comprises anogenital, cervical, esophageal, head and neck, lung, skin, urothelial, colorectal, and vulvar.
Clause 50. The method of any one of clauses 34-49, wherein the common characteristics further comprises similar phenotypes, prognosis, and predicted responses to treatment.
Clause 51. The method of clause 50, where the similar phenotypes comprise symptoms, comorbidities, and lifestyle habits.
Clause 52. The method of clauses 50 or 51, wherein the comorbidities comprise HPV status.
Clause 53. The method of any one of clauses 50-52, wherein the prognosis comprises survivability, aggressiveness, and stage.
Clause 54. The method of any one of clauses 50-53, wherein the predicted response to treatment comprises predicted response to chemotherapy.
Clause 55. The method of any one of clauses 50-54, wherein the predicted response to treatment comprises predicted response to an immunotherapy, or a chemotherapy.
Clause 56. The method of clause 55, wherein the immunotherapy comprises an immune checkpoint inhibitor (ICI).
Clause 57. The method of clause 56, wherein the chemotherapy comprises a platinum-based therapy or a taxane therapy.
Clause 58. The method of clause 57, wherein the platinum-based therapy comprises cisplatin.
Clause 59. The method of clause 57, wherein the taxane therapy comprises paclitaxel.
Clause 60. The method of any one of clauses 34-49, wherein each subject in the cohort of subjects has been diagnosed with a cancer that is different from other subjects in the cohort of subjects.
Clause 61. The method of any one of clauses 34-60, wherein each subject in the cohort of subjects has been diagnosed with a squamous cell carcinoma.
Clause 62. The method of any one of clauses 34-61, wherein the trained machine learning algorithm is comprises at least one of a gradient boosting model, a random forest model, a neural network, a regression model, ElasticNet, or a Naive Bayes model.
Clause 63. The method of any one of clauses 34-62, wherein the trained machine learning algorithm is ElasticNet.
Clause 64. The method of any one of clauses 34-63, wherein the method further comprises generating a report.
Clause 65. The method of clause 64, wherein the report comprises the subtype of cancer, the plurality of cell proliferative diseases with common characteristics, and the molecular profiles.
Clause 66. The method of any one of clauses 64-65, wherein the report further comprises patient data.
Clause 67. The method of any one of clauses 64-66, wherein the report further comprises recommended treatment options.
Clause 68. The method of clause 34, wherein the cancer comprises a squamous cell carcinoma.
Clause 69. The method of clause 34, wherein the cancer does not comprise a squamous cell carcinoma.
Clause 70. The method of clause 34, wherein limited treatments comprise at least one of ineffective treatments, few treatments, and no known treatments.
Clause 71. The method of clause 34, wherein the treatment options are identified based on the plurality of cell proliferative diseases with common characteristics and the molecular profile.
Clause 72. The method of clause 34, wherein the cancer with limited treatments is vulvar squamous cell carcinoma.

## Claims

1. A method of classifying a cancer from a subject:
obtaining, with a computer system, sequencing read data collected from a sample from the cancer of the subject, the read data comprising RNA sequencing data;
classifying, with the computer system, the cancer as a subtype of cancer, using a trained machine learning algorithm,
wherein the subtype of cancer comprises a plurality of cell proliferative diseases with common characteristics, wherein the common characteristics comprise similar molecular profiles,
wherein the trained machine learning algorithm is trained on a data set of sequencing read data collected from a cohort of subjects suffering from cancer, wherein the squamous cell carcinomas comprises anogenital, cervical, esophageal, head and neck, lung, skin, urothelial, colorectal, and vulvar squamous cell carcinomas.

2. The method of claim 1, wherein the sample comprises at least one of a tumor sample, blood sample, or cell free DNA.

3. The method of claim 1 or claim 2, wherein the plurality of cell proliferative diseases comprises squamous cell carcinomas (SCC).

4. The method of any preceding claim, wherein the common characteristics further comprises similar phenotypes, prognosis, and predicted responses to treatment; and optionally
wherein the predicted response to treatment comprises predicted response to chemotherapy.

5. The method of any preceding claim, wherein the similar molecular profiles comprise expression levels of one or more of RNF186, CCL15, TMIGD1, RPL10L, ATOH1, ANKS4B, ALPI, SCL17A4, B3GNT6, MOGAT3, SFTA3, GGTLCl, NAPSA, SFTPD, MS4A15, VWA3A, ANKRD66, HABP2, CPAMD8, KCNK3, CFAP95, CFAP43, OSGIN1, SRXN1, G6PD, ETNK2, DGKG, NDGA1, LDC1, RAB3B, TAGA3, PLCXD2, GSTM2, WNT5A, RAB25, TTLL10, SGPP2, SPINK9, IGSF9, ARHGEF26, PIR, RAPGEFL1, CIMAP2, SCNN1A, ZBTB7C, BDNF, ARG1, TREX2, CMA1, KRTAP5-4, LIPM, SPTLC3, GCSAML, HAL, LGALSL, VSIG8, TMC4, ELMOD1, SMPD3, GRACDL, DPF1, RAX, GATM, KLHL35, TMEM236, ACTBL2, TCEA3, EPB41LB, CT62, DKK3, FJX1, CASP5, MANEAL, or NUP210.

6. The method of any preceding claim, wherein the cohort of subjects comprises subjects diagnosed with at least 5 different types of cancers.

7. The method of any preceding claim, wherein each subject in the cohort of subjects has been diagnosed with a squamous cell carcinoma.

8. The method of any preceding claim, wherein the trained machine learning algorithm comprises at least one of a gradient boosting model, a random forest model, a neural network, a regression model, ElasticNet, or a Naive Bayes model; and optionally
wherein the trained machine learning algorithm is ElasticNet.

9. The method of any preceding claim, wherein the method further comprises generating a report; and optionally
wherein the report comprises the subtype of cancer, the plurality of cell proliferative diseases with common characteristics, and the molecular profiles.

10. The method of any preceding claim, wherein the report further comprises a list of treatment options.

11. The method of any preceding claim, wherein the cancer is classified as a squamous cell carcinoma; and/or
the method of any of preceding directly or indirectly dependent upon claim 3, wherein the cancer is not classified as a squamous cell carcinoma.

12. The method of any preceding claim, wherein the treatment options are identified based on the plurality of cell proliferative diseases with common characteristics and the molecular profile.

13. The method of any preceding claim, wherein the cancer has limited treatments comprising at least one of ineffective treatments, few treatments, and no known treatments; and optionally
wherein the cancer with limited treatments is vulvar squamous cell carcinoma.

14. The method of any preceding claim, wherein the plurality of signature genes comprise two or more genes selected from one of (i), (ii), (iii), (iv), (v), or (vi):
(i) CRACDL, DPF1, RAX, GATM, KLHL35, TMEM236, ACTBL2, TCEA3, EPB41L4B, CT62, DKK3, FJX1, CASP5, MANEAL, NUP210, RPL10L, FOXF2, LIPG, GRID2, C2orf48, SH3TC2, MECOM, SPACA5, SHC4, R3HDML, BRME1, L1TD1, ZAR1, SLC28A1, FAM169A, FEV, SPMIP11, GLI1, CRYBB2, KIRREL3, PI15, FEZ1, C2CD4B, PLEKHG4, GOLGA6L10, GRIN2C, CELF5, TSPAN18, CARD10, ACOD1, PLCH1, AR, MTNR1A, PPP1R14C, B4GALNT3, ESR1, PITX1, PRSS46P, CHRNA3, DNAJB13, RET, PAX8, ANKRD65, ZDHHC19, IGF2BP2, KLF8, TACSTD2, CCDC166, TRIL, ZP4, SHISAL2A, TMT1B, ADGRE1, OCM, PIWIL2, SNCB, PDPN, RASD2, NICOL1, COLEC10, GJE1, EGR3, RIBC2, SLC26A5, SLC2A12, GABRB1, SGCG, GABRA2, FAM81A, ATP8A2, USP2, RAPGEFL1, NAALADL2, CCDC185, NANOG, HTR2C, SLC10A4, PHACTR3, NPSR1, TRH, PMP2, HBEGF, C22orf31, LVRN, or ZSWIM5;
(ii) ARG1, TREX2, CMA1, KRTAP5-4, LIPM, SPTLC3, GCSAML, HAL, LGALSL, VSIG8, TMC4, ELMOD1, SMPD3, ACER1, ABCG4, ATP6V1C2, TPPP2, DCD, ELOVL4, KRT25, RNF222, ACSBG1, ANKRD31, MELTF, NPM2, FRMPD1, ENDOU, LCE5A, USP2, LCE1B, DGAT2, LCE1E, PNPLA1, SERPINA12, SYT17, TMEM45A, CCL27, LCE6A, RDH12, ASPRV1, XKRX, TUBB2A, MMP27, HOPX, MS4A2, KRT33B, ESYT3, GALNT6, DEGS2, LIPN, IL37, ACKR2, LCE1D, HTR3A, DCT, RARB, OPN1MW, SPAG11B, FLG2, DEFB105B, VIPR1, LCE1A, SPACA5, SCGB1D2, GLB1L3, TEX28P2, HDC, PTGS1, RDH16, KRT80, CIDEA, SCN4B, HYAL4, CTSG, GPR63, TYR, LELP1, LYPD5, SCGB2A2, HOXD1, TEX28P1, RHBG, FLG, AADACL3, BPIFC, TRPM1, OPN1LW, NEU2, NSG1, MECOM, GALNT12, COX8C, TEX28, IL1F10, LORICRIN, GATA3, PTPN5, NWD2, KRT84, or WNT16;
(iii) RAB25, TTLL10, SGPP2, SPINK9, IGSF9, ARHGEF26, PIR, RAPGEFL1, CIMAP2, SCNN1A, ZBTB7C, BDNF, ACSBG1, PGAP4, ZNF711, ACP3, TMEM125, CLDN4, GGT6, P2RY1, Clorf210, OTX1, CSN3, ESYT3, TTC39A, RNF183, VSIG8, DNAI7, C22orf31, FAM181A, GSTA4, ALG1L2, PLS1, BMP7, CFAP73, EFCC1, ISL2, ENDOU, L1CAM, CYP4X1, GPX2, IL20RA, COMMD5P1, SOX1, PCP4L1, KRTAP5-2, FA2H, SAMD12, SRXN1, GRID2, TRH, TLCD4-RWDD3, RNF225, MCIDAS, NDRG4, PRR35, CCN3, LIPM, OVOL2, CGN, POU2F3, HOPX, DOC2B, RBBP8NL, B4GALNT3, SPOCK1, GLYATL1, SRRM3, BSPRY, CACNA2D3, PHGDH, BCL2L15, B3GNT6, ZNF385C, VEGFC, EBF3, ACTBL2, VAX2, ZDHHC11, ART3, MYH14, TGFBI, C2orf48, LINC02898, CFAP276, PLA2G3, GCSAML, MYOM3, FGFR2, ALG1L1P, KLHDC7A, OPRKl, POF1B, CBX2, CEACAM1, THBS1, NEBL, CCDC185, C20orf144, or CHODL;
(iv) OSGIN1, SRXN1, G6PD, ETNK2, DGKG, MDGA1, ODC1, RAB3B, GATA3, PLCXD2, GSTM2, WNT5A, BDNF, PIR, OR6C2, ME1, GPAT3, NQOl, TRIM16L, JAKMIP3, NECAB2, GLI2, SLC38A8, CYP2S1, GSTM3, CCL28, GPX2, NOG, C1QTNF12, TSPAN7, OR56B4, SCN9A, NKX6-1, GLI1, PANX2, CFAP20DC, Clorf226, ENTHD1, SLC7A11, UGT1A1, MST1R, AKRIC1, RAB6B, H4C9, CCDC125, VPS37D, DPF1, SLC6A13, B4GALNT3, GCNT2, GASK1A, CCL26, NR0B1, KLRG1, ARTN, NRCAM, ELAPOR2, KCND3, TPRG1, ZMAT1, OTOP2, RORC, PCYT1B, RND2, SGCZ, SAMD12, HAP1, BRD2, DAZ3, AKR1C3, ENPP3, ANO1, MACROD2, UPK1B, JAKMIP2, AKR1C4, ETNPPL, PFN2, ANXA10, LRRC2, ZDHHC2, NUDT11, CNTN6, SLC4A3, ALDH3A1, TMC1, OR6C70, DLG2, CIMAP2, VIPR1, SPTLC3, KIT, CYP26A1, ROR1, PMP2, NYAP1, FGF13, SAMD3, S100A5, or LGSN;
(v) SFTA3, GGTLCl, NAPSA, SFTPD, MS4A15, VWA3A, ANKRD66, HABP2, CPAMD8, KCNK3, CFAP95, CFAP43, CFAP221, NKX2-1, FOXB1, C16orf89, C8B, NEK5, LRP2, AQP4, SLC9C2, C4BPA, TMEM212, STOML3, CDH7, KIAA2012, DLG2, TTC29, USP44, F11, PPM1H, PGC, SFTPB, ODAD1, CATSPERD, PEBP4, PLCH1, ZBBX, CFAP107, C1orf87, DAW1, ROPN1L, FYB2, KCTD16, C8orf34, PCDHAC2, CP, ERICH3, RP1, ABCC6, KHDRBS2, PLA2G1B, SPEF2, SCN1A, CFAP276, WFDC6, SLC22A31, RGPD3, KRTAP10-9, DNAI1, ACSM1, RAB6C, CFAP65, MARCHF10, CDHR3, FRMPD2, DNAI7, ERICH2, DNAH12, ZNF648, CIMIP1, GARIN6, ARMC3, HOATZ, C2orf73, C1orf222, TEKT2, CFAP90, AGBL1, SNTN, DRC1, MIA2, C4A, RSPHI1, ASB4, STMND1, DNAH5, CABCOCO1, NME5, HP, TSPAN19, CGNL1, MALRD1, SHISA3, CNTN6, SCGB3A2, NRGN, XAGE1C, ABCA3, or HYDIN;
(vi) RNF186, CCL15, TMIGD1, RPL10L, ATOH1, ANKS4B, ALPI, SLC17A4, B3GNT6, MOGAT3, NR1I2, IHH, MS4A12, A1CF, FEV, CLRN3, NHERF4, INSL5, R3HDML, GUCA2B, NXPE1, MYO1A, HNF1A, NAT2, PYY, NXPE4, AQP8, NOX1, REG3A, UGT2A3, TRIM15, B3GALT1, ISX, CDH17, NXPE2, MEP1A, GCG, CDHR2, CHST5, B3GNT7, ZG16, GALNT8, EFNA2, TINAG, LYPD8, SLC51B, FABP2, LEFTY1, HTR4, CHGA, TM4SF5, MYO7B, LGALS4, SLC6A19, CDX1, SI, RETNLB, PLA2G10, BCL2L15, TMEM236, SLC18A1, SAMD13, CA7, HHLA2, SULT1B1, C5orf52, GPA33, REG1B, GP9, HEPACAM2, LRRC31, GUCA2A, REG4, VSIG2, CLCAl, SLC26A3, IYD, BNIP5, GREM2, SGK2, HGD, VIL1, VSTM2A, KRT20, SPMIP10, SLC28A2, AOC1, ANXA13, GUCY2C, FAM135B, CA1, CAPN9, GABRA2, ALDOB, SULT1C3, HNF4A, MUC12, PPP1R14D, SPINK4, or BTNL3.

15. A system for classifying a cancer from a subject, the system comprising at least one memory, and at least one processor coupled to the at least one memory,
the system configured to cause the at least one processor to execute instructions stored in the at least one memory to:
obtain, with a computer system, sequencing read data collected from a sample from the cancer of the subject, the read data comprising RNA sequencing data;
classify, with the computer system, the cancer as a subtype of cancer, using a trained machine learning algorithm,
wherein the subtype of cancer comprises a plurality of cell proliferative diseases with common characteristics, wherein the common characteristics comprise similar molecular profiles,
wherein the trained machine learning algorithm is trained on a data set of sequencing read data collected from a cohort of subjects suffering from cancer.
